(19) Europäisches Patentamt
European Patent Office
Office européen des brevets

(11) Veröffentlichungsnummer: **0 318 893 B1**

# (12) EUROPÄISCHE PATENTSCHRIFT

(45) Veröffentlichungstag der Patentschrift: **19.01.94**

(51) Int. Cl.[5]: **C07F 17/00**, C08F 2/50, G03F 7/027, C07F 7/10, C07C 211/00, C07C 233/00

(21) Anmeldenummer: **88119798.2**

(22) Anmeldetag: **28.11.88**

## (54) Titanocene, deren Verwendung und N-substituierte Fluoraniline.

(30) Priorität: **01.12.87 CH 4682/87**

(43) Veröffentlichungstag der Anmeldung: **07.06.89 Patentblatt 89/23**

(45) Bekanntmachung des Hinweises auf die Patenterteilung: **19.01.94 Patentblatt 94/03**

(84) Benannte Vertragsstaaten: **AT BE CH DE FR GB IT LI NL SE**

(56) Entgegenhaltungen:
**EP-A- 0 119 162**
**EP-A- 0 122 223**
**EP-A- 0 173 284**
**EP-A- 0 186 626**
**EP-A- 0 207 893**
**EP-A- 0 242 330**
**EP-A- 0 255 486**
**EP-A- 0 256 981**
**EP-A- 0 269 573**
**DE-A- 3 715 181**
**DE-B- 1 193 030**
**US-A- 3 565 934**
**US-A- 4 120 693**

(73) Patentinhaber: **CIBA-GEIGY AG**
**Klybeckstrasse 141**
**CH-4002 Basel(CH)**

(72) Erfinder: **Riediker, Martin, Dr.**
**Gstaltenrainweg 75**
**CH-4125 Riehen(CH)**
Erfinder: **Steiner, Eginhard, Dr.**
**Obere Hofackerstrasse 3**
**CH-4414 Füllinsdorf(CH)**
Erfinder: **Beyeler, Harry**
**Marignanostrasse 35**
**CH-4059 Basel(CH)**
Erfinder: **Sitek, Franciszek, Dr.**
**Grossmattweg 11**
**CH-4106 Therwil(CH)**
Erfinder: **Hüsler, Rinaldo, Dr.**
**Route du Confin 52**
**CH-1723 Marly(CH)**

EP 0 318 893 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 82, 1975, Seite 164, Zusammenfassung Nr. 27107v, Columbus, Ohio, US; R.D. TREPKA et al.: "Synthesis and herbicidal activity of fluorinated N-phenylalkanesulfonamides" & J. AGRIC. FOOD CHEM. 1974, 22(6), 1111-19

JOURNAL OF MEDICINAL CHEMISTRY, Band 13, Nr. 3, Mai 1970, Seiten 546-549; R:K: RAZDAN et al: Antimalarials. Antagonists of pantothenic acid"

CHEMICAL ABSTRACTS, Band 95, 1981, Seite 599, Zusammenfassung Nr. 60751s, Columbus, Ohio, US; M.P. SIBI et al.: "Nitorgen-15 nuclear magnetic resonance spectroscopy. Nitrogen-15-fluroine-19 coupling constants in fluoropyridines and fluoroanilines" & ORG. MAGN. RESON. 1980, 14(6), 494-6

CHEMICAL ABSTRACTS, Band 87, 1977, Seite 661, Zusammenfassung Nr. 134366d, Columbus, Ohio, US; J.M. BARRALES-RIENDA et al.: "Synthesis of N-(fluuorophenyl)maleimides" & J. FLUROINE CHEM. 1977, 9(4), 293-308

Trepka; J. Agric. Food Chem. Vol. 22 Nr. 6 (1974); Seiten 1111 - 1119

J. Org. Magn. Res. 40(6) (1980; Seiten 494-496

Barrales-Rienda; J. Fluorine Chem. 9 (4) 1977 Seiten 293-308

THE JOURNAL OF ORGANIC CHEMISTRY, Band 50, Nr.23, Nov. 15, 1985, Seiten 4576-4582, The American Chemical Society; M.J. FIFOLT et al.

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

## Beschreibung

Die vorliegende Erfindung betrifft Titanocene mit N-substituierten fluorhaltigen aromatischen Resten, eine photopolymerisierbare Zusammensetzung aus ethylenisch ungesättigten Verbindungen, die diese Titanocene als Photoinitiatoren enthalten, ein mit dieser Zusammensetzung beschichtetes Substrat, ein Verfahren zur Herstellung photographischer Reliefabbildungen unter Verwendung dieses beschichteten Substrates und N-substituierte Fluoraniline.

Aus der EP-A-0 122 223 ist es bekannt, dass Titanocene mit Fluorarylliganden ausgezeichnete Photoinitiatoren sind. Die Fluorarylliganden dieser Titanocene können zum Beispiel mit primären oder sekundären Aminogruppen substituiert sein. Die Substitution mit Acylaminogruppen ist nicht erwähnt.

Ein Gegenstand der Erfindung sind Titanocene der Formel I

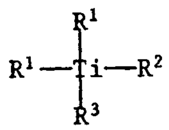

I,

worin beide $R^1$ unabhängig voneinander unsubstituiertes oder durch $C_1$-$C_{18}$-Alkyl oder -Alkoxy, $C_2$-$C_{18}$-Alkenyl, $C_5$-$C_8$-Cycloalkyl, $C_6$-$C_{16}$-Aryl, $C_7$-$C_{16}$-Aralkyl, $SiR^4{}_3$, $GeR^4{}_3$, Cyano oder Halogen substituiertes Cyclopentadienyl$^{\ominus}$, Indenyl$^{\ominus}$ oder 4,5,6,7-Tetrahydro-indenyl$^{\ominus}$ bedeuten oder beide $R^1$ zusammen für einen unsubstituierten oder wie zuvor substituierten Rest der Formel II

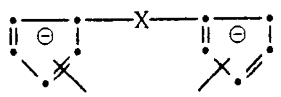

II

stehen, worin X $-(CH_2)_n-$ mit n = 1, 2 oder 3, gegebenenfalls durch Phenyl substituiertes Alkyliden mit 2 bis 12 C-Atomen, Cycloalkyliden mit 5 bis 7 Ringkohlenstoffatomen, $SiR^4{}_2$, $SiR^4{}_2$-O-$SiR^4{}_2$, $GeR^4{}_2$ oder $SnR^4{}_2$ ist, und $R^4$ $C_1$-$C_{12}$-Alkyl, $C_5$-$C_{12}$-Cycloalkyl, $C_6$-$C_{16}$-Aryl oder $C_7$-$C_{16}$-Aralkyl bedeutet,

$R^2$ einen 6-gliedrigen carbocyclischen oder 5- oder 6-gliedrigen heterocyclischen aromatischen Rest bedeutet, der in mindestens einer der beiden ortho-Stellungen zur Titankohlenstoffbindung mit Fluoratomen substituiert ist und wobei der aromatische Rest weitere Substituenten enthalten kann.

$R^3$ eine der für $R^2$ gegebenen Bedeutungen hat oder $R^2$ und $R^3$ zusammen einen Rest der Formel III bedeuten,

$$-Q-Y-Q- \qquad III$$

in dem Q für einen carbocyclischen aromatischen Rest steht, wobei die beiden Bindungen jeweils in Orthostellung zur Y-Gruppe stehen und die zweite Orthostellung zur Titankohlenstoffbindung jeweils durhc ein Fluoratom substituiert ist und wobei Q weitere Substituenten enthalten kann, und Y $CH_2$, Alkyliden mit 2 bis 12 C-Atomen, Cycloalkyliden mit 5 bis 7 Ringkohlenstoffatomen, $NR^4$, O, S, SO, $SO_2$, CO, $SiR^4{}_2$, $GeR^4{}_2$ oder $SnR^4{}_2$ bedeutet und $R^4$ die zuvor angegebene Bedeutung hat,

wobei die Titanocene dadurch gekennzeichnet sind, dass $R^2$ und $R^3$ oder der Rest der Formel III durch einen Rest der Formel IV, IVa oder IVb substituiert sind,

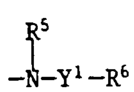

IV

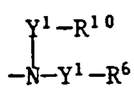

IVa

worin $R^5$ Wasserstoff, lineares oder verzweigtes $C_1$-$C_{20}$-Alkyl, $C_2$-$C_{20}$-Alkenyl, $C_3$-$C_8$-Cycloalkyl, $C_4$-$C_{20}$-Cycloalkylalkyl oder -Alkylcycloalkyl, $C_5$-$C_{20}$-Alkylcycloalkylalkyl, $C_6$-$C_{20}$ Cycloalkenylalkyl, $C_6$-$C_{14}$-Aryl, $C_7$-$C_{20}$-Aralkyl oder -Alkaryl, $C_8$-$C_{20}$-Alkaralkyl oder $C_3$-$C_{12}$-Trialkylsilyl darstellt, wobei diese Reste unsubstituiert oder durch $C_1$-$C_{18}$-Alkoxy, $C_1$-$C_{18}$-Alkylthio, $C_1$-$C_{18}$-Alkylsulfonyl, $C_6$-$C_{10}$-Arylsulfonyl, $C_7$-$C_{20}$-Alkarylsulfonyl, 2-Tetrahydrofuryl oder Cyano substituiert sind,

$R^6$ eine der für $R^5$ gegebenen Bedeutungen hat oder $C_1$-$C_{20}$-Halogenalkyl, durch -CO- unterbrochenes $C_2$-$C_{20}$-Alkyl oder durch -COOH oder -$COOR^4$ substituiertes $C_1$-$C_{12}$-Alkyl ist und im Falle, dass $Y^1$ -CO-, -CS- oder -$SO_2$- ist, auch -$NR^7R^8$ bedeuten kann, worin $R^7$ und $R^8$ unabhängig voneinander eine der für $R^5$ gegebenen Bedeutungen haben oder $R^7$ und $R^8$ zusammen $C_3$-$C_7$-Alkylen bedeuten, das durch -O-, -S- oder -$N(R^9)$- unterbrochen werden kann, worin $R^9$ Wasserstoff, $C_1$-$C_{12}$-Alkyl, $C_3$-$C_{12}$-Alkenyl, $C_7$-$C_{12}$-Aralkyl oder $C_2$-$C_{20}$-Alkanoyl bedeutet,
oder $R^5$ und $R^6$ zusammen lineares oder verzweigtes $C_2$-$C_8$-Alkylen oder durch Halogen, $C_1$-$C_4$-Alkoxy, Allyloxy oder -$NR^7R^8$ substituiertes $C_2$-$C_8$-Alkylen oder einen zweiwertigen Rest der Formel

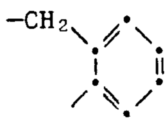 oder 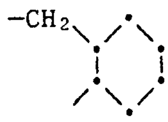 oder 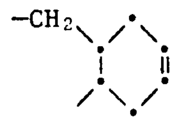

bedeuten,
$Y^1$ eine Gruppe -CO-, -CS-, -COO-, -$SO_2$- oder -$SiR^4{}_2$- bedeutet, worin $R^4$ die zuvor gegebene Bedeutung hat,
$R^{10}$ eine der für $R^6$ gegebenen Bedeutungen hat oder $R^{10}$ und $R^6$ zusammen $C_1$-$C_8$-Alkandiyl, $C_2$-$C_8$-Alkendiyl, $C_6$-$C_{14}$-Arendiyl, $C_4$-$C_{12}$-Cycloalkandiyl, $C_5$-$C_{12}$-Cycloalkendiyl, $C_6$-$C_{14}$Cycloalkadiendiyl, $C_7$-$C_{20}$-Bicycloalkandiyl, $C_7$-$C_{20}$-Bicycloalkendiyl oder durch -O-, -S-oder -$N(R^9)$-unterbrochenes $C_2$-$C_4$-Alkandiyl bedeuten, wobei diese Reste unsubstituiert oder durch einen oder mehrere der Substituenten Halogen, $C_1$-$C_{10}$-Alkoxy, $C_1$-$C_{20}$-Alkyl, $C_3$-$C_{20}$-Alkenyl oder $C_6$-$C_{14}$-Aryl substituiert sind.

Bei den Gruppen $R^1$ handelt es sich bevorzugt um gleiche Reste. Als Substituenten kommen für $R^1$ in Frage: lineares oder verzweigtes Alkyl oder Alkoxy mit 1 bis 18, besonders 1 bis 12 und insbesondere 1 bis 6 C-Atomen, und Alkenyl mit 2 bis 18, besonders 2 bis 12, und insbesondere 2 bis 6 C-Atomen, wie z.B. Methyl, Ethyl, Propyl, Isopropyl, n-Butyl, tert.-Butyl, Pentyl, Hexyl, Octyl, Decyl, Dodecyl, Tetradecyl, Hexadecyl, Octadecyl und entsprechende Alkenyl- und Alkoxygruppen; Cycloalkyl mit 5 bis 8 Ringkohlenstoffatomen wie z.B. Cyclopentyl, Cyclohexyl, Cycloheptyl, Methylcyclopentyl und Methylcyclohexyl; Aryl mit 6 bis 16 C-Atomen und Aralkyl mit 7 bis 16 C-Atomen wie z.B. Phenyl, Naphthyl, Benzyl und Phenylethyl; Cyano und Halogen, besonders F, Cl und Br; $SiR^4{}_3$ oder $GeR^4{}_3$, worin $R^4$ bevorzugt $C_1$-$C_8$-Alkyl, Cyclohexyl, Phenyl oder Benzyl ist. Beispiele für $R^4$ in der Bedeutung von Alkyl sind Methyl, Ethyl, n- und i-Propyl, n-, i- und t-Butyl, Pentyl, Hexyl, Heptyl und Octyl.

Die Reste $R^1$ können bis zu 5, besonders aber bis zu 3 Substituenten enthalten. Bevorzugt sind beide $R^1$ Cyclopentadienyl$^{\ominus}$ - oder Methylcyclopentadienyl$^{\ominus}$-Reste, insbesondere Cyclopentadienyl$^{\ominus}$-Reste.

X in Formel II enthält in seiner Bedeutung als Alkyliden bevorzugt 2 bis 6 C-Atome. Beispiele für Alkyliden, das gegebenenfalls durch Phenyl substituiert sein kann, und Cycloalkyliden sind Ethyliden, Propyliden, Butyliden, Hexyliden, Phenylmethylen, Diphenylmethylen, Cyclopentyliden und Cyclohexyliden. $R^4$ in der Gruppe X in seiner Bedeutung als Alkyl enthält bevorzugt 1 bis 6 C-Atome, und ist z.B. Methyl, Ethyl, Propyl, Butyl oder Hexyl, und ist in seiner Bedeutung als Cycloalkyl bevorzugt Cyclopentyl oder Cyclohexyl, in seiner Bedeutung als Aryl bevorzugt Phenyl und in seiner Bedeutung als Aralkyl bevorzugt Benzyl. X in der Bedeutung von

$$-(CH_2)_n-$$

ist bevorzugt Methylen.

Bei $R^2$ in seiner Bedeutung als 6-gliedriger carbocyclischer aromatischer und fluorsubstituierter Rest handelt es sich um fluorsubstituiertes Phenyl, $R^2$ als heterocyclischer aromatischer und 5-gliedriger Rest enthält bevorzugt ein Heteroatom und als 6-gliedriger Rest bevorzugt 1 oder 2 Heteroatome. Bevorzugt sind beide Orthostellungen mit Fluor substituiert. Beispiele sind 4,6-Difluorinden-5-yl, 5,7-Difluorindan-6-yl, 2,4-Difluorfluoren-3-yl, 1,3-Difluornaphth-2-yl, 2,6-Difluorphen-1-yl, 2,4-Difluorpyrr-3-yl, 2,4-Difluorfur-3-yl, 2,4-Difluorthien-3-yl, 2,4-Difluorpyrrid-3-yl, 4,6-Difluorpyrimidin-5-yl, 3,5-Difluorpyridazin-4-yl.

$R^2$ und $R^3$ zusammen als Rest der Formel III können z.B. die Gruppe

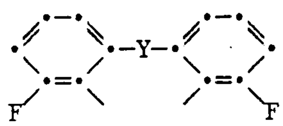

sein. Y in Formel III und in obiger Formel ist bevorzugt Methylen, Ethyliden, Propyliden, S oder O.

$R^3$ hat bevorzugt die Bedeutung von $R^2$. In einer bevorzugten Ausführungsform ist der Rest $R^2$ in beiden Orthostellungen durch Fluor substituiert.

Eine bevorzugte Ausführungsform ist dadurch gekennzeichnet, dass $R^2$ und $R^3$ für 2,6-Difluorphen-1-yl stehen, an das ein Rest der Formel IV, IVa oder IVb gebunden ist, und das weitere 1 oder 2 gleiche oder verschiedene Substituenten enthalten kann.

Eine bevorzugte Gruppe von Titanocenen der Formel I sind solche, worin beide $R^1$ Cyclopentadienyl$^{\ominus}$ und $R^2$ und $R^3$ Reste der Formel V

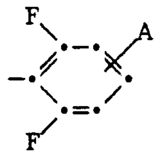

V

sind, worin A eine Gruppe der Formel IV, IVa oder IVb bedeutet, insbesondere solche, worin A eine Gruppe der Formel IV ist.

In Formel V ist die Gruppe A bevorzugt in Orthostellung zu einem F-Atom gebunden.

$R^5$ kann substituiert sein durch $C_1$-$C_{18}$-Alkoxy, $C_1$-$C_{18}$-Alkylthio und $C_1$-$C_{18}$-Alkylsulfonyl, die vorzugsweise 1 bis 12, besonders 1 bis 6 und insbesondere 1 bis 4 C-Atome enthalten. Beispiele für Alkylgruppen in diesen Substituenten sind Methyl, Ethyl und die Isomeren von Propyl, Butyl, Pentyl, Hexyl, Heptyl, Octyl, Nonyl, Decyl, Undecyl, Dodecyl, Tetradecyl, Hexadecyl und Octadecyl. Weitere Substituenten für $R^5$ sind Arylsulfonyl und Alkarylsulfonyl, wie z.B. Phenylsulfonyl, Tolylsulfonyl oder p-Dodecylphenylsulfonyl.

Bei $R^5$ kann es sich um lineares oder verzweigtes $C_1$-$C_{20}$-, bevorzugt $C_1$-$C_{12}$- und besonders $C_1$-$C_8$-Alkyl handeln. Beispiele sind Methyl, Ethyl und die Isomeren von Propyl, Butyl, Pentyl, Hexyl, Heptyl, Octyl, Nonyl, Decyl, Undecyl, Dodecyl, Tetradecyl, Hexadecyl und Octadecyl. $R^5$ kann $C_3$-$C_8$-, bevorzugt $C_5$ bis $C_7$- und besonders $C_5$- oder $C_6$-Cycloalkyl sein, z.B. Cyclopropyl, Cyclobutyl, Cyclopentyl, Cyclohexyl, Cycloheptyl und Cyclooctyl. $R^5$ kann $C_4$-$C_{20}$-, bevorzugt $C_6$-$C_{15}$- Cycloalkylalkyl oder -Alkylcycloalkyl sein, wobei das Cycloalkyl vorzugsweise Cyclopentyl oder Cyclohexyl ist. Beispiele sind Cyclopentyl- oder Cyclohexylmethyl, Cyclopentyl- oder Cyclohexylethyl, Cyclopentyl- oder Cyclohexylpropyl, Cyclopentyl- oder Cyclohexylbutyl, Methyl-, Dimethyl-, Ethyl-, n-Propyl-, i-Propyl-, n-Butyl-, i-Butyl-, t-Butylcyclopentyl oder -cyclohexyl. $R^5$ kann $C_5$-$C_{20}$-, bevorzugt $C_7$-$C_{16}$-Alkylcycloalkylalkyl bedeuten, z.B. (Methylcyclopentyl)methyl oder -ethyl, (Methylcyclohexyl)methyl oder -ethyl.

Bei $R^5$ kann es sich auch um $C_6$-$C_{14}$-, bevorzugt $C_6$-$C_{10}$-Aryl handeln, z.B. Naphthyl und besonders Phenyl. $R^5$ kann auch $C_7$-$C_{20}$-, bevorzugt $C_7$-$C_{16}$-Aralkyl oder -Alkaryl sein. Das Aryl ist hierbei bevorzugt ein Phenylrest. Beispiele sind Benzyl, Phenylethyl, Phenylpropyl, Phenylbutyl, Methylphenyl, Ethylphenyl, Propylphenyl und Butylphenyl. Bei $R^5$ kann es sich auch um $C_8$-$C_{20}$-, bevorzugt $C_8$-$C_{16}$-Alkaralkyl handeln, worin das Aryl bevorzugt Phenyl ist. Beispiele sind Methylbenzyl, (Methylphenyl)ethyl, (Methylphenyl)-propyl, (Methylphenyl)butyl, Ethylbenzyl und Propylbenzyl.

$R^6$ kann eine der für $R^5$ gegebenen Bedeutungen haben, einschliesslich der Bevorzugungen für $R^5$. Bei $R^6$ kann es sich um $C_1$-$C_{20}$-, bevorzugt $C_1$-$C_{12}$- und besonders $C_1$-$C_6$-Halogenalkyl handeln, wobei die Alkylgruppe teilweise oder ganz mit Halogen, bevorzugt Cl und/oder F substituiert sein kann. Beispiele sind Chlormethyl, Dichlormethyl, Trichlormethyl, Fluordichlormethyl, Difluorchlormethyl, Trifluormethyl, 2,2-Dichlor- oder -2,2-Difluorethyl, 1,1,1-Trichlor- oder -Trifluorethyl, Pentafluorethyl, Chlorpropyl, Fluorpropyl, Perfluorpropyl, Chlorbutyl, Fluorbutyl, Perfluorbutyl, Chlorpentyl, Perfluorpentyl und Perfluorhexyl.

Bei $R^6$ und $R^5$ kann es sich um lineares oder verzweigtes $C_2$-$C_{20}$-, bevorzugt $C_2$-$C_{12}$- und besonders $C_2$-$C_6$-Alkenyl handeln. Beispiele sind Vinyl, Crotonyl, Allyl, But-1-en-1-yl, But-1-en-4-yl, Pent-1-en-1-yl, Pent-2-en-2-yl, Hex-1-en-yl, Hex-3-en-3-yl und Hex-1-en-6-yl. $R^6$ kann auch mit -CO- unterbrochenes $C_2$-$C_{20}$-, bevorzugt $C_2$-$C_{12}$- und besonders $C_2$-$C_6$-Alkyl sein, z.B. Acetylmethyl, Propionylmethyl, Acetylethyl

und Propionylethyl.

$R^6$ kann auch die Gruppe $NR^7R^8$ bedeuten, wenn $Y^1$ $-SO_2-$, -CO- oder -CS- ist, worin $R^7$ und $R^8$ unabhängig voneinander eine der für $R^5$ gegebenen Bedeutungen haben, einschliesslich bevorzugter Ausführungsformen. Bevorzugt stehen $R^7$ und $R^8$ für ein Wasserstoffatom oder $C_1$-$C_{12}$-, besonders $C_1$-$C_6$-Alkyl, z.B. Hexyl, Pentyl, Butyl, Propyl und besonders Ethyl oder Methyl.

$R^5$ und $R^6$ zusammen können gegebenenfalls mit Halogen substituiertes $C_2$-$C_8$-Alkylen sein, z.B. 1,2-Ethylen, 1,3-Propylen, 1,4-Butylen, 1-Dimethylethylen, 1-Methyl-1-chlormethylethylen oder 1-Diethylethylen.

$Y^1$ ist bevorzugt -CO-, -COO- oder $-SO_2-$. $R^4$ in der Gruppe $-SiR^4{}_2$ bedeutet besonders Methyl.

Eine bevorzugte Ausführungsform ist dadurch gekennzeichnet, dass $R^2$ und $R^3$ durch eine Gruppe der Formel IV substituiert sind, worin $R^5$ Wasserstoff, unsubstituiertes oder durch $C_1$-$C_{12}$-Alkoxy oder Tetrahydrofuryl substituiertes $C_1$-$C_{12}$-Alkyl, $C_2$-$C_5$-Alkenyl, $C_5$-$C_7$-Cycloalkyl, $C_6$-$C_{18}$-Cycloalkylalkyl oder -Alkylcycloalkyl, $C_7$-$C_{18}$-Alkylcycloalkylalkyl, $C_7$-$C_{16}$-Aralkyl oder $C_8$-$C_{16}$-Alkaralkyl bedeutet, $R^6$ eine der für $R^5$ gegebenen Bedeutungen hat oder $C_6$-$C_{10}$-Aryl, $C_7$-$C_{18}$-Alkaryl, $C_1$-$C_{12}$-Halogenalkyl, oder $-NR^7R^8$ darstellt, worin $R^7$ und $R^8$ unabhängig voneinander Wasserstoff, $C_1$-$C_{12}$-Alkyl, Phenyl, Benzyl oder Cyclohexyl bedeuten oder $R^7$ und $R^8$ zusammen $C_4$-$C_5$-Alkylen oder 3-Oxapentamethylen bedeuten oder $R^5$ und $R^6$ zusammen $C_2$-$C_8$-Alkylen bedeuten und $Y^1$ -CO-, -CS-, -COO- oder $-SO_2-$ bedeutet.

Eine weitere bevorzugte Klasse von Titanocenen sind die Verbindungen der Formel I, worin $R^2$ und $R^3$ durch eine Gruppe der Formel IV substituiert sind, worin $R^5$ Wasserstoff, $C_1$-$C_{12}$-Alkyl, Cyclohexyl, Cyclohexylmethyl, 2-Tetrahydrofurylmethyl, $C_2$-$C_8$-Alkoxyalkyl, Allyl oder $C_7$-$C_9$-Aralkyl ist, $R^6$ $C_1$-$C_{18}$-Alkyl, $C_1$-$C_4$-Halogenalkyl, Cyclohexyl, $C_6$-$C_{10}$-Aryl oder -Halogenaryl oder $C_7$-$C_{18}$-Alkaryl bedeutet oder $R^5$ und $R^6$ zusammen $C_2$-$C_6$-Alkylen bedeuten und $Y^1$ -CO-, -COO- oder $-SO_2-$ ist oder der Rest $-Y^1$-$R^6$ eine Gruppe $-CO-NHR^7$, $-CS-NHR^7$, $-CO-NR^7R^8$ oder $-SO_2-NR^7R^8$ bedeutet, worin $R^7$ $C_1$-$C_{12}$-Alkyl oder Phenyl ist, $R^8$ $C_1$-$C_{12}$-Alkyl ist oder $R^7$ und $R^8$ zusammen $C_4$-$C_5$-Alkylen oder 3-Oxapentamethylen bedeuten, insbesondere solche Verbindungen der Formel I mit der Gruppe der Formel IV, worin $R^5$ Wasserstoff, $C_1$-$C_8$-Alkyl oder $C_7$-$C_9$-Aralkyl ist, $R^6$ $C_1$-$C_{18}$-Alkyl, Trifluormethyl, Phenyl oder durch Halogen oder $C_1$-$C_{12}$-Alkyl substituiertes Phenyl bedeutet oder $R^5$ und $R^6$ zusammen $C_2$-$C_6$-Alkylen bedeuten und $Y^1$ -CO- oder $-SO_2-$ ist.

Eine weitere bevorzugte Klasse von Titanocenen sind die Verbindungen der Formel I, worin $R^2$ und $R^3$ durch eine Gruppe der Formel IVa substituiert sind, worin $R^6$ und $R^{10}$ zusammen $C_2$-$C_8$-Alkandiyl, $C_2$-$C_8$-Alkendiyl, $C_6$-$C_{14}$-Arendiyl oder $C_7$-$C_{12}$-Bicycloalkendiyl bedeuten und $Y^1$ -CO- ist.

Beispiele für einzelne Verbindungen der Formel I sind:

Bis(cyclopentadienyl)-bis[2,6-difluor-3-(N-ethyl-pivaloylamino)phenyl]-titan,
Bis(cyclopentadienyl)-bis[2,6-difluor-3-(N-butyl-pivaloylamino)phenyl]-titan,
Bis(cyclopentadienyl)-bis[2,6-difluor-3-(N-ethyl-acetylamino)phenyl]-titan,
Bis(cyclopentadienyl)-bis[2,6-difluor-3-(N-methyl-acetylamino)phenyl]-titan,
Bis(cyclopentadienyl)-bis[2,6-difluor-3-(N-ethyl-propionylamino)phenyl]-titan,
Bis(cyclopentadienyl)-bis[2,6-difluor-3-(N-ethyl-(2,2-dimethylbutanoyl)-amino)phenyl]titan,
Bis(cyclopentadienyl)-bis[2,6-difluor-3-(N-butyl-(2,2-dimethylbutanoyl)amino)phenyl]-titan,
Bis(cyclopentadienyl)-bis[2,6-difluor-3-(N-pentyl-(2,2-dimethylbutanoyl)amino)phenyl]-titan,
Bis(cyclopentadienyl)-bis[2,6-difluor-3-(N-hexyl-(2,2-dimethylbutanoyl)amino)phenyl]-titan,
Bis(cyclopentadienyl)-bis[2,6-difluor-3-(N-methyl-butyrylamino)phenyl]-titan,
Bis(cyclopentadienyl)-bis[2,6-difluor-3-(N-methyl-pentanoylamino)phenyl]-titan,
Bis(cyclopentadienyl)-bis[2,6-difluor-3-(N-methyl-cyclohexylcarbonylamino)phenyl]-titan,
Bis(cyclopentadienyl)-bis[2,6-difluor-3-(N-ethyl-isobutyrylamino)phenyl]-titan,
Bis(cyclopentadienyl)-bis[2,6-difluor-4-(N-ethyl-acetylamino)phenyl]-titan,
Bis(cyclopentadienyl)-bis[2,6-difluor-3-(2,2,5,5-tetramethyl-1,2,5-azadisilolidin-1-yl)-phenyl]-titan,
Bis(cyclopentadienyl)-bis[2,6-difluor-3-(methylsulfonamido)phenyl]-titan,
Bis(cyclopentadienyl)-bis[2,6-difluor-3-(octylsulfonamido)phenyl]-titan,
Bis(cyclopentadienyl)-bis[2,6-difluor-3-(4-tolylsulfonamido)phenyl]-titan,
Bis(cyclopentadienyl)-bis[2,6-difluor-3-(4-dodecylphenylsulfonylamido)phenyl]-titan,
Bis(cyclopentadienyl)-bis[2,6-difluor-3-(4-(1-pentylheptyl)phenylsulfonylamido)phenyl]-titan,
Bis(cyclopentadienyl)-bis[2,6-difluor-3-(ethylsulfonylamido)phenyl]-titan,
Bis(cyclopentadienyl)-bis[2,6-difluor-3-((4-bromphenyl)sulfonylamido)phenyl]-titan,
Bis(cyclopentadienyl)-bis[2,6-difluor-3-(2-naphthylsulfonylamido)phenyl]-titan,
Bis(cyclopentadienyl)-bis[2,6-difluor-3-(hexadecylsulfonylamido)phenyl]-titan,
Bis(cyclopentadienyl)-bis[2,6-difluor-3-(N-methyl-(4-dodecylphenyl)sulfonylamido)phenyl]-titan,
Bis(cyclopentadienyl)-bis[2,6-difluor-3-(N-methyl-(4-(1-pentylheptyl)phenyl)sulfonylamido)phenyl]-titan,
Bis(cyclopentadienyl)-bis[2,6-difluor-3-(N-hexyl-(4-tolyl)sulfonylamido)phenyl]-titan,

Bis(cyclopentadienyl)-bis[2,6-difluor-3-(pyrrolidin-2,5-dion-1-yl)phenyl]-titan,
Bis(cyclopentadienyl)-bis[2,6-difluor-3-(3,4-dimethyl-3-pyrrolin-2,5-dion-1-yl)phenyl]-titan,
Bis(cyclopentadienyl)-bis[2,6-difluor-3-phthalimido)phenyl]-titan,
Bis(cyclopentadienyl)-bis[2,6-difluor-3-(isobutoxycarbonylamino)phenyl]-titan,
Bis(cyclopentadienyl)-bis[2,6-difluor-3-(ethoxycarbonylamino)phenyl]-titan,
Bis(cyclopentadienyl)-bis[2,6-difluor-3-((2-chlorethoxy)carbonylamino)phenyl]-titan,
Bis(cyclopentadienyl)-bis[2,6-difluor-3-(phenoxycarbonylamino)phenyl]-titan,
Bis(cyclopentadienyl)-bis[2,6-difluor-3-(3-phenylthioureido)phenyl]-titan,
Bis(cyclopentadienyl)-bis[2,6-difluor-3-(3-butylthioureido)phenyl]-titan,
Bis(cyclopentadienyl)-bis[2,6-difluor-3-(3-phenylureido)phenyl]-titan,
Bis(cyclopentadienyl)-bis[2,6-difluor-3-(3-butylureido)phenyl]-titan,
Bis(cyclopentadienyl)-bis[2,6-difluor-3-(N,N-diacetylamino)phenyl]-titan,
Bis(cyclopentadienyl)-bis[2,6-difluor-(3,3-dimethylureido)phenyl]-titan,
Bis(cyclopentadienyl)-bis[2,6-difluor-3-(acetylamino)phenyl]-titan,
Bis(cyclopentadienyl)-bis[2,6-difluor-3-(butyrylamino)phenyl]-titan,
Bis(cyclopentadienyl)-bis[2,6-difluor-3-(decanoylamino)phenyl]-titan,
Bis(cyclopentadienyl)-bis[2,6-difluor-3-(octadecanoylamino)phenyl]-titan,
Bis(cyclopentadienyl)-bis[2,6-difluor-3-(isobutyrylamino)phenyl]-titan,
Bis(cyclopentadienyl)-bis[2,6-difluor-3-(2-ethylhexanoylamino)phenyl]-titan,
Bis(cyclopentadienyl)-bis[2,6-difluor-3-(2-methylbutanoylamino)phenyl]-titan,
Bis(cyclopentadienyl)-bis[2,6-difluor-3-(pivaloylamino)phenyl]-titan,
Bis(cyclopentadienyl)-bis[2,6-difluor-3-(2,2-dimethylbutanoylamino)phenyl]-titan,
Bis(cyclopentadienyl)-bis[2,6-difluor-3-(2-ethyl-2-methylheptanoylamino)phenyl]-titan,
Bis(cyclopentadienyl)-bis[2,6-difluor-3-(cyclohexylcarbonylamino)phenyl]-titan,
Bis(cyclopentadienyl)-bis[2,6-difluor-3-(2,2-dimethyl-3-chlorpropanoylamino)phenyl]-titan,
Bis(cyclopentadienyl)-bis[2,6-difluor-3-(3-phenylpropanoylamino)phenyl]-titan,
Bis(cyclopentadienyl)-bis[2,6-difluor-3-(2-chlormethyl-2-methyl-3-chlorpropanoylamino)phenyl]-titan,
Bis(cyclopentadienyl)-bis[2,6-difluor-3-(3,4-xyloylamino)phenyl]-titan,
Bis(cyclopentadienyl)-bis[2,6-difluor-3-(4-ethylbenzoylamino)phenyl]-titan,
Bis(cyclopentadienyl)-bis[2,6-difluor-3-(2,4,6-mesitylcarbonylamino)phenyl]-titan,
Bis(cyclopentadienyl)-bis[2,6-difluor-3-(benzoylamino)phenyl]-titan,
Bis(cyclopentadienyl)-bis[2,6-difluor-3-(N-(3-phenylpropyl)-benzoylamino)phenyl]-titan,
Bis(cyclopentadienyl)-bis[2,6-difluor-3-(N-(3-ethylheptyl)-2,2-dimethylpentanoylamino)phenyl-titan,
Bis(cyclopentadienyl)-bis[2,6-difluor-3-(N-isobutyl-(4-toluyl)amino)phenyl]titan,
Bis(cyclopentadienyl)-bis[2,6-difluor-3-(N-isobutyl-benzoylamino)phenyl]-titan,
Bis(cyclopentadienyl)-bis[2,6-difluor-3-(N-cyclohexylmethyl-pivaloylamino)phenyl]-titan,
Bis(cyclopentadienyl)-bis[2,6-difluor-3-(N-(oxolan-2-ylmethyl)-benzoylamino)phenyl]-titan,
Bis(cyclopentadienyl)-bis[2,6-difluor-3-(N-(3-ethylheptyl)-2,2-dimethylbutanoylamino]phenyl-titan,
Bis(cyclopentadienyl)-bis[2,6-difluor-3-(N-(3-phenylpropyl)-(4-toluyl)amino)phenyl]-titan,
Bis(cyclopentadienyl)-bis[2,6-difluor-3-(N-(oxolan-2-ylmethyl)-(4-toluyl)amino)phenyl]-titan,
Bis(cyclopentadienyl)-bis[2,6-difluor-3-(N-(4-tolylmethyl)-benzoylamino)phenyl]-titan,
Bis(cyclopentadienyl)-bis[2,6-difluor-3-(N-(4-tolylmethyl)-(4-toluyl)amino)phenyl]-titan,
Bis(cyclopentadienyl)-bis[2,6-difluor-3-(N-butyl-benzoylamino)phenyl]-titan,
Bis(cyclopentadienyl)-bis[2,6-difluor-3-(N-butyl-(4-toluyl)amino)phenyl]-titan,
Bis(cyclopentadienyl)-bis[2,6-difluor-3-(N-hexyl-(4-toluyl)-amino)phenyl]-titan,
Bis(cyclopentadienyl)-bis[2,6-difluor-3-(N-(2,4-dimethylpentyl)-2,2-dimethylbutanoylamino)phenyl]-titan,
Bis(cyclopentadienyl)-bis[2,6-difluor-3-(N-(2,4-dimethylpentyl)-2,2-dimethylpentanoylamino)phenyl]-titan,
Bis(cyclopentadienyl)-bis[2,6-difluor-3-((4-toluyl)amino)phenyl]-titan,
Bis(cyclopentadienyl)-bis[2,6-difluor-3-(2,2-dimethylpentanoylamino)phenyl]-titan,
Bis(cyclopentadienyl)-bis[2,6-difluor-3-(2,2-dimethyl-3-ethoxypropanoylamino)phenyl]-titan,
Bis(cyclopentadienyl)-bis[2,6-difluor-3-(2,2-dimethyl-3-allyloxypropanoylamino)phenyl]-titan,
Bis(cyclopentadienyl)-bis[2,6-difluor-3-(N-allyl-acetylamino)phenyl]-titan,
Bis(cyclopentadienyl)-bis[2,6-difluor-3-(2-ethylbutanoylamino)phenyl]-titan,
Bis(cyclopentadienyl)-bis[2,6-difluor-3-(N-cyclohexylmethyl-benzoylamino)phenyl]-titan,
Bis(cyclopentadienyl)-bis[2,6-difluor-(N-cyclohexylmethyl-(4-toluyl)-amino)phenyl]-titan,
Bis(cyclopentadienyl)-bis[2,6-difluor-3-(N-(2-ethylhexyl)-benzoylamino)phenyl]-titan,
Bis(cyclopentadienyl)-bis[2,6-difluor-3-(N-isopropyl-benzoylamino)phenyl]-titan,
Bis(cyclopentadienyl)-bis[2,6-difluor-3-(N-(3-phenylpropyl)-2,2-dimethylpentanoyl)amino)phenyl]-titan,

Bis(cyclopentadienyl)-bis[2,6-difluor-3-(N-hexyl-benzoylamino)phenyl]-titan,
Bis(cyclopentadienyl)-bis[2,6-difluor-3-(N-cyclohexylmethyl-2,2-dimethylpentanoyl)amino)phenyl]-titan,
Bis(cyclopentadienyl)-bis[2,6-difluor-3-(N-butyl-benzoylamino)phenyl]-titan,
Bis(cyclopentadienyl)-bis[2,6-difluor-3-(N-(2-ethylhexyl)-2,2-dimethylpentanoyl)amino)phenyl]-titan,
Bis(cyclopentadienyl)-bis[2,6-difluor-3-(N-hexyl-2,2-dimethylpentanoylamino)phenyl]-titan,
Bis(cyclopentadienyl)-bis[2,6-difluor-3-(N-isopropyl-2,2-dimethylpentanoylamino)phenyl]-titan,
Bis(cyclopentadienyl)-bis[2,6-difluor-3-(N-(3-phenylpropyl)-pivaloylamino)phenyl]-titan,
Bis(cyclopentadienyl)-bis[2,6-difluor-3-(N-butyl-2,2-dimethylpentanoylamino)phenyl]-titan,
Bis(cyclopentadienyl)-bis[2,6-difluor-3-(N-(2-methoxyethyl)benzoylamino)phenyl]-titan,
Bis(cyclopentadienyl)-bis[2,6-difluor-3-(N-benzyl-benzoylamino)phenyl]-titan,
Bis(cyclopentadienyl)-bis[2,6-difluor-3-(N-benzyl-(4-toluyl)amino)phenyl]-titan,
Bis(cyclopentadienyl)-bis[2,6-difluor-3-(N-(2-methoxyethyl)-(4-toluyl)amino)phenyl]-titan,
Bis(cyclopentadienyl)-bis[2,6-difluor-3-(N-(4-methylphenylmethyl)-2,2-dimethylpentanoylamino)phenyl]-titan,
Bis(cyclopentadienyl)-bis[2,6-difluor-3-(N-(2-methoxyethyl)-2,2-dimethylpentanoylamino)phenyl]-titan,
Bis(cyclopentadienyl)-bis[2,6-difluor-3-(N-cyclohexylmethyl-(2-ethyl-2-methylheptanoyl)amino)phenyl]-titan,
Bis(cyclopentadienyl)-bis[2,6-difluor-3-(N-butyl-(4-chlorbenzoyl)amino)phenyl]-titan,
Bis(cyclopentadienyl)-bis[2,6-difluor-3-(N-hexyl-(2-ethyl-2-methylbutanoyl)amino)phenyl]-titan,
Bis(cyclopentadienyl)-bis[2,6-difluor-3-(N-cyclohexyl-2,2-dimethylpentanoyl)amino)phenyl]-titan,
Bis(cyclopentadienyl)-bis[2,6-difluor-3-(N-(oxolan-2-ylmethyl)-2,2-dimethylpentanoyl)amino)phenyl]-titan,
Bis(cyclopentadienyl)-bis[2,6-difluor-3-[N-cyclohexyl-(4-chlorbenzoyl)amino)phenyl]-titan,
Bis(cyclopentadienyl)-bis[2,6-difluor-3-(N-cyclohexyl-(2-chlorbenzoyl)amino)phenyl]-titan,
Bis(cyclopentadienyl)-bis[2,6-difluor-3-(3,3-dimethyl-2-azetidinon-1-yl)phenyl]-titan,
Bis(cyclopentadienyl)-bis(2,6-difluor-3-isocyanatophenyl)-titan,
Bis(cyclopentadienyl)-bis[2,6-difluor-3-(N-ethyl-(4-tolylsulfonyl)amino)phenyl]-titan,
Bis(cyclopentadienyl)-bis[2,6-difluor-3-(N-hexyl-(4-tolylsulfonyl)amino)phenyl]-titan,
Bis(cyclopentadienyl)-bis[2,6-difluor-3-(N-butyl-(4-tolylsulfonyl)amino)phenyl]-titan,
Bis(cyclopentadienyl)-bis[2,6-difluor-3-(N-isobutyl-(4-tolylsulfonyl)amino)phenyl]-titan,
Bis(cyclopentadienyl)-bis[2,6-difluor-3-(N-butyl-(2,2-dimethyl-3-chlorpropanoyl)amino)phenyl]-titan,
Bis(cyclopentadienyl)-bis[2,6-difluor-3-(N-(3-phenylpropyl)-(2,2-dimethyl-3-chlorpropanoyl)amino)phenyl]-titan,
Bis(cyclopentadienyl)-bis[2,6-difluor-3-(N-cyclohexylmethyl-(2,2-dimethyl-3-chlorpropanoyl)amino)phenyl]-titan,
Bis(cyclopentadienyl)-bis[2,6-difluor-3-(N-isobutyl-(2,2-dimethyl-3-chlorpropanoyl)amino)phenyl]-titan,
Bis(cyclopentadienyl)-bis[2,6-difluor-3-(N-butyl-(2-chlormethyl-2-methyl-3-chlorpropanolyl)amino)phenyl]-titan,
Bis(cyclopentadienyl)-bis[2,6-difluor-3-(butylthiocarbonylamino)phenyl]-titan,
Bis(cyclopentadienyl)-bis[2,6-difluor-3-(phenylthiocarbonylamino)phenyl]-titan,
Bis(methylcyclopentadienyl)-bis[2,6-difluor-3-(N-hexyl-2,2-dimethylbutanoyl)amino)phenyl]-titan,
Bis(methylcyclopentadienyl)-bis[2,6-difluor-3-(N-hexyl-2,2-dimethylpentanoylamino)phenyl]-titan,
Bis(methylcyclopentadienyl)-bis[2,6-difluor-3-(N-ethyl-acetylamino)phenyl]-titan,
Bis(methylcyclopentadienyl)-bis[2,6-difluor-3-(N-ethylpropionylamino)phenyl]-titan,
Bis(trimethylsilylpentadienyl)-bis[2,6-difluor-3-(N-butyl-2,2-dimethylpropanoylamino)phenyl]-titan,
Bis(cyclopentadienyl)-bis[2,6-difluor-3-(N-(2-methoxyethyl)-trimethylsilylamino)phenyl]-titan,
Bis(cyclopentadienyl)-bis[2,6-difluor-3-(N-butyl-hexyldimethylsilylamino)phenyl]-titan,
Bis(cyclopentadienyl)-bis[2,6-difluor-3-(N-ethyl-(1,1,2-trimethylpropyl)dimethylsilylamino)phenyl]-titan,
Bis(cyclopentadienyl)-bis[2,6-difluor-3-(3-ethoxymethyl)-3-methyl-2-azetidinon-1-yl)phenyl]-titan,
Bis(cyclopentadienyl)-bis[2,6-difluor-3-(3-allyloxymethyl)-3-methyl-2-azetidinon-1-yl)phenyl]-titan,
Bis(cyclopentadienyl)-bis[2,6-difluor-3-(3-chlormethyl-3-methyl-2-azetidinon-1-yl)phenyl]-titan,
Bis(cyclopentadienyl)-bis[2,6-difluor-3-(N-benzyl-2,2-dimethylpropanoylamino)phenyl]-titan,
Bis(cyclopentadienyl)-bis[2,6-difluor-3-(5,5-dimethyl-2-pyrrolidinon-1-yl)phenyl]-titan,
Bis(cyclopentadienyl)-bis[2,6-difluor-3-(6,6-diphenyl-2-piperidinon1-yl)phenyl]-titan,
Bis(cyclopentadienyl)-bis[2,6-difluor-3-(N-(2,3-dihydro-1,2-benzisothiazol-3-on-1,1-dioxid-2-yl)phenyl]-titan,
Bis(cyclopentadienyl)-bis[2,6-difluor-3-(N-hexyl-(4-chlorbenzoyl)amino)phenyl]-titan,
Bis(cyclopentadienyl)-bis[2,6-difluor-3-(N-hexyl-(2-chlorbenzoyl)amino)phenyl]-titan,
Bis(cyclopentadienyl)-bis[2,6-difluor-3-(N-isopropyl-(4-chlorbenzoyl)amino)phenyl]-titan,
Bis(cyclopentadienyl)-bis[2,6-difluor-3-(N-(4-methylphenylmethyl)-(4-chlorbenzoyl)amino)phenyl]-titan,
Bis(cyclopentadienyl)-bis[2,6-difluor-3-(N-(4-methylphenylmethyl)-(2-chlorbenzoyl)amino)phenyl]-titan,
Bis(cyclopentadienyl)-bis[2,6-difluor-3-(N-butyl-(4-chlorbenzoyl)amino)phenyl]-titan,

Bis(cyclopentadienyl)-bis[2,6-difluor-3-(N-benzyl-2,2-dimethylpentanoylamino)phenyl]-titan,
Bis(cyclopentadienyl)-bis[2,6-difluor-3-(N-(2-ethylhexyl)-(4-tolylsulfonyl)amino)phenyl]-titan,
Bis(cyclopentadienyl)-bis[2,6-difluor-3-(N-(3-oxaheptyl)-benzoylamino)phenyl]-titan,
Bis(cyclopentadienyl)-bis[2,6-difluor-3-(N-(3,6-dioxadecyl)-benzoylamino)phenyl]-titan,
Bis(cyclopentadienyl)-bis[2,6-difluor-3-(trifluormethylsulfonyl)amino)phenyl]-titan,
Bis(cyclopentadienyl)-bis[2,6-difluor-3-(trifluoracetylamino)phenyl]-titan,
Bis(cyclopentadienyl)-bis[2,6-difluor-3-((2-chlorbenzoyl)amino)phenyl]-titan,
Bis(cyclopentadienyl)-bis[2,6-difluor-3-((4-chlorbenzoyl)amino)phenyl]-titan,
Bis(cyclopentadienyl)-bis[2,6-difluor-3-(N-(3,6-dioxadecyl)-2,2-dimethylpentanoylamino)phenyl]-titan,
Bis(cyclopentadienyl)-bis[2,6-difluor-3-(N-(3,7-dimethyl-7-methoxyoctyl)-benzoylamino)phenyl]-titan,
Bis(cyclopentadienyl)-bis[2,6-difluor-3-(N-cyclohexyl-benzoylamino)phenyl]-titan.

Die Herstellung der Titanocene der Formel I kann nach bekannten oder analogen Verfahren erfolgen, indem man 1 Mol einer Verbindung der Formel VI

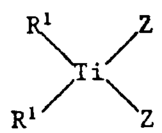 VI,

worin $R^1$ die angegebene Bedeutung hat und Z für Halogen, besonders Chlor, steht, entweder mit einem Mol $LiR^2$ oder $LiR^3$ und danach mit einem Mol $LiR^3$ bzw. $LiR^2$ umsetzt, oder mit 2 Mol $LiR^2$ oder mit 1 Mol $Li_2QYQ$ umsetzt, wobei $R^2$, $R^3$ und QYQ die zuvor angegebenen Bedeutungen haben, und danach die Verbindung der Formel I in an sich bekannter Weise isoliert.

Die bekannten Verfahren sind z.B. in J. Organometal. Chem., 2 (1964) 206-212, J. Organometal. Chem., 4 (1965) 445-446 und in der EP-A-0 122 223 beschrieben.

Die Ausgangsverbindungen der Formel VI, in denen Z besonders für Chlor steht, sind bekannt oder können nach analogen Verfahren durch die Umsetzung von $TiCl_4$ mit 2 Mol einer Natriumverbindung $NaR^1$ erhalten werden.

Die Lithiumverbindungen $LiR^2$, $LiR^3$ und $Li_2QYQ$ sind neu. Sie können nach an sich bekannten Verfahren durch die Umsetzung von z.B. Lithiumbutyl mit Verbindungen der Formel VII oder VIII hergestellt werden.

Ein weiterer Gegenstand der Erfindung sind die Zwischenprodukte der Formel VII

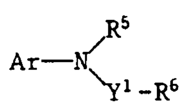 VII,

worin Ar einen 6-gliedrigen carbocyclischen oder 5- oder 6-gliedrigen heterocyclischen aromatischen Rest, der mindestens ein Fluoratom, in Orthostellung hierzu ein Wasserstoffatom oder ein Halogenatom und gegebenenfalls weitere Substituenten enthält, oder Ar einen Rest der Formel

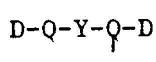 oder 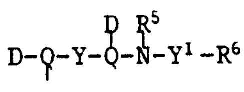

bedeutet, worin D für ein in Orthostellung zu Y gebundenes Wasserstoffatom oder Halogenatom steht, Q einen carbocyclischen aromatischen Rest bedeutet, der in Orthostellung zur D-Gruppe jeweils durch ein Fluoratom substituiert ist und Q weitere Substituenten enthalten kann, und Y, $Y^1$, $R^5$ und $R^6$ die zuvor angegebenen Bedeutungen haben.

Für Ar, $Y^1$, $R^5$, $R^6$, Q und Y gelten sinngemäss die gleichen Ausführungsformen und Bevorzugungen wie sie zuvor für $R^2$ bzw. $R^2$ und $R^3$ zusammen und für den Rest der Formel IV beschrieben sind.

Der Rest Ar in der Bedeutung eines 6-gliedrigen carbocyclischen oder 5- oder 6-gliedrigen heterocyclischen aromatischen Restes enthält vorzugsweise in Orthostellung zum Wasserstoff- bzw. Halogenatom ein

weiteres Fluoratom. Das Halogenatom ist bevorzugt aus F, Cl oder Br ausgewählt. Beim aromatischen Rest handelt es sich vorzugsweise um einen substituierten Phenylrest. In Orthostellung zum Fluoratom bzw. zu Y ist insbesondere ein Wasserstoffatom gebunden. Die Gruppe $-N(R^5)-Y^1R^6$ ist vorzugsweise in Orthostellung zu einem der F-Atome gebunden. Es wurde gefunden, dass überraschend bei solchen Verbindungen das zu den F-Atomen benachbarte H-Atom direkt durch Lithium ersetzt werden kann. Eine bevorzugte Gruppe von Verbindungen sind solche der Formel VIIa

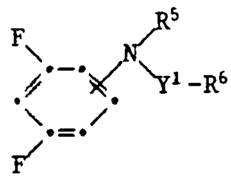

VIIa,

worin $R^5$, $R^6$ und $Y^1$ die zuvor angegebenen Bedeutungen haben. Die Gruppe $-N(R^5)-Y^1R^6$ ist vorzugsweise in Orthostellung zum Fluoratom gebunden.

Die Herstellung der Verbindungen der Formel VII kann durch N-Acylierung nach an sich bekannten Verfahren erfolgen. Man kann z.B. von den entsprechenden primären oder sekundären Fluorarylaminen ausgehen, die teilweise kommerziell erhältlich sind oder nach an sich bekannten Verfahren hergestellt werden können. Sekundäre Amine können auch durch Alkylierung und Aralkylierung von primären Aminen nach bekannten Methoden erhalten werden. Beispielsweise kann man ein primäres Amin mit einem Aldehyd umsetzen und das gebildete Azomethin hydrieren. Man kann auch von einem fluorierten Nitrobenzol ausgehen und durch Hydrierung in Gegenwart eines Aldehydes das monoalkylierte Anilin herstellen.

Die Acylierung ($Y^1$ = -CO-, -CS- oder $-SO_2-$) kann nach bekannten Verfahren durch die Umsetzung der Amine mit Säurehalogeniden, Säureanhydriden oder Säureestern erfolgen. Urethane ($Y^1$ = -COO-) können durch die Umsetzung der Amine mit Chlorkohlensäureestern erhalten werden. Harnstoffe ($Y^1$ = -CO- oder -CS- und $R^6$ = $-NR^7R^8$) können z.B. durch die Umsetzung der Amine mit Isocyanaten, Isothiocyanaten oder Carbamoylhalogeniden hergestellt werden. Silylamine können z.B. durch die Umsetzung eines Amins mit einem entsprechenden Silylhalogenid, $R^6Si(R^4)_2Cl$ erhalten werden.

Gleichzeitige Alkylierung und Acylierung der primären Amine kann durch Reaktion mit Orthocarbonsäureestern geschehen.

Verbindungen der Formel VII, worin $R^5$ und $R^6$ zusammen lineares oder verzweigtes $C_2$-$C_8$-Alkylen oder durch Halogen substituiertes $C_2$-$C_8$-Alkylen oder einen zweiwertigen Rest der Formel

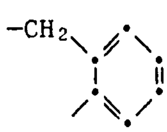

oder

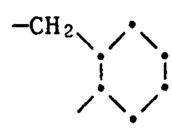

oder

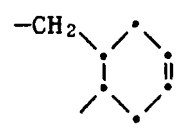

bedeuten, können z.B. aus den primären Fluoranilinen durch Umsetzung mit einem entsprechenden Halogencarbonsäurehalogenid oder Halogensulfonsäurehalogenid hergestellt werden. Beispiele hierfür sind $\beta$-Chlorpropionsäurechlorid, $\beta$-Chlorpivalinsäurechlorid, $\gamma$-Brombutyrylbromid, $\delta$-Bromvalerylbromid, o-Chlormethylbenzoylchlorid oder 2-(Chlormethyl)-cyclohexancarbonylchlorid. Eine weitere Methode ist die Umsetzung der primären Amine mit Lactonen.

Weitere Zwischenprodukte sind die Verbindungen der Formel VIII

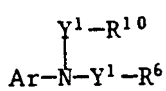

VIII

worin Ar, $Y^1$, $R^6$ und $R^{10}$ die vorhin gegebenen Bedeutungen haben.

Bevorzugt ist Ar ein 1,3-Difluorphenylrest, was den Verbindungen der Formel VIIIa entspricht:

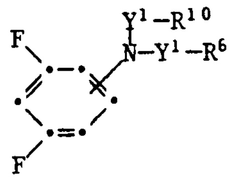

VIIIa

Die Gruppe $-N(Y^1R^{10})-Y^1R^6$ befindet sich bevorzugt in Orthostellung zum Fluoratom.

Bevorzugt sind Verbindungen der Formel VIIIa, worin $Y^1$ die Gruppe -CO- ist und $R^6$ und $R^{10}$ zusammen $C_2$-$C_8$-Alkandiyl, $C_2$-$C_8$-Alkendiyl, $C_6$-$C_{10}$-Arendiyl, $C_6$-$C_{12}$-Cycloalkandiyl, $C_6$-$C_{12}$-Cycloalkendiyl, $C_7$-$C_{12}$-Bicycloalkandiyl oder $C_7$-$C_{12}$-Bicycloalkendiyl bedeuten.

Die Verbindungen der Formel VIII können durch zweifache Acylierung der entsprechenden primären Amine hergestellt werden. Verbindungen der Formel VIII, worin $Y^1$ die Gruppe -CO- ist und $R^6$ und $R^{10}$ zusammen einen zweiwertigen Rest bilden, können durch Umsetzung der primären Amine mit cyclischen 1,2-Dicarbonsäureanhydriden hergestellt werden. Beispiele hierfür sind Bernsteinsäure-, Maleinsäure-, Phthalsäure-, Hexahydrophthalsäure- oder Cyclohexen-4,5-dicarbonsäureanhydrid.

Eine spezielle Type von Verbindungen der Formel VIII bzw. VIIIa sind solche der Formel VIIIb:

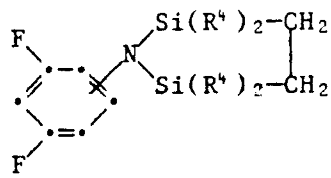

VIIIb

Diese können z.B. aus den entsprechenden primären Aminen durch Umsetzung mit $X^1$-$Si(R^4)_2$-$CH_2$-$CH_2$-$Si(R^4)_2$-$X^1$, worin $X^1$ Chlor oder Dimethylamino ist, hergestellt werden. Die Verbindungen der Formel VIIIb stellen eine maskierte Form der primären Amine dar. Nach ihrer Umsetzung zu den entsprechenden Titanocenen kann die Schutzgruppe $-Si(R^4)_2-CH_2-CH_2-Si(R^4)_2-$ hydrolytisch abgespalten werden unter Zurückbildung der $NH_2$-Gruppe.

Die Herstellung der Titanocene der Formel I erfolgt im allgemeinen in Gegenwart inerter Lösungsmittel wie z.B. Kohlenwasserstoffen oder Ethern, bei Temperaturen von -30 bis -100 °C, vorzugsweise -60 bis -90 °C und unter Schutzgasatmosphäre. In einer Ausführungsform des Verfahrens wird zunächst $LiR^2$ bzw. $LiR^3$ durch Umsetzung der Verbindungen der Formel VII oder VIII in einem Ether als Lösungsmittel, z.B. Tetrahydrofuran, mit Lithiumbutyl bei Temperaturen um -78 °C hergestellt. Zu der gekühlten Reaktionsmischung gibt man dann das entsprechende Titanocendihalogenid, entfernt die Kühlung und lässt auf Raumtemperatur erwärmen. Die Reaktionsmischung wird dann, gegebenenfalls nach Zugabe von Lösungsmitteln, filtriert und aus der Lösung durch Ausfällen oder Verdampfen des Lösungsmittels das erfindungsgemässe Titanocen isoliert.

Verwendet man für die Umsetzung mit Lithiumbutyl und Titanocendichlorid ein maskiertes primäres Amin, beispielsweise eine Verbindung der Formel VIIIb, so kann durch Hydrolyse des gebildeten Titanocens eine Verbindung der Formel I hergestellt werden, in der $R^2$ und $R^3$ durch eine $NH_2$-Gruppe substituiert sind. Diese $NH_2$-Gruppe kann anschliessend durch entsprechende N-Substitution in die Gruppe $-N(R^5)-Y^1R^6$ oder $-N(Y^1R^{10})-Y^1R^6$ übergeführt werden. Hierfür kommen dieselben Verfahren in Frage wie für die Herstellung von VII und VIII. Die $NH_2$-Gruppe kann auch durch Umsetzung mit Phosgen oder Triphosgen in eine Isocyanatgruppe -NCO übergeführt werden, wodurch man zu Verbindungen der Formel I gelangt, in denen $R^2$ und $R^3$ durch einen Rest der Formel IVb substituiert sind.

Bei den Verbindungen der Formel I handelt es sich im allgemeinen um kristalline, meist orangegefärbte, Verbindungen, die sich durch eine hohe thermische Stabilität auszeichnen und sich erst bei hohen Temperaturen zersetzen. Auch unter Lufteinwirkung sowie unter Einwirkung von Wasser wird keine Zersetzung beobachtet. Viele dieser Verbindungen können in härtbaren Zusammensetzungen auch in höheren Mengen gelöst werden, und bieten daher wertvolle anwendungstechnische Eigenschaften. Die Verbindungen sind auch in Lösungsmitteln gut löslich, und können in Form von Lösungen in härtbare Zusammensetzungen eingearbeitet werden, wonach das Lösungsmittel gegebenenfalls entfernt wird.

Die Verbindungen sind dunkellagerstabil und können ohne Schutzgas gehandhabt werden. Sie eignen sich alleine hervorragend als sehr wirksame Photoinitiatoren für die lichtinduzierte Polymerisation ethylenisch ungesättigter Verbindungen. Sie zeichnen sich hierbei durch eine hohe Lichtempfindlichkeit und Wirksamkeit über einen grossen Wellenlängenbereich von ca. 200 nm (UV-Licht) bis etwa 600 nm aus. Ferner vermögen die Titanocene auch wirksam die Polymerisation unter dem Einfluss von Wärme zu initiieren, wobei ein Erwärmen auf 170 °C bis 240 °C zweckmässig ist. Selbstverständlich kann auch Lichteinwirkung in Kombination mit Erwärmung zur Polymerisation benutzt werden, wobei eine Erwärmung nach der Belichtung tiefere Temperaturen, z.B. 80-150 °C, zur Polymerisation erlaubt. Die Lichtempfindlichkeit ist überraschend höher als bei den entsprechenden Dialkylaminderivaten.

Ein weiterer Gegenstand vorliegender Erfindung ist eine durch Strahlung polymerisierbare Zusammensetzung, enthaltend (a) mindestens eine nichtflüchtige, monomere, oligomere oder polymere Verbindung mit mindestens einer polymerisierbaren ethylenisch ungesättigten Doppelbindung und (b) mindestens ein Titanocen der Formel I als Photoinitiator.

Die Zusammensetzungen können weitere von (b) verschiedene Photoinitiatoren (c), z.B. solche vom Typ der Benzophenone, Benzoinalkylether, Benzilketale, 4-Aroyl-1,3-dioxolane, Dialkoxyacetophenone, α-Hydroxy- oder α-Aminoacetophenone, α-Hydroxycycloalkylphenylketone oder Mischungen davon enthalten. Der Vorteil besteht darin, dass man geringere Mengen der erfindungsgemässen Titanocene verwenden kann und trotzdem gleiche oder verbesserte Lichtempfindlichkeiten erzielen kann. Das Gewichtsverhältnis dieser Komponenten (c):(b) kann z.B. von 1:1 bis 30:1, bevorzugt 5:1 bis 15:1 betragen.

Die Zusatzmenge der erfindungsgemässen Titanocene richtet sich im wesentlichen nach wirtschaftlichen Gesichtspunkten, deren Löslichkeiten und nach der gewünschten Empfindlichkeit. Im allgemeinen werden 0,01 bis 20, vorzugsweise 0,05-10 und besonders 0,1 bis 5 Gew.% verwendet, bezogen auf die Komponente (a).

Als Komponente (a) kommen solche ethylenisch ungesättigten monomeren, oligomeren und polymeren Verbindungen in Frage, die durch Photopolymerisation zu höhermolekularen Produkten reagieren und hierbei ihre Löslichkeit verändern.

Besonders geeignet sind z.B. Ester von ethylenisch ungesättigten Carbonsäuren und Polyolen oder Polyepoxiden, und Polymere mit ethylenisch ungesättigten Gruppen in der Kette oder in Seitengruppen, wie z.B. ungesättigte Polyester, Polyamide und Polyurethane und Copolymere hiervon, Polybutadien und Butadien-Copolymere, Polyisopren und Isopren-Copolymere, Polymere und Copolymere mit (Meth)-Acrylgruppen in Seitenketten, sowie Mischungen von einem oder mehreren solcher Polymere.

Beispiele für ungesättigte Carbonsäuren sind Acrylsäure, Methacrylsäure, Crotonsäure, Itaconsäure, Zimtsäure, ungesättigte Fettsäuren wie Linolensäure oder Oelsäure. Bevorzugt sind Acryl- und Methacrylsäure.

Als Polyole sind aromatische und besonders aliphatische und cycloaliphatische Polyole geeignet. Beispiele für aromatische Polyole sind Hydrochinon, 4,4'-Dihydroxydiphenyl, 2,2-Di(4-hydroxyphenyl)-propan, sowie Novolake und Resole. Beispiele für Polyepoxide sind solche auf der Basis der genannten Polyole, besonders der aromatischen Polyole und Epichlorhydrin. Ferner sind auch Polymere oder Copolymere, die Hydroxylgruppen in der Polymerkette oder in Seitengruppen enthalten, wie z.B. Polyvinylalkohol und Copolymere davon oder Polymethacrylsäurehydroxyalkylester oder Copolymere davon, als Polyole geeignet. Weitere geeignete Polyole sind Oligoester mit Hydroxylendgruppen.

Beispiele für aliphatische und cycloaliphatische Polyole sind Alkylendiole mit bevorzugt 12 bis 12 C-Atomen, wie Ethylenglykol, 1,2- oder 1,3-Propandiol, 1,2-, 1,3 oder 1,4-Butandiol, Pentandiol, Hexandiol, Octandiol, Dodecandiol, Diethylenglykol, Triethylenglykol, Polyethylenglykole mit Molekulargewichten von bevorzugt 200 bis 1500, 1,3-Cyclopentandiol, 1,2-, 1,3- oder 1,4-Cyclohexandiol, 1,4-Dihydroxymethylcyclohexan, Glycerin, Tris-(β-hydroxyethyl)amin, Trimethylolethan, Trimethylolpropan, Pentaerythrit, Dipentaerythrit und Sorbit.

Die Polyole können teilweise oder vollständig mit einer oder verschiedenen ungesättigten Carbonsäuren verestert sein, wobei in Teilestern die freien Hydroxylgruppen modifiziert, z.B. verethert oder mit anderen Carbonsäuren verestert sein können.

Beispiele für Ester sind:

Trimethylolpropantriacrylat, Trimethylolethantriacrylat, Trimethylolpropantrimethacrylat, Trimethylolethantrimethacrylat, Tetramethylenglykoldimethacrylat, Triethylenglykoldimethacrylat, Tetraethylenglykoldiacrylat, Pentaerythritdiacrylat, Pentaerythrittriacrylat, Pentaerythrittetraacrylat, Dipentaerythritdiacrylat, Dipentaerythrittriacrylat, Dipentaerythrittetraacrylat, Dipentaerythritpentaacrylat, Dipentaerythrithexaacrylat, Tripentaerythritoctaacrylat, Pentaerythritdimethacrylat, Pentaerythrittrimethacrylat, Dipentaerythritdimethacrylat, Dipentaerythrittetramethacrylat, Tripentaerythritoctamethacrylat, Pentaerythritdiitaconat, Dipentaerythrittrisitaconate, Dipentaerythritpentaitaconat, Dipentaerythrithexaitaconat, Ethylenglykoldimethacrylat, 1,3-Butandioldiacrylat,

1,3-Butandioldimethacrylat, 1,4-Butandioldiitaconat, Sorbittriacrylat, Sorbittetraacrylat, Sorbittetramethacrylat, Sorbitpentaacrylat, Sorbithexaacrylat, Oligoesteracrylate und -methacrylate, Glyzerindi- und -triacrylat, 1,4-Cyclohexandiacrylat, Bisacrylate und Bismethacrylate von Polyethylenglykol mit Molekulargewicht von 200-1500, oder Gemische davon.

Als Komponente (a) sind auch die Amide gleicher oder verschiedener ungesättigter Carbonsäuren von aromatischen, cycloaliphatischen und aliphatischen Polyaminen mit bevorzugt 2 bis 6, besonders 2 bis 4 Aminogruppen geeignet. Beispiele für solche Polyamine sind Ethylendiamin, 1,2- oder 1,3-Propylendiamin, 1,2-, 1,3- oder 1,4-Butylendiamin, 1,5-Pentylendiamin, 1,6-Hexylendiamin, Octylendiamin, Dodecylendiamin, 1,4-Diaminocyclohexan, Isophorondiamin, Phenylendiamin, Bisphenylendiamin, Di-$\beta$-aminoethylether, Diethylentriamin, Triethylentetramin, Di-($\beta$-aminoethoxy)- oder Di($\beta$-aminopropoxy)ethan. Weitere geeignete Polyamine sind Polymere und Copolymere mit Aminogruppen in der Seitenkette und Oligoamide mit Aminoendgruppen.

Beispiele für solche ungesättigten Amide sind: Methylen-bis-acrylamid, 1,6-Hexamethylen-bis-acrylamid, Diethylentriamin-tris-methacrylamid, Bis(methacrylamidopropoxy)-ethan, $\beta$-Methacrylamidoethylmethacrylat,N[($\beta$-Hydroxyethoxy)ethyl]-acrylamid.

Geeignete ungesättigte Polyester und Polyamide leiten sich z.B. von Maleinsäure und Diolen oder Diaminen ab. Die Maleinsäure kann teilweise durch andere Dicarbonsäuren ersetzt sein. Sie können zusammen mit ethylenisch ungesättigten Comonomeren, z.B. Styrol, eingesetzt werden. Die Polyester und Polyamide können sich auch von Dicarbonsäuren und ethylenisch ungesättigten Diolen oder Diaminen ableiten, besonders von längerkettigen mit z.B. 6 bis 20 C-Atomen. Beispiele für Polyurethane sind solche, die aus gesättigten oder ungesättigten Diisocyanaten und ungesättigten bzw. gesättigten Diolen aufgebaut sind.

Polybutadien und Polyisopren und Copolymere davon sind bekannt. Geeignete Comonomere sind z.B. Polyolefine wie Ethylen, Propen, Buten, Hexen, (Meth)Acrylate, Acrylnitril, Styrol oder Vinylchlorid. Polymere mit (Meth)Acrylatgruppen in der Seitenkette sind ebenfalls bekannt. Es kann sich z.B. um Umsetzungsprodukte von Epoxidharzen auf Novolakbasis mit (Meth)Acrylsäure handeln, um Homo- oder Copolymere des Polyvinylalkohols oder deren Hydroxyalkylderivaten, die mit (Meth)Acrylsäure verestert sind, oder um Homo- und Copolymere von (Meth)Acrylaten, die mit Hydroxyalkyl(meth)acrylaten verestert sind.

Die photopolymerisierbaren Verbindungen können alleine oder in beliebigen Mischungen eingesetzt werden. Bevorzugt werden Gemische von Polyol-(Meth)Acrylaten verwendet.

Den erfindungsgemässen Zusammensetzungen können auch Bindemittel zugesetzt werden, was besonders zweckmässig ist, wenn es sich bei den photopolymerisierbaren Verbindungen um flüssige oder viskose Substanzen handelt. Die Menge des Bindemittels kann z.B. 5-95, vorzugsweise 10-90 und besondere 50-90 Gew.% betragen, bezogen auf die gesamte Zusammensetzung. Die Wahl des Bindemittels erfolgt je nach dem Anwendungsgebiet und hierfür geforderter Eigenschaften wie Entwickelbarkeit in wässrigen und organischen Lösungsmittelsystemen. Adhäsion auf Substraten und Sauerstoffempfindlichkeit.

Geeignete Bindemittel sind z.B. Polymere mit einem Molekulargewicht von etwa 5000-2 000 000, bevorzugt 10 000 bis 1 000 000. Beispiele sind: Homo- und copolymere Acrylate und Methacrylate, z.B. Copolymere aus Methylmethacrylat/Ethylacrylat/Methacrylsäure, Poly(methacrylsäurealkylester),Poly-(acrylsäurealkylester); Celluloseester und -ether wie Celluloseacetat, Celluloseacetatbutyrat, Methylcellulose, Ethylcellulose; Polyvinylbutyral, Polyvinylformal, cyclisierter Kautschuk, Polyether wie Polyethylenoxid, Polypropylenoxid, Polytetrahydrofuran; Polystyrol, Polycarbonat, Polyurethan, chlorierte Polyolefine, Polyvinylchlorid, Copolymere aus Vinylchlorid/Vinylidenchlorid, Copolymere von Vinylidenchlorid mit Acrylnitril, Methylmethacrylat und Vinylacetat, Polyvinylacetat, Copoly(ethylen/vinylacetat), Polyamide wie Polycaprolactam und Poly(hexamethylenadipamid), Polyester wie Poly(äthylenglykolterephthalat) und Poly-(hexamethylenglykolsuccinat).

Die erfindungsgemässen Zusammensetzungen eignen sich als Beschichtungsmittel für Substrate aller Art, z.B. Holz, Papier, Keramik, Kunststoffe, wie Polyester und Celluloseacetatfilme, und Metalle, wie Kupfer und Aluminium, bei denen durch Photopolymerisation eine Schutzschicht oder eine photographische Abbildung aufgebracht werden soll. Ein weiterer Gegenstand vorliegender Erfindung sind die beschichteten Substrate und ein Verfahren zum Aufbringen photographischer Abbildungen auf den Substraten. Die beschichteten Substrate können auch als Aufzeichnungsmaterial für Hologramme (Volumen-Phasen-Diagramm) verwendet werden, wobei vorteilhaft ist, dass für diesen Zweck keine Nassentwicklung notwendig ist.

Die Beschichtung der Substrate kann erfolgen, indem man eine flüssige Zusammensetzung, eine Lösung oder Suspension auf das Substrat aufbringt. Flüssige Zusammensetzungen ohne Lösungsmittel sind bevorzugt. Hierbei kann es zweckmässig sein, die erfindungsgemässen Titanocene in Form eines flüssigen Photoinitiatorengemisches, enthaltend andere Photoinitiatoren, z.B. ein Benzilketal, ein 4-Aroyl-1,3-dioxolan, ein Dialkoxyacetophenon, ein α-Hydroxy- oder α-Aminoacetophenon, ein α-Hydroxycycloalkylphenylketon oder Mischungen hiervon einzusetzen. Besonders vorteilhaft sind flüssige Mischungen aus flüssigen bis festen Photoinitiatoren und flüssigen Titanocenen oder flüssigen Photoinitiatoren und sirupösen bis festen Titanocenen. Diese Gemische bieten anwendungstechnische Vorteile und zeichnen sich durch eine hohe Dunkellagerstabilität aus.

Beispiele für Benzilketale sind solche der Formel

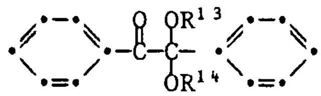

$R^{13} = R^{14} = -CH_3$

$-CH_2CH_3$

$-(CH_2)_2CH_3$

$-(CH_2)_3CH_3$

$-CH_2CH_2CH(CH_3)_2$

$-CH_2-CH(C_2H_5)-C_4H_9$

$-(CH_2)_9CH_3$

$-C_{10}H_{21}$-iso

$-C_{12}H_{25}$-n

$-C_9H_{19}$ bis $-C_{11}H_{23}$-Gemisch

$-C_{12}$-$H_{25}$-bis-$C_{15}H_{31}$-Gemisch

$-CH_2CH=CH_2$

$-CH(CH_3)CH=CH_2$

$-CH_2CH_2OC_3H_7$-iso

$-CH_2CH_2OC_4H_9$

$-CH_2CH_2OCH_2CH=CH_2$

$-CH(CH_3)-CH_2OC_4H_9$

$-CH_2COOCH_3$

$-CH_2COOC_4H_9$

$-CH(CH_3)COOCH_3$

$-CH_2CH_2COOC_2H_5$

$-CH(CH_3)CH_2COOCH_3$

$-CH_2CH_2CH(CH_3)OCH_3$

$-CH_2-$ 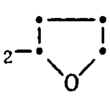

$-(CH_2CH_2O)_2CH_3$

$-(CH_2CH_2O)_2C_2H_5$

$-(CH_2CH_2O)_2C_4H_9$

$-(CH_2CH_2O)_3CH_3$

$-(CH_2CH_2O)_3C_2H_5$

$-(CH_2CH_2O)_3C_{12}H_{25}$

$-(CH_2CH_2O)_5C_{10}H_{21}$

$-(CH_2CH_2O)_8C_9H_{19}$-bis-$C_{11}H_{23}$ (Gemisch)

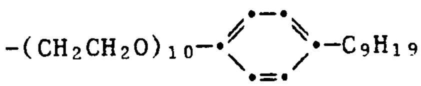

$-CH_2CH_2N(C_2H_5)_2$

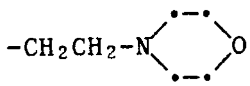

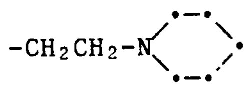

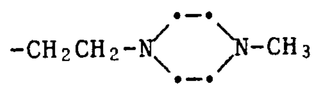

$R^{14} = CH_3$, $R^{13} = C_6H_{13}$

$R^{14} = CH_3$, $R^{13} = C_{10}H_{21}$

$R^{14} = CH_3$, $R^{13} = (CH_2CH_2O)_3-C_{12}H_{25}$ bis-$C_{15}H_{31}$ (Gemisch)

$R^{14} = CH_3$, $R^{13} = (CH_2CH_2O)_5-C_9H_{19}$ bis $-C_{11}H_{23}$ (Gemisch)

$R^{14} = CH_3$, $R^{13} = (CH_2CH_2O)_8-\overset{O}{\overset{\|}{C}}-C_{11}H_{23}$.

Beispiele für 4-Aroyl-1,3-dioxolane sind:
4-Benzoyl-2,2,4-trimethyl-1,3-dioxolan
4-Benzoyl-4-methyl-2,2-tetramethylen-1,3-dioxolan
4-Benzoyl-4-methyl-2,2-pentamethylen-1,3-dioxolan
cis-trans 4-Benzoyl-2,4-dimethyl-2-methoxymethyl-1,3-dioxolan
cis-trans 4-Benzoyl-4-methyl-2-phenyl-1,3-dioxolan
4-(4-Methoxybenzoyl)-2,2,4-trimethyl-1,3-dioxolan
4-(4-Methoxybenzoyl)-4-methyl-2,2-pentamethylen-1,3-dioxolan
4-(4-Methylbenzoyl)-2,2,4-trimethyl-1,3-dioxolan
cis-trans 4-Benzoyl-2-methyl-4-phenyl-1,3-dioxolan
4-Benzoyl-2,2,4,5,5-pentamethyl-1,3-dioxolan
cis-trans 4-Benzoyl-2,2,4,5-tetramethyl-1,3-dioxolan
cis-trans 4-Benzoyl-4-methyl-2-pentyl-1,3-dioxolan
cis-trans 4-Benzoyl-2-benzyl-2,4-dimethyl-1,3-dioxolan
cis-trans 4-Benzoyl-2-(2-furyl)-4-methyl-1,3-dioxolan
cis-trans 4-Benzoyl-5-phenyl-2,2,4-trimethyl-1,3-dioxolan
4-(4-Methoxybenzoyl)-2,2,4,5,5-pentamethyl-1,3-dioxolan.

Beispiele für Dialkoxyacetophenone sind:
α,α-Dimethoxyacetophenon
α,α-Diethoxyacetophenon
α,α-Di-isopropoxyacetophenon
α,α-Di-(2-methoxyethoxy)acetophenon
α-Butoxy-α-ethoxyacetophenon
α,α-Dibutoxy-4-chloracetophenon
α,α-Diethoxy-4-fluoroacetophenon
α,α-Dimethoxy-4-methylacetophenon
α,α-Diethoxy-4-methylacetophenon

α,α-Dimethoxypropiophenon
α,α-Diethoxypropiophenon
α,α-Diethoxybutyrophenon
α,α-Dimethoxyisovalerophenon
α,α-Diethoxy-α-cyclohexylacetophenon
α,α-Dipropoxy-4-chlorpropiophenon.

Beispiele für α-Hydroxy- und α-Aminoacetophenone sind:
2-Hydroxy-2-methyl-1-phenyl-propanon-1
2-Hydroxy-2-ethyl-1-phenylhexanon-1
1-(4-Dodecylphenyl)-2-hydroxy-2-methylpropanon-1
1-(2,4-Dimethylphenyl)-2-hydroxy-2-methylpropanol-1
2-Hydroxy-1-(4-methoxyphenyl)-2-methylpropanon-1
2-Hydroxy-2-methyl-1-phenylbutanon-1
2-Hydroxy-1-[4-(2-hydroxyethoxy)phenyl]-2-methylpropanon-1
2-Dimethylamino-2-methyl-1-phenylpropanon-1
2-Dibutylamino-2-methyl-1-phenylpropanol-1
1-(4-Fluorphenyl)-2-methyl-2-morpholinopentanon-1
2-Methyl-1-(4-methylthiophenyl)-2-morpholinopropanon-1
2-Dimethylamino-1-(4-methoxyphenyl)-2-methylpropanon-1
2-Diethylamino-1-(4-diethylaminophenyl)-2-methylpropanon-1.
2-Dimethylamino-2-(4-methylbenzyl)-1-(4-morpholinophenyl)-butanon-1
2-Benzyl-2-dimethylamino-1-(4-morpholinophenyl)-butanon-1

Beispiele für α-Hydroxycycloalkylphenylketone sind:
α-Hydroxycyclohexylphenylketon
α-Hydroxycyclopentylphenylketon

Das Photoinitiatorengemisch (b) + (c) kann in Mengen von 0,5-20, vorzugsweise 1 bis 10 Gew.-%, zugegeben werden, bezogen auf die Komponente (a).

Die Wahl des Lösungsmittels und die Konzentration richtet sich hauptsächlich nach der Art der Zusammensetzung und nach dem Beschichtungsverfahren. Die Zusammensetzung wird mittels bekannter Beschichtungsverfahren auf ein Substrat gleichförmig aufgebracht, z.B. durch Tauchen, Rakelbeschichtung, Vorhanggiessverfahren, Elektrophorese, Aufpinseln, Sprayen oder Reverseroll-Beschichtung. Die Auftragsmenge (Schichtdicke) und Art des Substrates (Schichtträger) sind abhängig vom gewünschten Applikationsgebiet. Als Schichtträger für photographische Informationsaufzeichnung dienen z.B. Folien aus Polyester, Celluloseacetat oder mit Kunststoff beschichtete Papiere; für Offsetdruckformen speziell behandeltes Aluminium und für die Herstellung gedruckter Schaltungen kupferkaschierte Laminate. Die Schichtdicken für photographische Materialien und Offsetdruckformen betragen im allgemeinen ca. 0,5 bis ca. 10 µm; für gedruckte Schaltungen im allgemeinen 1 bis ca. 100 µm. Bei Mitverwendung von Lösungsmitteln werden diese nach dem Beschichten entfernt.

Photohärtbare Zusammensetzungen, wie sie für die verschiedenen Zwecke verwendet werden, enthalten meist ausser den photopolymerisierbaren Verbindungen und den Photoinitiatoren eine Reihe sonstiger Zusätze. So ist es vielfach üblich, thermische Inhibitoren zuzusetzen, die vor allem während der Herstellung der Zusammensetzungen durch Mischen der Komponenten vor einer vorzeitigen Polymerisation schützen sollen. Hierzu werden beispielsweise Hydrochinon, Hydrochinonderivate, p-Methoxyphenol, β-Naphthole oder sterisch gehinderte Phenole wie z.b. 2,6-Di(tert-butyl)-p-kresol verwendet. Weiter können geringe Mengen von UV-Absorbern zugesetzt werden, wie z.B solche vom Benztriazol-, Benzophenon- oder Oxalanilid-Typ. Ebenso lassen sich Lichtschutzmittel vom Typus sterisch gehinderter Amine (HALS) zusetzen.

Zur Erhöhung der Dunkellagerstabilität können Kupferverbindungen, wie Kupfernaphthenat, -stearat, oder -octoat, Phosphorverbindungen, wie Triphenylphosphin, Tributylphosphin, Triethylphosphit, Triphenylphosphit oder Tribenzylphosphit, quaternäre Ammoniumverbindungen, wie Tetramethylammoniumchlorid oder Trimethyl-benzylammoniumchlorid oder Hydroxylaminderivate, wie z.B. N-Diethylhydroxylamin, zugesetzt werden.

Um die inhibierende Wirkung des Luftsauerstoffs auszuschliessen setzt man photohärtbaren Gemischen häufig Paraffin oder ähnliche wachsartige Stoffe zu. Diese schwimmen bei Beginn der Polymerisation wegen mangelnder Löslichkeit im Polymeren aus und bilden eine transparente Oberflächenschicht, die den Zutritt von Luft verhindert.

Weitere übliche Zusätze sind Photosensibilisatoren, welche in bestimmten Wellenlängen absorbieren und die absorbierte Energie an den Initiatoren weitergeben oder selbst als zusätzlicher Initiator fungieren.

Beispiele hierfür sind vor allem Thioxanthon-, Anthracen-, Anthrachinon- und Cumarinderivate.

Weitere übliche Zusätze sind Beschleuniger vom Amin-Typ, die vor allem in pigmentierten Zubereitungen von Bedeutung sind, da sie als Kettenüberträger wirken. Beispiele hierfür sind N-Methyldiethanolamin, Triethylamin, p-Dimethylaminobenzoesäureethylester oder Michler's Keton. Die Wirkung der Amine kann verstärkt werden durch den Zusatz von aromatischen Ketonen vom Benzophenontyp. Weitere Beschleuniger sind Thiadiazolderivate, wie z.B. 2-Mercapto-2-methylthio-1,3,4-thiadiazol.

Weitere übliche Zusätze sind z.B. Füllstoffe, Pigmente, Farbstoffe, Haft-, Netz- und Verlaufmittel.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Insbesondere für den Siebdruck sind UV-härtbare Druckfarben von Bedeutung.

Gut geeignet sind die erfindungsgemässen photohärtbaren Zusammensetzungen auch zur Herstellung von Druckplatten, insbesondere Flexodruckplatten. Hierbei werden z.B. Gemische von löslichen linearen Polyamiden oder von Styrol-Butadien-Kautschuk mit photopolymerisierbaren Monomeren, beispielsweise Acrylamiden oder Acrylaten, und einem Photoinitiator verwendet. Filme und Platten aus diesen Systemen werden über das Negativ (oder Positiv) der Druckvorlage belichtet und die ungehärteten Anteile anschliessend mit einem Lösungsmittel eluiert.

Ein weiteres Einsatzgebiet der Photohärtung ist die Metallbeschichtung, beispielsweise bei der Lackierung von Blechen für Tuben, Dosen oder Flaschenverschlüssen, sowie die Photohärtung von Kunststoffbeschichtungen, beispielsweise von Fussboden- oder Wandbelägen auf PVC-Basis.

Beispiele für die Photohärtung von Papierbeschichtungen sind die farblose Lackierung von Etiketten, Schallplatten-Hüllen oder Buchumschlägen.

Wichtig ist auch die Verwendung der photohärtbaren Zusammensetzungen für Abbildungsverfahren und zur optischen Herstellung von Informationsträgern. Hierbei wird die auf dem Träger aufgebrachte Schicht (nass oder trocken) durch eine Photomaske mit kurzwelligem Licht bestrahlt und die unbelichteten Stellen der Schicht durch Behandlung mit einem Lösungsmittel (= Entwickler) entfernt. Die belichteten Stellen sind vernetzt-polymer und dadurch unlöslich und bleiben auf dem Träger stehen. Bei entsprechender Anfärbung entstehen sichtbare Bilder. Ist der Träger eine metallisierte Schicht, so kann das Metall nach dem Belichten und Entwickeln an den unbelichteten Stellen weggeätzt oder durch Galvanisieren verstärkt werden. Auf diese Weise lassen sich gedruckte Schaltungen und Photoresists herstellen.

Zur Belichtung eignen sich Lichtquellen mit hohem Anteil an kurzwelligem Licht. Hierfür stehen heute entsprechende technische Vorrichtungen und verschiedene Lampenarten zur Verfügung. Beispiele sind Kohlelichtbogenlampen, Xenonlichtbogenlampen, Quecksilberdampflampen, Metall-Halogenlampen, Fluoreszenzlampen, Argonlampen oder photographische Flutlichtlampen. Neuerdings werden auch Laserlichtquellen verwendet. Diese haben den Vorteil, dass keine Photomasken notwendig sind; der gesteuerte Laserstrahl schreibt direkt auf die photohärtbare Schicht.

Die erfindungsgemässen Titanocene sind mit den Komponenten der photohärtbaren Zusammensetzungen gut vermischbar bzw. in der Zusammensetzung gut löslich, wodurch eine hohe Lichtempfindlichkeit erzielt werden kann. Sie sind auch gut zugänglich, da die Lithiumfluorarylamine als Ausgangsprodukte durch einen Lithium/Wasserstoffaustausch erhältlich sind. Eine vorherige Einführung von Halogenen in das Fluorarylamin erübrigt sich daher.

Die nachfolgenden Beispiele erläutern die Erfindung näher. Darin bedeuten Teile und % Gewichtsteile und Gewichts-%, soweit nicht anders angegeben. Die Temperaturen sind in °C angegeben.

A) Herstellung der sekundären 2,4-Difluoraniline

a) aus 2,4-Difluoranilin

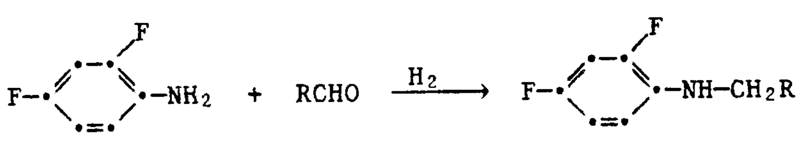


1 Mol 2,4-Difluoranilin wird mit 2 Mol des entsprechenden Aldehyds in 1 l Tetrahydrofuran gelöst. Nach Zusatz von 19 g Raney-Nickel und 2 g Essigsäure wird bei 60° und 60 bar $H_2$ hydriert. Der Endpunkt der Reaktion kann dünnschicht- oder gas-chromatographisch bestimmt werden. Die Aufarbeitung erfolgt durch

Abdestillieren des Lösungsmittels am Rotationsverdampfer und Rektifikation des Rückstandes.

Tabelle 1

| R | Kp |
|---|---|
| $CH_3$ | 86-88°/20 mbar |
| n-$C_3H_7$ | 108-110°/20 mbar |
| iso-$C_3H_7$ | 100-101°/20 mbar |
| n-$C_4H_9$ | 58-62°/0,6 mbar |
| n-$C_5H_{11}$ | 115°/2 mbar |
| n-$C_7H_{15}$ | 81-83°/0,15 mbar |
| 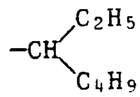 | 70-80°/1,5 mbar |
| -$CH_2OCH_3$ | 124-25°/23 mbar |
| -$CH_2O(CH_2)_2OCH_3$ | 98-104°/0,04 mbar |
| -$CH_2OC_4H_9$ | 84-87°/0,04 mbar |
| 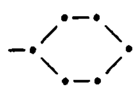 | 79°/2 mbar |
| 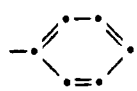 | 119°/0,04 mbar |
| -$CH_2CH_2$-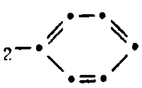 | 106-9°/2 mbar |
| 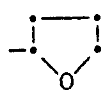 | 74-78°/2 mbar |

Ebenso werden mit 2 Mol des entsprechenden Ketons erhalten:

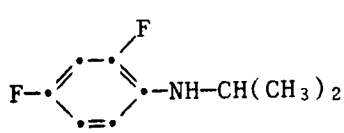

Kp. 79-81°/20 mbar

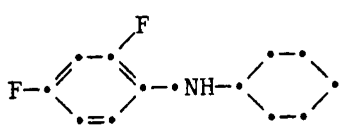

Kp. 70-72°/0,04 mbar

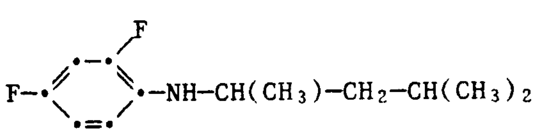

Kp. 120-125°/14 mbar

b) aus 2,4-Difluornitrobenzol

159,1 g 2,4-Difluor-nitrobenzol und 150 g 4-Methyl-pentanon-2 werden in 1,2 l Methanol mit 3 g $H_2SO_4$ konz. und 5 g Pt/C 5 % gemischt und während 3 Stunden bei 30 - 35° unter konstant 5 bar $H_2$ katalytisch hydriert. Der Fortgang der Hydrierung wird dünnschichtchromatographisch an Kieselgel mit dem Gemisch Petrolether/Dioxan 4:1 als Laufmittel verfolgt. Nach beendeter Reaktion wird der Katalysator abfiltriert und das Lösungsmittel am Rotationsverdampfer abdestilliert. Es bleibt ein braunes Oel zurück, das nach der Entfernung von etwas harzigem Nebenprodukt im Vakuum rektifiziert wird. Die Fraktion von Kp. 120 - 125° bei 14 mbar wird aufgefangen. Man erhält 173,4 g N-(1,3-Dimethylbutyl)-2,4-difluoranilin als farbloses Oel.

B) Herstellung der Zwischenprodukte der Formel VII und VIII

Beispiel 1: N-Ethyl-N-pivaloyl-2,4-difluoranilin
15,7 g N-Ethyl-2,4-difluoranilin und 11,0 g Triethylamin werden in 40 ml Toluol gelöst. Unter Kühlung werden 12,1 g Pivalinsäurechlorid zugetropft und das Gemisch hierauf während 1 Stunde zum Sieden erhitzt. Nach dem Aufgiessen auf 100 ml Eiswasser wird die Toluolphase abgetrennt, mit 1N-HCl und darauf mit $H_2O$ gewaschen und im Vakuum eingedampft. Man erhält 23,7 g eines gelblichen Oels das beim Stehen kristallisiert. Schmelzpunkt: 69 - 71° (aus verdünntem Ethanol umkristallisiert).

| | | | | | |
|---|---|---|---|---|---|
| Elementaranalyse: | ber. | C 64,7 | H 7,1 | F 15,8 | N 5,8 % |
| | gef. | C 65,0 | H 7,1 | F 15,9 | N 5,8 % |

Beispiele 2 - 43: In Analogie zu Beispiel 1 werden weitere N-Acyl-2,4-difluoraniline hergestellt. Diese Verbindungen sind in der tabelle 2 aufgeführt.

Tabelle 2

Produkte der Formel 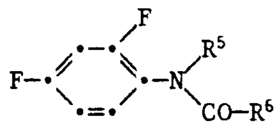

| Beispiel Nr. | $R^5$ | $R^6$ | Physikal. Eigenschaften | Analyse % N ber. | gef. |
|---|---|---|---|---|---|
| 2 | H | $CH_3$ | Fp. 116-120° | 8,2 | 8,1 |
| 3 | H | $-CH(CH_3)_2$ | Fp. 99-101° | 7,0 | 6,9 |
| 4 | H | $CH_3$<br>$-C-CH_2Cl$<br>$CH_3$ | Fp. 88-93° | 5,7 | 5,3 |
| 5 | H | $-C(CH_3)(CH_2Cl)_2$ | Fp. 83-86° | 4,9 | 4,6 |
| 6 | H | $-C(CH_3)_3$ | Fp. 67° | 6,6 | 6,6 |
| 7 | $CH_3$ | $n-C_3H_7$ | $Kp_9$ 100-103° | 6,6 | 6,6 |
| 8 | $CH_3$ | $n-C_4H_9$ | $Kp_9$ 115-117° | 6,2 | 6,2 |
| 9 | $C_2H_5$ | $CH_3$ | $Kp_{20}$ 92-95° | 7,0 | 7,1 |
| 10 | $C_2H_5$ | $C_2H_5$ | $Kp_{44}$ 120-125° | 6,6 | 6,7 |
| 11 | $C_2H_5$ | $CH_3$<br>$-C-C_2H_5$<br>$CH_3$ | Fp. 47-50° | 5,5 | 5,6 |
| 12 | $C_2H_5$ | 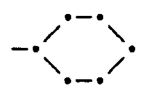 | Fp. 67-68° | 5,5 | 5,4 |
| 13 | $C_2H_5$ | $-CH(CH_3)_2$ | Fp. 46-47° | 6,2 | 6,3 |
| 14 | iso-$C_3H_7$ | Phenyl | Fp. 110-112° | 5,1 | 5,0 |
| 15 | iso-$C_3H_7$ | p-Tolyl | Fp. 90-94° | 4,8 | 4,9 |
| 16 | n-$C_4H_9$ | $CH_3$ | $Kp._{0.5}$ 80-83° | 6,2 | 6,3 |
| 16a | n-$C_4H_9$ | $CF_3$ | $Kp._{10}$ 102-10° | 5,0 | 5,0 |
| 17 | n-$C_4H_9$ | $-C(CH_3)_3$ | $Kp._5$ 143° | 5,2 | 5,3 |
| 18 | n-$C_4H_9$ | $CH_3$<br>$-C-C_2H_5$<br>$CH_3$ | $Kp._{0,4}$ 92-95° | 5,0 | 5,2 |
| 18a | n-$C_4H_9$ | $CH_3$<br>$-C-C_3H_7$<br>$CH_3$ | Oel | 4,7 | 4,4 |
| 19 | n-$C_4H_9$ | $CH_3$<br>$-C-CH_2Cl$<br>$CH_3$ | $Kp._{0,6}$ 95-100° | 4,6 | 4,8 |
| 20 | n-$C_4H_9$ | Phenyl | Oel | 4,8 | 5,0 |
| 21 | n-$C_4H_9$ | p-Tolyl | Oel | 4,6 | 4,4 |
| 22 | n-$C_4H_9$ | o-Chlorphenyl | Fp. 60-67° | 4,3 | 4,1 |
| 23 | iso-$C_4H_9$ | Phenyl | Fp. 69-73° | 4,8 | 4,6 |
| 23a | iso-$C_4H_9$ | p-Chlorphenyl | Fp. 82-84° | 4,3 | 4,2 |
| 24 | iso-$C_4H_9$ | p-Tolyl | Fp. 70-75° | 4,6 | 4,5 |
| 25 | iso-$C_4H_9$ | $CH_3$<br>$-C-C_3H_7$<br>$CH_3$ | Oel | 4,7 | 4,1 |

| Beispiel Nr. | $R^5$ | $R^6$ | Physikal. Eigenschaften | Analyse % N ber. | gef. |
|---|---|---|---|---|---|
| 26 | iso-$C_4H_9$ | 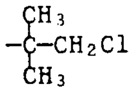 | Fp. 65-70° | 4,6 | 4,5 |
| 27 | n-$C_5H_{11}$ | 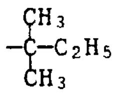 | $Kp_{0,5}$ 95-100° | 4,7 | 4,5 |
| 28 | n-$C_6H_{13}$ | $CH_3$ | $Kp_{0,5}$ 94-98° | 5,5 | 5,7 |
| 29 | n-$C_6H_{13}$ | 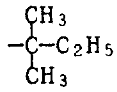 | $Kp_{0,2}$ 122-124° | 4,5 | 4,4 |
| 30 | n-$C_6H_{13}$ | Phenyl | Oel | 4,4 | 4,4 |
| 31 | n-$C_6H_{13}$ | p-Tolyl | Oel | 4,2 | 3,9 |
| 32 | n-$C_6H_{13}$ | 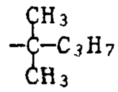 | Oel | 4,3 | 4,2 |
| 33 | n-$C_6H_{13}$ | p-Chlorphenyl | Oel | 4,0 | 3,7 |
| 34 | n-$C_6H_{13}$ | o-Chlorphenyl | Oel | 4,0 | 3,8 |
| 35 | n-$C_8H_{17}$ | $CH_3$ | $Kp_{0,5}$ 115-118° | 5,0 | 5,3 |
| 36 | 2-Ethylhexyl | Phenyl | Oel | 4,1 | 4,1 |
| 37 | 2-Ethylhexyl | p-Tolyl | Oel | 3,9 | 3,9 |
| 38 | 2-Ethylhexyl | 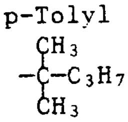 | Oel | 4,0 | 3,9 |
| 39 | $-CH_2CH_2OCH_3$ | Phenyl | Oel | 4,8 | 5,0 |
| 40 | $-CH_2CH_2OCH_3$ | p-Tolyl | Oel | 4,6 | 4,5 |
| 41 | $-CH_2CH_2OCH_3$ | 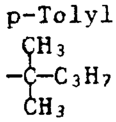 | Oel | 4,7 | 4,3 |
| 42 | $-CH_2CH_2OC_4H_9$ | Phenyl | Oel | 4,4 | 4,4 |
| 43 | $-CH_2CH_2OC_4H_9$ | 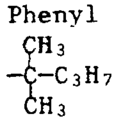 | Oel | 3,9 | 4,0 |
| 44 | $-CH_2CH_2O(CH_2)_2OCH_3$ | Phenyl | Fp. 70° | 4,0 | 4,3 |
| 45 | $-CH_2CH_2O(CH_2)_2OCH_3$ | 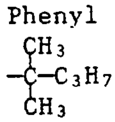 | Oel | 4,1 | 4,0 |
| 46 | 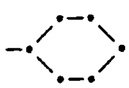 | Phenyl | Fp. 60-63° | 4,4 | 4,0 |
| 47 | $-CH_2-$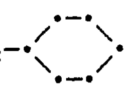 | Phenyl | Fp. 75-80° | 4,2 | 4,0 |
| 48 | $-CH_2-$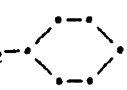 | p-Tolyl | Fp. 87-91° | 4,1 | 4,3 |
| 49 | $-CH_2-$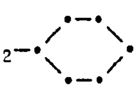 | p-Chlorphenyl | Fp. 87-90° | 3,9 | 3,7 |

| Beispiel Nr. | $R^5$ | $R^6$ | Physikal. Eigenschaften | Analyse % N ber. | gef. |
|---|---|---|---|---|---|
| 50 | 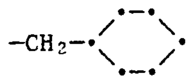 | 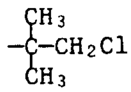 | Oel | 4,1 | 4,0 |
| 51 | 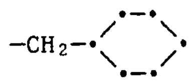 | $-C(CH_3)_3$ | Fp. 74-78° | 4,5 | 4,5 |
| 52 | 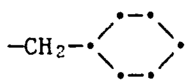 | 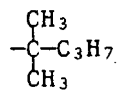 | Fp. 52-54° | 4,2 | 4,0 |
| 53 | 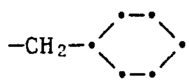 | 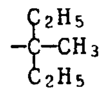 | Oel | 4,2 | 4,0 |
| 54 | 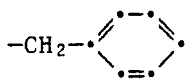 | Phenyl | Fp. 128-34° | 4,3 | 4,2 |
| 55 | 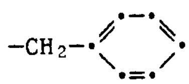 | p-Tolyl | Fp. 87-93° | 3,99 | 3,75 |
| 56 | 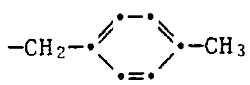 | Phenyl | Fp. 85-90° | 4,15 | 4,04 |
| 56a | 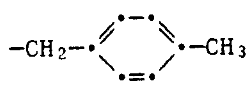 | p-Chlorphenyl | Fp. 107-10° | 3,8 | 3,7 |
| 57 | 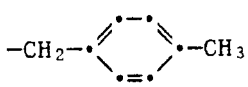 | p-Tolyl | Fp. 87-93° | 4,0 | 3,8 |
| 58 | 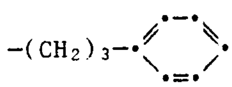 | Phenyl | Oel | 4,0 | 4,0 |
| 59 | 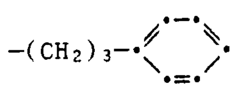 | p-Tolyl | Oel | 3,8 | 3,7 |
| 60 | 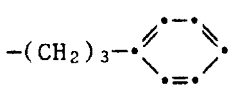 | $-C(CH_3)_3$ | Oel | 4,3 | 4,1 |
| 61 | 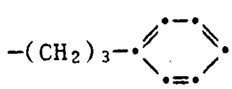 | 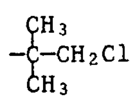 | Oel | 3,8 | 3,5 |
| 62 | 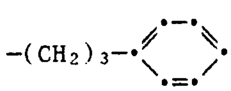 | 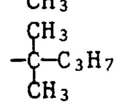 | Oel | 3,9 | 3,7 |
| 63 | 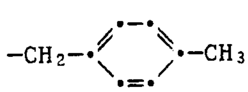 | 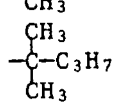 | Fp. 76-77° | 4,1 | 4,0 |
| 64 | 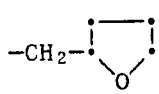 | Phenyl | Oel | 4,4 | 4,3 |
| 65 | 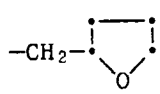 | p-Tolyl | Oel | 4,2 | 4,0 |

| Beispiel Nr. | $R^5$ | $R^6$ | Physikal. Eigenschaften | Analyse % N ber. | gef. |
|---|---|---|---|---|---|
| 66 | $-CH_2-$ 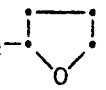 | 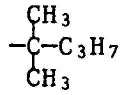 | Oel | 4,3 | 4,3 |

Beispiel 67: Acylierung mit Carbonsäureanhydrid

15,7 g N-Ethyl-2,4-difluoranilin werden in 40 ml Toluol gelöst und mit 23,2 g Pivalinsäureanhydrid versetzt. Es wird zum Sieden erhitzt und das Reaktionsgemisch während 8 Stunden am Sieden gehalten. Nach dem Aufgiesssen auf Eiswasser wird die Toluolphase mit 1N-HCl, Wasser, Bicarbonatlösung und nochmals mit Wasser gewaschen. Man erhält 21,0 g eines gelblichen Oels, das beim Stehen kristallisiert. Das Produkt ist mit der nach Beispiel 1 hergestellten Verbindung identisch.

Beispiel 68: Alkylierung einer N-Acylverbindung

Eine Mischung von 60,8 g n-Butyl-methansulfonat und 22,5 g Trifluoracetyl-2,4-difluoranilin werden in 50 ml Aceton bei 40 °C gelöst und mit 22,4 g KOH-Pulver versetzt. Die Reaktionsmischung wird 30 Minuten am Rückfluss gehalten, filtriert und am Rotationsverdampfer eingedampft. Der ölige Rückstand wird rektifiziert. Man erhält 15 g N-Butyl-trifluoracetyl-2,4-difluoranilin als bei 106 - 110 °/11,7 mbar destillierendes gelbliches Oel.

| | | | | | |
|---|---|---|---|---|---|
| Analyse: | ber. | C 51,25 | H 4,30 | F 33,78 | N 4,98 % |
| | gef. | C 51,3 | H 4,4 | F 33,4 | N 5,0 % |

Beispiele 69 - 73: N-Sulfonylierung

Eine Lösung von 14,8 g N-Butyl-2,4-difluoranilin, 16,2 g Triethylamin und 0,5 g Dimethylaminopyridin in 150 ml Toluol wird bei 0 - 5 °C tropfenweise mit einer Lösung von 15,2 g p-Toluolsulfonsäurechlorid in 80 ml Toluol versetzt. Dann wird das Reaktionsgemisch auf 90 °C erwärmt und 48 h bei dieser Temperatur gerührt. Nach dem Abkühlen wird mit 5%iger HCl und mit Wasser gewaschen, die Toluol-Lösung wird über $Na_2SO_4$ getrocknet und eingedampft. Der Rückstand wird bei 0,01 mbar destilliert. Bei 150 - 160 ° erhält man 18,5 g N-Butyl-N-(2,4-difluorphenyl)-p-toluolsulfonamid als farbloses Oel.

Nach dieser Methode wurden die in Tabelle 3 aufgeführten Sulfonamide hergestellt.

Tabelle 3:

Verbindungen der Formel

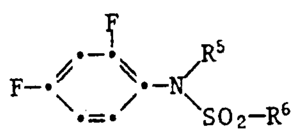

| Beispiel Nr. | $R^5$ | $R^6$ | Physik. Eigensch. | Analyse % N ber. | gef. |
|---|---|---|---|---|---|
| 69 | n-$C_4H_9$ | p-Tolyl | Oel | 4,1 | 4,0 |
| 70 | iso-$C_4H_9$ | p-Tolyl | Fp. 68 - 70° | 4,1 | 4,1 |
| 71 | n-$C_6H_{13}$ | p-Tolyl | Fp. 45 - 49° | 3,8 | 3,8 |
| 72 | 2-Ethylhexyl | p-Tolyl | Oel | 3,5 | 3,5 |
| 73 | $C_2H_5$ | p-Tolyl | Fp. 100° | 4,5 | 4,6 |

Beispiele 74 - 80: Cyclische Imide

18,1 g 2,4-Difluoranilin und 14 g Bernsteinsäureanhydrid werden in 300 ml Toluol gelöst und die Lösung mit 0,5 g 4-(Dimethylamino)pyridin versetzt. Danach wird zum Rückfluss erhitzt, bis gemäss DC-Analyse kein 2,4-Difluoranilin mehr nachgewiesen werden kann (16 Std). Nach dem Abkühlen wird auf 2N HCl ausgegossen, die organische Phase mit Wasser gewaschen und nach Trocknen über Magnesiumsulfat

eingeengt. Man erhält 23,4 g Rohprodukt, das nach Umkristallisation aus Isopropanol bei 140 - 143 °C schmilzt.

Nach dieser Methode werden die in Tabelle 4 aufgeführten Verbindungen hergestellt.

Tabelle 4:

Verbindungen der Formel 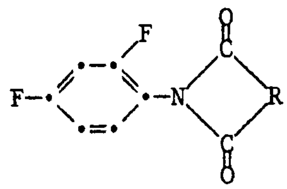

| Beispiel Nr. | R | Fp | Analyse % N ber. | gef. |
|---|---|---|---|---|
| 74 | $-CH_2-CH_2-$ | 140-143° | 6,63 | 6,61 |
| 75 | $-CH_2-CH(CH_3)-$ | 83-87° | 6,22 | 6,12 |
| 76 | $-CH{=}CH-$ | 79-82° | 6,70 | 6,75 |
| 77 | $-CH{=}C(CH_3)-$ | 80-84° | 6,28 | 6,15 |
| 78 | $-C(CH_3){=}C(CH_3)-$ | 76-80° | 5,91 | 5,74 |
| 79 | 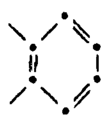 | 166-170° | 5,40 | 5,36 |
| 80 | 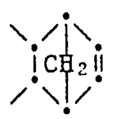 | 150-53° | 5,09 | 4,89 |

Beispiel 81: Gleichzeitige Alkylierung und Acylierung

Eine Mischung von 12,9 g 2,4-Difluoranilin und 24,3 g Orthoessigsäure-triethylester wird mit einem Tropfen $H_2SO_4$ versetzt und am absteigenden Kühler erwärmt bis die Innentemperatur 120 °C erreicht hat. Das Reaktionsgemisch wird im Vakuum destilliert und man erhält bei 86 - 87 °/8 mbar 16,9 g N-Ethyl-N-acetyl-2,4-difluoranilin.

Beispiel 82: Cyclische Anilide (Lactame)

Zu einer Lösung von 11,4 g N-(3-Chlorpivaloyl)-2,4-difluoranilin (Beispiel 4) in 30 ml Methyl-ethyl-keton werden 10,4 g $K_2CO_3$ zugegeben. Die Suspension wird 24 h bei 50 °C gerührt, dann mit 50 ml Wasser versetzt und zweimal mit 50 ml Toluol extrahiert. Die Toluol-Lösung wird über $MgSO_4$ getrocknet und im Vakuum eingedampft. Man erhält 9,3 g rohes 1-(2,4-Difluorphenyl)-3,3-dimethyl-2-azetidinon, das nach Umkristallisation aus Hexan bei 86 - 87 ° schmilzt.

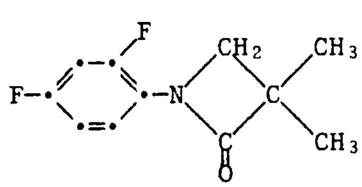

| Analyse: | ber. | C 62,5 | H 5,3 | N 6,6 % |
|---|---|---|---|---|
| | gef. | C 62,3 | H 5,2 | N 6,5 % |

In analoger Weise wird hergestellt die Verbindung 82a

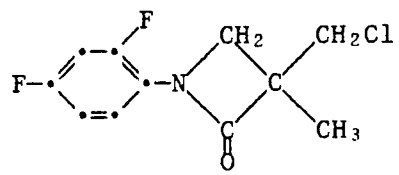

die bei 70° schmilzt.

| Analyse: | ber. | C 53,8 | H 4,1 | N 5,7 | Cl 14,4 % |
|---|---|---|---|---|---|
| | gef. | C 53,7 | H 4,1 | N 5,6 | Cl 14,4 % |

Beispiel 83: 1-(2,4-Difluorphenyl)-2,2,5,5-tetramethyl-1,2,5-azadisilolidin

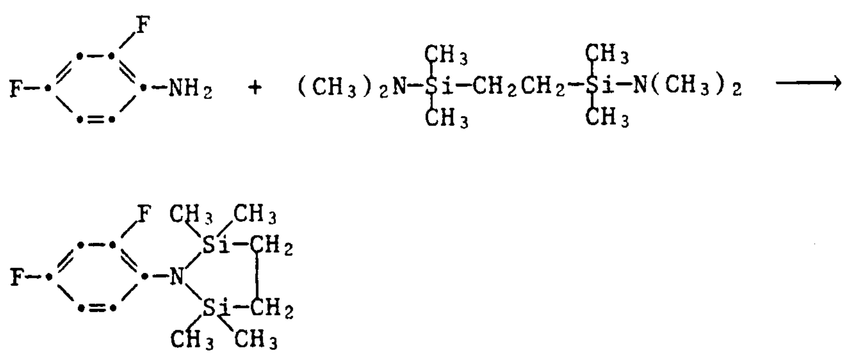

Eine Mischung von 12,9 g 2,4-Difluoranilin und 23,2 g 1,1,4,4-Tetramethyl-1,4-bis(dimethylamino)-disilethylen wird mit 0,5 g Zinkiodid versetzt und unter Stickstoff auf 140°C erwärmt bis kein Dimethylamin mehr entweicht. Das Reaktionsgemisch wird im Vakuum destilliert. Man erhält die Titelverbindung als farblose Flüssigkeit, die bei 119 - 122°/16 mbar destilliert.

Beispiel 84: Alkylierung eines N-Acylanilins

Zu einer Lösung von 3,4 g N-Acetyl-2,4-difluoranilin (Beispiel 2) in 60 ml Toluol werden 0,9 g Triethylbenzylammoniumchlorid, 6,9 g Butylbromid und eine Lösung von 5,7 g KOH in 6 ml Wasser unter Rühren zugegeben. Die entstehende Emulsion wird auf 97° (Rückfluss) erwärmt. Nach 1 h wird die Emulsion auf Raumtemperatur gekühlt und mit 20 ml Wasser verdünnt. Die beiden Phasen werden getrennt und die organische Phase wird über $MgSO_4$ getrocknet und im Vakuum eingedampft. Das flüssige Rohprodukt wird durch Mitteldruck-Chromatographie gereinigt. Man erhält 3,1 g N-Butyl-N-acetyl-2,4-difluoranilin als bräunliches Oel.

| Analyse: | ber. | C 63,42 | H 6,65 | N 6,16 % |
|---|---|---|---|---|
| | gef. | C 63,72 | H 6,80 | N 5,99 % |

Beispiel 85: Umsetzung mit Lactonen

Ein Gemisch aus 64,6 g 2,4-Difluoranilin, 51,7 g Butyrolacton, 2 g p-Toluolsulfonsäure und einigen Tropfen Wasser wird unter Rühren zum Rückfluss (100°C) erwärmt. Das sich bildende Wasser wird durch einen starken Stickstoffstrom abdestilliert. Dabei steigt die Innentemperatur innerhalb von 15 h aus 162°. Nach Abkühlen auf Raumtemperatur wird mit 100 ml Diethylether verdünnt. Die Etherlösung wird erst mit 5 %iger HCl und dann mit 10 %iger NaOH gewaschen, getrocknet und eingedampft. Der kristalline Rückstand wird aus Ethanol Umkristallisiert. Das so erhaltene 1-(2,4-Difluorphenyl)-pyrrolidon-2schmilzt bei 95 - 97°.

| | | | | |
|---|---|---|---|---|
| Analyse: | ber.<br>gef. | C 60,9<br>C 60,8 | H 4,6<br>H 4,7 | N 7,1 %<br>N 7,2 % |

Beispiel 86: N-Ethyl-N-acetyl-3,5-difluoranilin

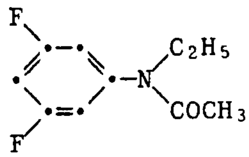

Diese Verbindung wird analog Beispiel 1 durch Acetylierung von N-Ethyl-3,5-difluoranilin hergestellt. Sie siedet bis 10 mbar bei 92 - 93°.

C) Herstellung der Titanocene der Formel I

Beispiel 87: Bis(cyclopentadienyl)-bis-[2,6-difluor-3-(N-ethylpivaloylamino)phenyl]-titan
48,2 g N-Ethyl-N-pivaloyl-2,4-difluoranilin (0.2 Mol) (Beispiel 1) werden in der Mischung von 100 ml Tetrahydrofuran und 300 ml Diethylether unter Argon-Schutzgas bei -75° vorgelegt. Nach dem Zutropfen von 136 ml einer 1,6-molaren Lithiumbutyl-Hexan-Lösung wird noch 30 Minuten bei -75° gerührt. Hierauf setzt man 24,9 g Biscyclopentadienyltitandichlorid (0.1 Mol) als Pulver zu und entfernt die Kühlung. Die Mischung erwärmt sich innert 3 Stunden auf Raumtemperatur. Das Reaktionsgemisch wird 1 l Wasser gegossen und in Portionen mit total 800 ml Ethylacetat extrahiert. Die organische Phase wird mit $Na_2SO_4$ getrocknet und im Vakuum eingedampft. Der Rückstand besteht aus 57 g eines zähflüssigen orangeroten Oels. Durch Behandeln mit n-Hexan kann dieses Oel zur Kristallisation gebracht werden. Man erhält 32,3 g oranger Kristalle vom Schmelzpunkt 215° (aus Ethanol umkristallisiert).

| | | | | | |
|---|---|---|---|---|---|
| Analyse: | ber.<br>gef. | C 65,6<br>C 65,2 | H 6,4<br>H 6,5 | F 11,5<br>F 11,4 | N 4,2 %<br>N 4,2 % |

Beispiele 88 - 142:
In analoger Weise werden die in Tabelle 5 aufgeführten Titanocene hergestellt.

Tabelle 5:

Verbindungen der Formel $(Cp)_2Ti$ 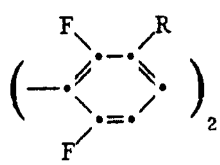

Cp = Cyclopentadienyl

| Beispiel Nr. | Anilinderivat aus Beispiel | R | Fp | Analyse % N ber. | gef. |
|---|---|---|---|---|---|
| 88 | 7 | 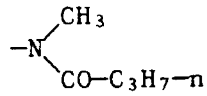 | Oel | 4,7 | 4,6 |
| 89 | 8 | 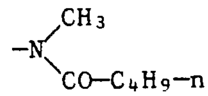 | Oel | 4,4 | 4,5 |
| 90 | 9 | 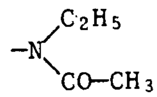 | 131-33° | 4,9 | 5,0 |
| 91 | 10 | 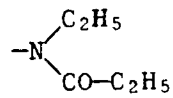 | 125-27° | 4,7 | 4,8 |
| 92 | 11 | 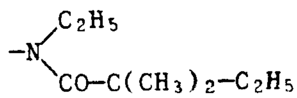 | 177-78° | 4,1 | 4,1 |
| 93 | 12 | 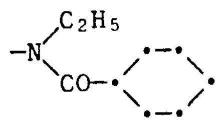 | Oel | 3,9 | 4,1 |
| 94 | 13 | 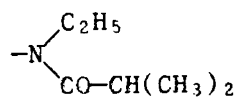 | 185-86° | 4,4 | 4,4 |
| 95 | 14 | 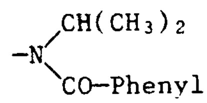 | 120-30° | 3,9 | 3,5 |
| 96 | 17 | 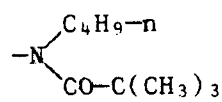 | 85-88° | 3,9 | 3,7 |
| 97 | 18 | 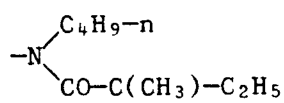 | 143-45° | 3,8 | 3,8 |
| 98 | 18a | 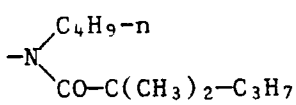 | 135-41° | 3,7 | 3,4 |
| 99 | 20 | 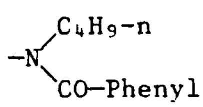 | 180-85° | 3,7 | 3,4 |
| 100 | 21 | 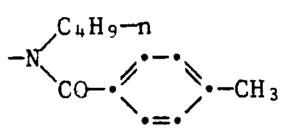 | Glas | 3,6 | 3,3 |

| Beispiel Nr. | Anilinderivat aus Beispiel | R | Fp | Analyse % N ber. | gef. |
|---|---|---|---|---|---|
| 101 | 22 | 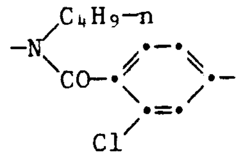 | 205-09° | 3,4 | 3,1 |
| 102 | 23 | 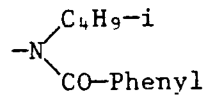 | Glas | 3,7 | 3,5 |
| 103 | 24 | 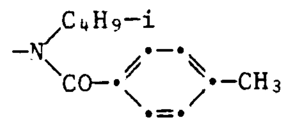 | Glas | 3,6 | 3,0 |
| 104 | 26 | 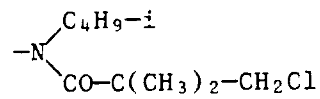 | 95-100° | 3,6 | 3,2 |
| 105 | 27 | 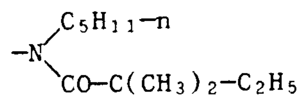 | 119-21° | 3,6 | 3,7 |
| 106 | 29 | 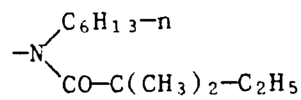 | 102-04° | 3,5 | 3,6 |
| 107 | 30 | 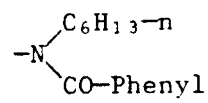 | 78-88° | 3,5 | 3,2 |
| 108 | 31 | 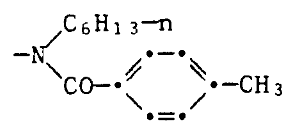 | Glas | 3,3 | 3,1 |
| 109 | 32 | 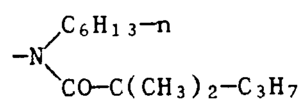 | Glas | 3,4 | 3,2 |
| 110 | 33 | 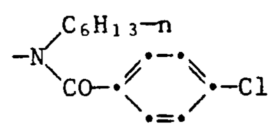 | 208-09° | 3,2 | 2,9 |
| 111 | 34 | 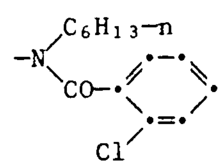 | Glas | 3,2 | 3,1 |
| 112 | 36 | 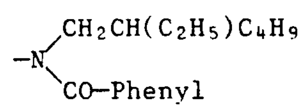 | 80-86° | 3,2 | 3,1 |

| Beispiel Nr. | Anilinderivat aus Beispiel | R | Fp | Analyse % N ber. | gef. |
|---|---|---|---|---|---|
| 113 | 38 | 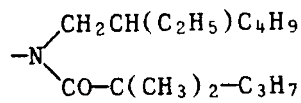 | Glas | 3,2 | 2,6 |
| 114 | 39 | 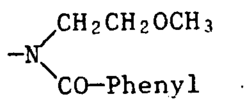 | 204-09° | 3,7 | 3,5 |
| 115 | 40 | 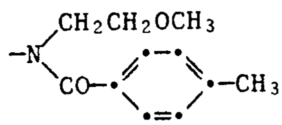 | 183-89° | 3,6 | 3,4 |
| 116 | 41 | 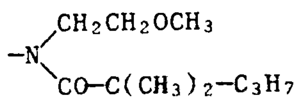 | 55-60° | 3,6 | 3,4 |
| 117 | 42 | 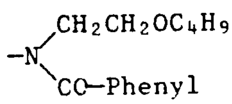 | Glas | 3,3 | 3,1 |
| 117a | 43 | 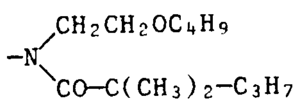 | Glas | 3,3 | 2,6 |
| 118 | 44 | 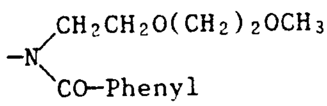 | Glas | 3,2 | 3,1 |
| 118a | 46 | 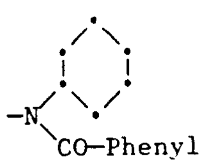 | Glas | 3,5 | 3,0 |
| 119 | 47 | 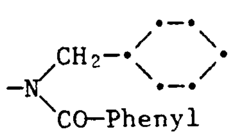 | 125-35° | 3,4 | 3,2 |
| 120 | 48 | 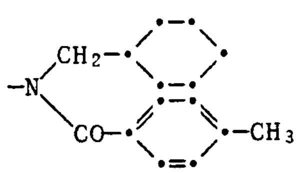 | 130-40° | 3,3 | 2,9 |
| 121 | 49 | 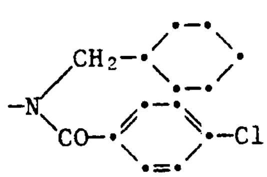 | 133-35° | 3,1 | 2,7 |

| Beispiel Nr. | Anilinderivat aus Beispiel | R | Fp | Analyse % N ber. | Analyse % N gef. |
|---|---|---|---|---|---|
| 122 | 50 | 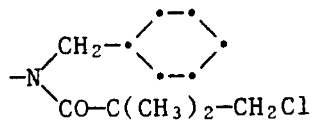 | 195-202° | 3,2 | 3,0 |
| 123 | 51 | 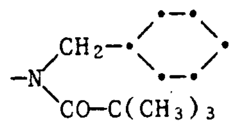 | Glas | 3,5 | 3,1 |
| 124 | 52 | 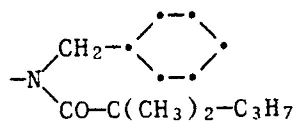 | 90-100° | 3,3 | 3,0 |
| 124a | 53 | 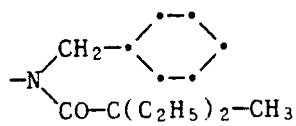 | Glas | 3,5 | 3,1 |
| 125 | 54 | 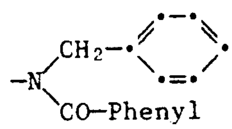 | 105-08° | 3,4 | 2,9 |
| 126 | 55 | 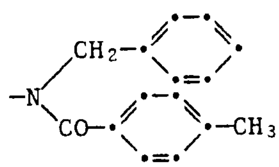 | 110-20° | 3,3 | 2,8 |
| 127 | 56 | 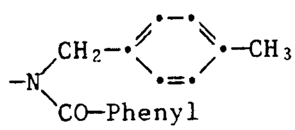 | Glas | 3,3 | 3,1 |
| 128 | 57 | 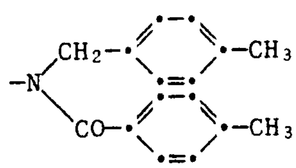 | Glas | 3,2 | 2,9 |
| 129 | 58 | 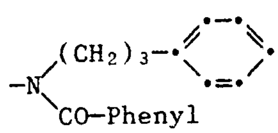 | Glas | 3,2 | 2,8 |
| 130 | 59 | 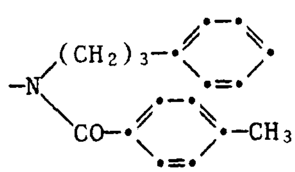 | Glas | 3,1 | 2,9 |

| Beispiel Nr. | Anilinderivat aus Beispiel | R | Fp | Analyse % N ber. | gef. |
|---|---|---|---|---|---|
| 131 | 60 | 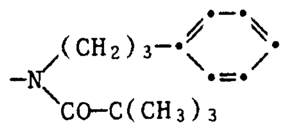 | 165-70° | 3,3 | 2,9 |
| 132 | 61 | 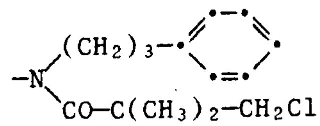 | 80-85° | 3,1 | 2,9 |
| 133 | 62 | 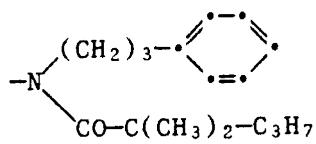 | 70-80° | 3,2 | 2,9 |
| 134 | 64 | 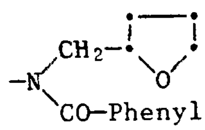 | Glas | 3,5 | 3,1 |
| 135 | 65 | 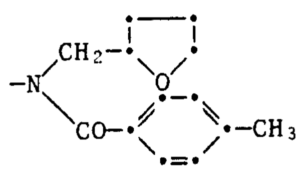 | Glas | 3,3 | 3,0 |
| 136 | 66 | 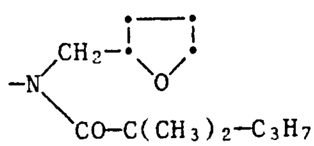 | 75-80° | 3,4 | 3,2 |
| 137 | 69 | 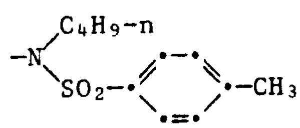 | 165-70° | 3,3 | 3,0 |
| 138 | 70 | 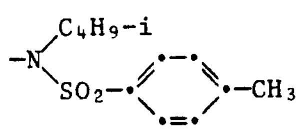 | 227-31° | 3,3 | 2,9 |
| 139 | 71 | 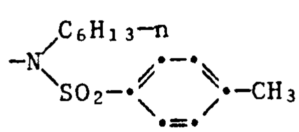 | 76-80° | 3,1 | 2,8 |
| 140 | 72 | 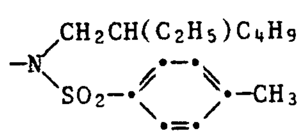 | Glas | 2,9 | 2,7 |

| Beispiel Nr. | Anilinderivat aus Beispiel | R | Fp | Analyse % N ber. | gef. |
|---|---|---|---|---|---|
| 141 | 73 | 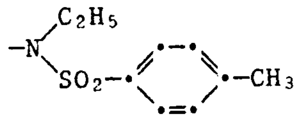 | 231-33° | 3,5 | 3,3 |
| 142 | 82 | 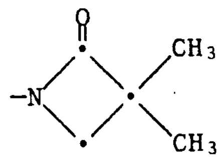 | 130-40° | 4,7 | 4,3 |

Beispiel 143: In analoger Weise wird aus N-Ethyl-N-acetyl-3,5-difluoranilin (Beispiel 86) die Verbindung

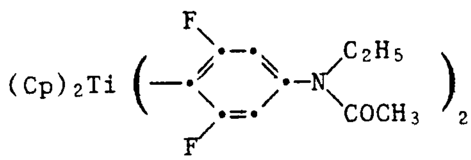

hergestellt, die bei 168-169° schmilzt.

| Analyse | ber. | C 62,7 | H 5,3 | N 4,9 % |
|---|---|---|---|---|
| | gef. | C 62,6 | H 5,4 | N 5,0 % |

Beispiele 144-146: Methylcyclopentadienyltitanocene
Verwendet man anstelle von Bis(cyclopentadienyl)titandichlorid das Bis(methylcyclopentadienyl)-titandichlorid zur Umsetzung mit den entsprechenden Anilinderivaten in Analogie zu Beispiel 87, so erhält man die folgenden Verbindungen der Formel

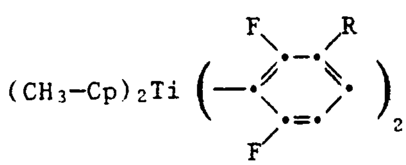

| Beispiel Nr. | Anilinderivat aus Beispiel | R | Fp | Analyse % N ber. | gef. |
|---|---|---|---|---|---|
| 144 | 9 | 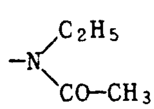 | 138-39° | 4,6 | 4,6 |

| Beispiel Nr. | Anilinderivat aus Beispiel | R | Fp | Analyse % N ber. | gef. |
|---|---|---|---|---|---|
| 145 | 10 | 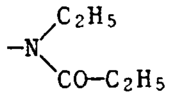 | 139-41° | 4,4 | 4,5 |
| 146 | 29 | 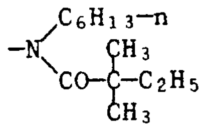 | 50-60° | 3,4 | 3,2 |

Beispiel 147: Bis(cyclopentadienyl)-bis(2,6-difluor-3-aminophenyl)-titan

a) 87 g (0,32 Mol) 1-(2,4-Difluorphenyl)-2,2,5,5-tetramethyl-1,2,5-azadisilolidin werden (Beispiel 83) in einer Mischung von 160 ml Tetrahydrofuran und 480 ml Diethylether gelöst und unter Stickstoff als Schutzgas und Lichtausschluss auf -75° gekühlt. Dann werden unter Kühlung 218 ml 1,6 molare Lithiumbutyl-Hexan-Lösung zugetropft und 30 Minuten nachgerührt bei -75°. Anschliessend setzt man 39,5 g (0,16 Mol) Bis(cyclopentadien)-titandichlorid als Pulver zu. Die Temperatur des Reaktionsgemisches lässt man hierauf innerhalb von 12 Stunden auf Raumtemperatur ansteigen. Die Suspension wird filtriert und der Rückstand mit 100 ml Diethylether gewaschen. Das Filtrat wird am Rotationsverdampfer vollständig eingedampft. Man erhält 139 g eines orangen Oels. Dieses Oel wird mit 150 ml Acetonitril digeriert, wobei Kristallisation eintritt. Nach der Filtration des kristallisierten Produktes erhält man 71,6 g gelborange Kristalle vom Smp. 207-211°.

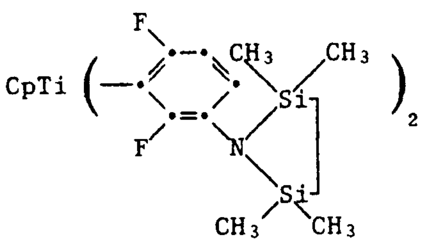

| Analyse: | ber. | C 56,8 | H 6,4 | N 3,9 | F 10,6 | Si 15,6 % |
|---|---|---|---|---|---|---|
| | gef. | C 56,0 | H 6,4 | N 3,9 | F 10,5 | Si 15,6 % |

b) 30 g des unter a) beschriebenen Titanocens werden unter Lichtausschluss in einer Mischung von 250 ml Dioxan und 10 ml Methanol gelöst. Nach Zusatz von 0,6 g p-Toluolsulfosäure wird 3 h bei 40°C gerührt. Die Reaktionslösung wird auf 0° gekühlt und unter Rühren zu 250 ml Eiswasser zugetropft, wobei das Rohprodukt in Form von orangeroten Kristallen ausfällt, die über 200°C unter Zersetzung schmelzen.

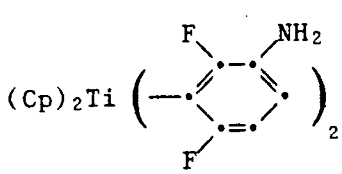

| Analyse: | ber. | C 60,9 | H 4,2 | F 17,5 | N 6,5 % |
|---|---|---|---|---|---|
| | gef. | C 61,3 | H 4,2 | F 17,5 | N 6,4 % |

Beispiele 148 - 168: Bis(cyclopentadienyl)-bis[2,6-difluor-3-(acetylamino)phenyl]-titan

In einem Sulfierkolben werden 4,3 g (0,01 Mol) Bis(cyclopentadienyl)-bis(2,6-difluor-3-aminophenyl)-titan (Beispiel 147) und 4,4 g (0,044 Mol) Triethylamin in 30 ml Dimethylformamid gelöst. Dann werden unter Rühren bei 0 - 5° innerhalb 20 Minuten 1,6 g (0,02 Mol) Acetylchlorid zugetropft. Es bildet sich eine rote Suspension, die noch 5 Stunden, bis zum Verschwinden des Eduktes im DC, bei Raumtemperatur nachgerührt wird. Die Suspension wird mit 50 ml Wasser verdünnt und dann zweimal mit 100 ml Ethylacetat extrahiert. Die organischen Phasen werden abgetrennt, mit $MgSO_4$ getrocknet und im Vakuum eingeengt. Das erhaltene braune Oel wird in 50 ml Diethylether erwärmt und die Lösung dann langsam mit 400 ml Hexan verdünnt. Anschliessend wird auf Raumtemperatur gekühlt und filtriert. Man erhält 4,3 g orange Kristalle mit einem Schmelzpunkt von 85°.

In analoger Weise werden die in Tabelle 6 aufgeführten Verbindungen hergestellt.

Tabelle 6:

Verbindungen der Formel $(Cp)_2Ti$ 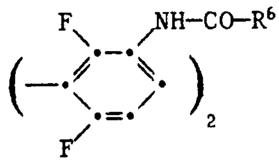

| Beispiel Nr. | $R^6$ | Fp | Analyse % N ber. | gef. |
|---|---|---|---|---|
| 148 | $CH_3$ | 85° | 5,4 | 5,8 |
| 149 | $CF_3$ | 146-48° | 4,5 | 4,3 |
| 150 | $C_3H_7$-n | 170-75° | 4,9 | 5,1 |
| 151 | $-CH(CH_3)_2$ | 195-200° | 4,9 | 5,1 |
| 152 | $-CH(CH_3)C_2H_5$ | 120-30° | 4,7 | 4,6 |
| 153 | $-C(CH_3)_3$ | ca. 150° | 4,7 | 5,5 |
| 154 | $-C(CH_3)_2-CH_2Cl$ | 125-45° | 4,2 | 3,3 |
| 155 | $-C(CH_2Cl)_2-CH_3$ | 108-15° | 3,8 | 3,4 |
| 156 | $-CH(C_2H_5)_2$ | ca. 180° | 4,4 | 4,7 |
| 157 | $-C(CH_3)_2-C_2H_5$ | Sirup | 4,4 | 3,9 |
| 158 | $-C(CH_3)_2-C_3H_7$ | 50-70° | 4,3 | 3,7 |
| 159 | $-CH(C_2H_5)-C_4H_9$ | 155° | 4,1 | 4,8 |
| 160 | $-C_9H_{19}$-n | 60-65° | 3,9 | 3,8 |
| 161 | $-C_{17}H_{35}$-n | 67-69° | 2,9 | 3,3 |
| 162 | 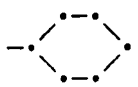 | 150-60° | 4,3 | 4,3 |
| 163 | 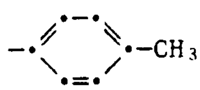 | 204-06° | 4,2 | 4,1 |
| 164 | 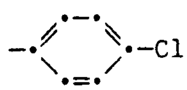 | 233-36° | 3,9 | 3,8 |
| 165 | 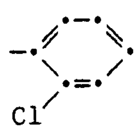 | 225-30° | 3,9 | 3,8 |
| 166 | 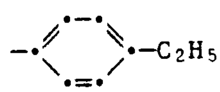 | 160-70° | 4,0 | 3,6 |
| 167 | 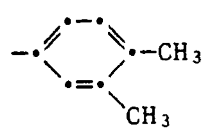 | 80-90° | 4,0 | 3,3 |
| 168 | $-CH_2CH_2-$ 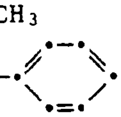 | 70-80° | 4,0 | 5,3 |

Beispiel 169: N-Allylierung

In eine Emulsion von 4,1 g Bis(cyclopentadienyl)-bis(2,6-difluor-3-acetylaminophenyl)-titan (Beispiel 148) in 120 ml $CH_2Cl_2$ und 53,2 g 30 %ige Natronlauge werden 0,8 g Triethylbenzylammoniumchlorid und 4,8 g Allylbromid unter Rühren zugegeben. Nach 5 h ist die Reaktion beendet. Die Emulsion wird mit 50 ml Wasser verdünnt und mit 100 ml $CH_2Cl_2$ extrahiert. Die organische Phase wird abgetrennt, über $MgSO_4$ getrocknet und im Vakuum eingedampft. Das hinterbleibende Oel wird in wenig Ethylacetat gelöst und durch Zusatz von Hexan zur Kristallisation gebracht. Man erhält 3,6 g Bis(cyclopentadienyl)-bis[2,6-difluor-3-(N-allyl-acetylamino)-phenyl]-titan in Form von orangen Kristallen, die bei 168 - 172° schmelzen.

| | | | | |
|---|---|---|---|---|
| Analyse: | ber. | C 64,2 | H 5,0 | N 4,7 % |
| | gef. | C 63,6 | H 5,2 | N 4,4 % |

Beispiel 170: Bis(cyclopentadienyl)-bis(2,6-difluor-3-diacetylaminophenyl)-titan

8,7 g Bis(cyclopentadienyl)-bis(2,6-difluor-3-aminophenyl)-titan (Beispiel 147) werden in 100 ml Pyridin gelöst und bei 20 - 30° mit 9,4 g Acetylchlorid versetzt. Das Reaktionsgemisch wird 2 h bei Raumtemperatur und danach 5 Tage bei 60° gerührt. Dann werden weitere 9,4 g Acetylchlorid zugegeben und nochmals 5 Tage auf 60° erwärmt. Nach Erkalten wird die Reaktionslösung mit Wasser verdünnt und mit Ethylacetat extrahiert. Die organische Phase wird mit 1 N HCl und Wasser gewaschen, über $MgSO_4$ getrocknet und eingedampft. Der ölige Rückstand wird durch Chromatographie über einer $SiO_2$-Säule gereinigt. Man erhält die Titelverbindung als orange Kristalle, die bei 211° unter Zersetzung schmelzen.

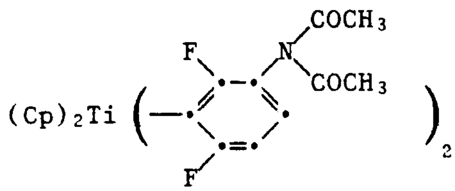

| | | | | |
|---|---|---|---|---|
| Analyse: | ber. | C 59,8 | H 4,4 | N 4,6 % |
| | gef. | C 58,9 | H 4,4 | N 4,4 % |

Beispiele 171 - 174: Carbamate

4,3 g Bis(cyclopentadienyl)-bis-(2,6-difluor-3-aminophenyl)titan (Verbindung aus Bsp. 147) und 2,4 g Triethylamin werden in 100 ml DMF gelöst und die Lösung auf 0° gekühlt. Die Lösung wird tropfenweise mit 3,0 g Chlorameisensäureisobutylester versetzt und 7 Std. bei 0° gerührt. Nach dieser Zeit werden nochmals 2,4 g Triethylamin und 3,0 g Chlorameisensäureisobutylester zugegeben und über Nacht bei 0°C gerührt. Anschliessend wird mit 100 ml Essigester und 100 ml Wasser versetzt, die Phasen getrennt und die organische Phase über $MgSO_4$ getrocknet. Nach dem Einengen wird ein orange-braunes Oel erhalten, das durch Chromatographie an Kieselgel (Elutionsmittel: Hexan/Essigester 3:1) weiter gereinigt wird. Die das Produkt enthaltende Fraktion wird aus Ether/Hexan umkristallisiert. Fp 90° (Zersetzung).

In analoger Weise werden die in Tabelle 7 aufgeführten Verbindungen hergestellt.

Tabelle 7:

Verbindungen der Formel 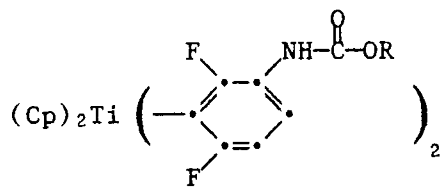

| Beispiel Nr. | R | Fp | Analyse % N ber. | Analyse % N gef. |
|---|---|---|---|---|
| 171 | $C_4H_9$-i | 90° (Zers.) | 4,4 | 4,2 |
| 172 | $C_2H_5$ | 90° (Zers.) | 4,8 | 4,8 |
| 173 | $-CH_2CH_2Cl$ | 90° (Zers.) | 4,3 | 3,9 |
| 174 | 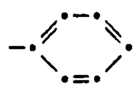 | 110° (Zers.) | 4,2 | 4,2 |

Beispiel 175: Bis(cyclopentadienyl)-bis[2,6-difluor-3-(3,3-dimethylureido)-phenyl] -titan

8,7 g Bis(cyclopentadienyl)-bis-(2,6-difluor-3-aminophenyl)titan (Beispiel 147) und 3,8 g Pyridin werden in 150 ml DMF gelöst und auf 0 ° C gekühlt. Die Lösung wird tropfenweise mit 5,2 g Dimethylcarbamoylchlorid versetzt und während 6 Std. bei 0 ° gerührt. Anschliessend wird 10 Std. bei Raumtemperatur gerührt. Danach werden nochmals 5,2 g Dimethylcarbamoylchlorid zugegeben und auf 40 ° erwärmt. Nach 7 Stunden wird auf Wasser ausgegossen, mit Toluol aufgenommen und über $MgSO_4$ getrocknet. Nach dem Einengen wird der Rückstand durch Chromatographie an Kieselgel [Elutionsmittel: Hexan/Essigester (Methanol 2:7:1) gereinigt. Man erhält die Titelverbindung als glasigen Feststoff, der sich bei 110 ° zersetzt.

| Analyse | | | | |
|---|---|---|---|---|
| | ber. | C 58,34 | H 4,90 | N 9,72 % |
| | gef. | C 57,29 | H 5,37 | N 8,83 % |

Beispiele 176 - 179: Harnstoff- und Thioharnstoffderivate

Zu einer Suspension von 8,7 g Bis(cyclopentadienyl)-bis(2,6-difluor-3-aminophenyl)-titan (Beispiel 147) in 50 ml Tetrahydrofuran werden 0,1 g Triethylamin zugegeben und bei 0 - 5 ° C unter Rühren 0,04 Mol des jeweiligen Isocyanates oder Isothiocyanates zugetropft. Dann lässt man die Temperatur langsam auf 25 ° steigen und rührt bei dieser Temperatur 10 Stunden. Die resultierende Lösung wird im Vakuum eingedampft und der ölige Rückstand mit einem Gemisch Ethylacetat/Ethanol 1:1 zur Kristallisation gebracht. Nach dieser Methode werden die in Tabelle 8 aufgeführten Verbindungen hergestellt.

Tabelle 8

Verbindungen der Formel 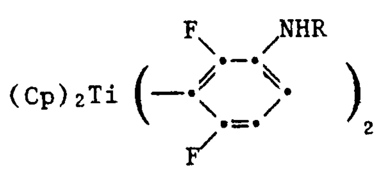

| Beispiel Nr. | R | Fp | Analyse % N ber. | gef. |
|---|---|---|---|---|
| 176 | $-CO-NH-C_4H_9$ | 210° (Zers.) | 8,9 | 8,7 |
| 177 | -CO-NH- 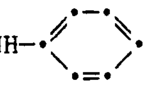 | > 250° (Zers.) | 8,3 | 8,2 |
| 178 | $-CS-NH-C_4H_9$ | 182 - 84° | 8,4 | 8,3 |
| 179 | -CS-NH- 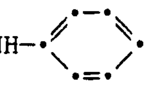 | 210° (Zers.) | 8,0 | 7,7 |

Beispiele 180 - 182: Cyclische Imidderivate

Eine Suspension von 4,3 g Bis(cyclopentadienyl)-bis(2,6-difluor-3-aminophenyl)-titan (Beispiel 147) und 2,4 g Bernsteinsäureanhydrid in 100 ml Toluol wird unter Zusatz von 0,2 g 4-Dimethylaminopyridin während 24 h am Wasserabscheider zum Rückfluss erwärmt. Die Reaktionslösung wird im Vakuum eingedampft. Der ölige Rückstand kristallisiert beim Stehen und wird aus Ethanol umkristallisiert. Das erhaltene Bis-(cyclopentadienyl)-bis[2,6-difluor-3-(pyrrolidin-2,5-dion-1-yl)-phenyl]-titan schmilzt bei 251 - 253° unter Zersetzung.

In analoger Weise werden die folgenden Verbindungen hergestellt.

Tabelle 9:

Verbindungen der Formel $(Cp)_2Ti$ 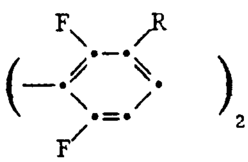

| Beispiel Nr. | R | Fp | Analyse % N ber. | gef. |
|---|---|---|---|---|
| 180 | 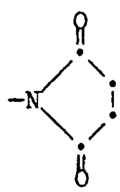 | 251 - 53°(Z) | 4,5 | 4,3 |
| 181 | 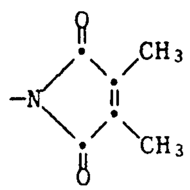 | 208 - 10° (Z) | 4,3 | 4,1 |

| Beispiel Nr. | R | Fp | Analyse % N ber. | gef. |
|---|---|---|---|---|
| 182 | 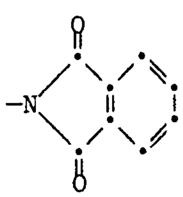 | 191 - 93° (Z) | 4,0 | 3,7 |

Beispiele 183 - 189: N-Sulfonylierung

8,7 g Bis(cyclopentadienyl)-bis(2,6-difluor-3-aminophenyl)-titan (Beispiel 147) werden in einem Gemisch von 50 ml Toluol und 50 ml Dimethylformamid suspendiert. Nach Zusatz von 3,8 g Pyridin wird die Suspension auf 0° gekühlt und bei dieser Temperatur eine Lösung von 5,5 g Methansulfochlorid in 50 ml Toluol zugetropft. Die Suspension wird 5 h bei 0° gerührt und dann auf Wasser gegossen. Das Produkt wird mit Ethylacetat extrahiert, die organische Phase mit 1 N HCl und Wasser gewaschen, über $MgSO_4$ getrocknet und eingedampft. Der feste Rückstand wird mit Ethanol digeriert, filtriert und getrocknet. Man erhält 7,1 g Bis(cyclopentadienyl)-bis(2,6-difluor-3-methylsulfonamidophenyl)-titan als gelbes Pulver, das bei 209 - 11° schmilzt.

In analoger Weise werden die in Tabelle 10 aufgeführten Verbindungen hergestellt.

Tabelle 10:

Verbindungen der Formel $(Cp)_2Ti$ 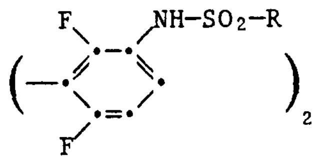

| Beispiel Nr. | R | Fp | Analyse % N ber. | gef. |
|---|---|---|---|---|
| 183 | $-CH_3$ | 209 - 11° | 4,7 | 4,3 |
| 184 | $-C_2H_5$ | Glas | 4,5 | 4,2 |
| 185 | $-C_8H_{17}-n$ | Glas | 3,6 | 3,4 |
| 186 | 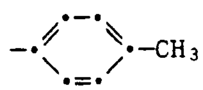 | 208 - 10° | 3,8 | 3,4 |

| Beispiel Nr. | R | Fp | Analyse % N ber. | gef. |
|---|---|---|---|---|
| 187 | 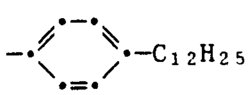 | Glas | 2,7 | 2,6 |
| 188 | 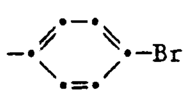 | 172 - 76° (Z) | 3,2 | 2,8 |
| 189 | 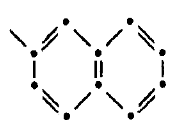 | 190 - 92° (Z) | 3,4 | 3,1 |

Beispiel 190: Bis(cyclopentadienyl)-bis[2,6-difluor-3-(N-methyl-p-dodecylphenylsulfonamido)-phenyl]-titan

5,3 g Bis(cyclopentadienyl)-bis[2,6-difluor-3-(4-dodecylphenylsulfonamido)-phenyl]-titan (Beispiel 187) und 2,8 g trockenes $K_2CO_3$ werden in 50 ml Aceton gerührt und mit 1,7 g Methyliodid versetzt. Nach 2 h bei Raumtemperatur wird das Reaktionsgemisch filtriert und das Filtrat eingedampft. Man erhält 4,6 g eines glasigen Rückstandes.

| Analyse: | ber.<br>gef. | C 66,7<br>C 66,1 | H 7,3<br>H 7,3 | N 2,6<br>N 2,4 | S 5,9 %<br>S 6,0 % |
|---|---|---|---|---|---|

Beispiel 191: Bis(cyclopentadienyl)-bis[2,6-difluor-3-(N-hexyl-p-methylphenylsulfonamido)-phenyl]-titan
Analog Beispiel 190 wird das Produkt von Beispiel 186 mit 1-Jodhexan bei 50° umgesetzt. Das Produkt ist ein oranges Harz.
Analyse: N ber. 3,1 % gef. 2,8 %

Beispiel 192: Bis(cyclopentadienyl)-bis(2,6-difluor-3-isocyanatophenyl)-titan
4,3 g Bis(cyclopentadienyl)-bis(2,6-difluor-3-aminophenyl)-titan (Beispiel 147) wird in 50 ml Dichlorbenzol suspendiert. Dazu gibt man 2,0 g Bis(trichlormethyl)carbonat und tropft 4,0 g Triethylamin zu. Die Reaktion ist schwach exotherm. Anschliessend wird 2 h auf 70° erwärmt. Nach dem Abkühlen wird das Reaktionsgemisch filtriert und im Vakuum eingedampft.

Der teilkristalline Rückstand wird mit Diethylether digeriert. Die Kristalle werden verworfen und die Lösung eingedampft. Der Rückstand word in $CH_2Cl_2$ gelöst und durch Zugabe von Hexan das Isocyanat ausgefällt. Das abfiltrierte und getrocknete Produkt ist ein oranges Pulver, das bis 224° unter Zersetzung schmilzt.

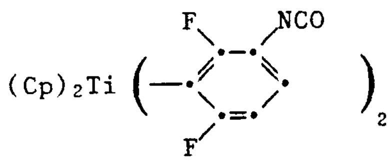

| Analyse: | ber. | C 59,3 | H 2,9 | N 5,8 % |
|---|---|---|---|---|
| | gef. | C 58,6 | H 3,5 | N 5,4 % |

Beispiel 193: Bis(cyclopentadienyl)-bis[2,6-difluor-3-(dimethylaminosulfonylamino)phenyl]-titan

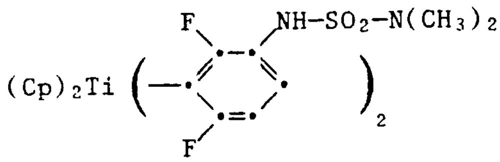

In eine Lösung von 8,7 g der Aminoverbindung von Beispiel 147 und 3,5 g Pyridin in 100 ml Dimethylformamid werden bei 0° 6,3 g Dimethylsulfamoylchlorid zugetropft. Das Reaktionsgemisch wird 4 h bei 0° und anschliessend 10 h bei Raumtemperatur gerührt. Dann werden 200 ml Wasser und 200 ml Ethylacetat zugegeben. Die organische Phase wird abgetrennt, mit 1 N HCl und Wasser gewaschen, über $MgSO_4$ getrocknet und im Vakuum eingedampft. Der viskose Rückstand wird mit 20 ml Ethylacetat kurz erwärmt. In der Kälte fällt ein gelbes Pulver aus, das nach Trocknung bei 185-86° schmilzt.

| Analyse: | ber. | N 8,6 | S 9,9 % |
|---|---|---|---|
| | gef. | N 8,4 | S 9,7 % |

D) Anwendungsbeispiele

Beispiel 194: Photohärtung eines Acrylat-Gemisches
Es wird eine photohärtbare Zusammensetzung hergestellt durch Mischen der folgenden Komponenten:

| | Feststoffgehalt |
|---|---|
| 150,30 g Scripset 540[1)] (30 %-ige Lsg. in Aceton)<br>48.30 g Trimethylolpropantriacrylat<br>6,60 g Polyethylenglykoldiacrylat<br>0,08 g Kristallviolett | 45,1 g<br>48,3 g<br>6,6 g |
| 205,28 g | 100,0 g |

[1)] Polystyrol-Maleinsäureanhydrid-Copolymer (Monsanto)

Portionen dieser Zusammensetzung werden mit jeweils 0,3 % (bezogen auf den Feststoffgehalt) an Photoinitiator vermischt. Alle Operationen werden unter Rotlicht oder Gelblicht ausgeführt.

Die mit Initiator versetzten Proben werden in einer Stärke von 150 µm auf 200 µm Aluminiumfolie (10 x 15 cm) aufgetragen. Das Lösungsmittel wird durch Erwärmung auf 60°C während 15 Minuten im Umluftofen entfernt. Auf die flüssige Schicht wird eine 76 µm dicke Polyesterfolie gelegt und auf diese ein standardisiertes Testnegativ mit 21 Stufen verschiedener optischer Dichte (Stouffer-Keil) gelegt. Darüber wird eine zweite Polyesterfolie gelegt und das so erhaltene Laminat auf einer Metallplatte fixiert. Die Probe wird dann mit einer 5 KW-Metallhalogenid-Lampe im Abstand von 30 cm belichtet und zwar in einer ersten Testreihe 10 Sekunden, einer zweiten Testreihe 20 Sekunden und einer dritten Testreihe 40 Sekunden. Nach der Belichtung werden die Folien und die Maske entfernt, die belichtete Schicht in einem Ultraschallbad 120 Sekunden mit Entwickler A entwickelt und anschliessend bei 60° 15 Minuten im Umluftofen getrocknet. Die Empfindlichkeit des verwendeten Initiatorsystems wird durch die Angabe der letzten klebefrei abgebildeten Keilstufe charakterisiert. Je höher die Zahl der Stufen ist, desto empfindlicher ist das System. Eine Erhöhung um zwei Stufen bedeutet dabei etwa eine Verdopplung der Härtungsgeschwindigkeit. Die Ergebnisse sind in Tabelle 11 angegeben. Entwickler A enthält 15 g Natriummetasilikat$\cdot 9H_2O$; 0,16 g KOH; 3 g Polyethylenglykol 6000; 0,5 g Lävulinsäure und 1000 g deionisiertes Wasser.

Tabelle 11:

| Titanocen Beispiel | Zahl der abgebildeten Stufen nach 10s | 20s | 40s Belichtung |
|---|---|---|---|
| 87 | 12 | 14 | 17 |
| 90 | 10 | 12 | 15 |
| 91 | 9 | 11 | 13 |
| 92 | 12 | 14 | 16 |
| 94 | 10 | 12 | 15 |
| 95 | 9 | 12 | 15 |
| 96 | 11 | 13 | 16 |
| 97 | 12 | 14 | 16 |
| 100 | 8 | 12 | 14 |
| 101 | 7 | 10 | 13 |
| 103 | 6 | 11 | 13 |
| 106 | 11 | 13 | 16 |
| 108 | 8 | 11 | 13 |
| 109 | 9 | 13 | 15 |
| 110 | 8 | 11 | 14 |
| 111 | 9 | 13 | 15 |
| 112 | 8 | 11 | 14 |
| 113 | 8 | 12 | 14 |
| 114 | 8 | 11 | 15 |
| 115 | 8 | 10 | 12 |
| 116 | 7 | 11 | 13 |
| 117 | 8 | 11 | 12 |
| 117a | 9 | 11 | 13 |
| 118 | 7 | 10 | 13 |
| 118a | 7 | 9 | 11 |
| 119 | 8 | 12 | 14 |
| 121 | 7 | 11 | 13 |
| 124 | 8 | 11 | 15 |
| 124a | 9 | 12 | 15 |
| 126 | 7 | 9 | 11 |
| 127 | 8 | 11 | 13 |
| 128 | 6 | 10 | 13 |
| 130 | 8 | 10 | 13 |
| 131 | 7 | 12 | 14 |
| 135 | 8 | 11 | 13 |
| 136 | 7 | 11 | 13 |
| 137 | 9 | 12 | 14 |
| 138 | 6 | 8 | 12 |

| Titanocen Beispiel | Zahl der abgebildeten Stufen nach 10s | 20s | 40s Belichtung |
|---|---|---|---|
| 140 | 8 | 11 | 13 |
| 141 | 8 | 12 | 15 |
| 142 | 10 | 14 | 16 |
| 144 | 7 | 10 | 12 |
| 145 | 7 | 10 | 12 |
| 146 | 7 | 10 | 13 |
| 147 | 8 | 10 | 12 |
| 148 | 11 | 13 | 16 |
| 149 | 9 | 12 | 14 |
| 150 | 10 | 14 | 17 |
| 151 | 10 | 13 | 15 |
| 153 | 10 | 14 | 16 |
| 154 | 10 | 11 | 13 |
| 156 | 9 | 14 | 17 |
| 157 | 10 | 13 | 16 |
| 158 | 11 | 13 | 15 |
| 159 | 10 | 13 | 16 |
| 160 | 11 | 14 | 16 |
| 161 | 8 | 12 | 15 |
| 162 | 10 | 13 | 16 |
| 164 | 8 | 11 | 13 |
| 165 | 6 | 9 | 12 |
| 166 | 9 | 13 | 15 |
| 167 | 7 | 10 | 13 |
| 168 | 10 | 14 | 16 |
| 170 | 9 | 12 | 15 |
| 171 | 11 | 13 | 16 |
| 174 | 10 | 14 | 16 |
| 176 | 9 | 12 | 14 |
| 177 | 9 | 12 | 14 |
| 178 | 10 | 13 | 16 |
| 179 | 8 | 11 | 13 |
| 180 | 11 | 14 | 16 |
| 181 | 12 | 15 | 17 |
| 182 | 12 | 14 | 17 |
| 183 | 11 | 13 | 16 |
| 184 | 10 | 12 | 15 |
| 185 | 10 | 14 | 16 |
| 186 | 9 | 13 | 15 |
| 187 | 10 | 12 | 15 |
| 188 | 9 | 12 | 13 |
| 189 | 7 | 12 | 14 |
| 190 | 8 | 12 | 15 |
| 191 | 9 | 11 | 13 |
| 192 | 9 | 12 | 15 |
| 193 | 12 | 15 | 17 |

Beispiel 195: Photohärtung eines Monomer-Polymer-Gemisches

Es wird eine photohärtbare Zusammensetzung hergestellt durch Mischen der folgenden Komponenten:

| | |
|---|---|
| 37,64 g | Sartomer SR 444 (Pentaerythritol-triacrylat) (Sartomer Company, Westchester) |
| 10,76 g | Cymel 301 Hexamethoxymethylmelamin (Cyanamid) |
| 47,30 g | Carboset 525 (Thermoplastisches Polyacrylat mit Carboxylgruppen/B.F. Goodrich) |
| 4,30 g | Polyvinylpyrrolidon PVP (GAF) |
| 100,00 g | der obigen Mischung |
| 0,50 g | Irgalitgrün GLN |
| 319,00 g | Methylenchlorid |
| 30,00 g | Methanol |
| 450,00 g | |

Portionen dieser Zusammensetzung werden mit jeweils 0,3 % (bezogen auf Feststoff) der in der folgenden Tabelle angegebenen Titanocene vermischt. Alle Operationen werden unter Rotlicht oder Gelblicht ausgeführt.

Die mit Initiator versetzten Proben werden in einer Stärke von 200 µm auf 200 µm Aluminiumfolie (10 x 15 cm) aufgetragen. Das Lösungsmittel wird durch Erwärmung auf 60°C während 15 Minuten im Umluftofen entfernt. Auf die flüssige Schicht wird eine 76 µm dicke Polyesterfolie gelegt und auf diese ein standardisiertes Testnegativ mit 21 Stufen verschiedener optischer Dichte (Stouffer-Keil) gelegt. Darüber wird eine zweite Polyesterfolie gelegt und das so erhaltene Laminat auf einer Metallplatte fixiert. Die Probe wird dann mit einer 5 KW-Metallhalogenid-Lampe im Abstand von 30 cm belichtet und zwar in einer ersten Testreihe 10 Sekunden, in einer zweiten Testreihe 20 Sekunden und in einer dritten Testreihe 40 Sekunden. Nach der Belichtung werden die Folien und die Maske entfernt, die belichtete Schicht in einem Ultraschallbad 240 Sekunden mit Entwickler A entwickelt und anschliessend bei 60° 15 Min. im Umluftofen getrocknet. Die Empfindlichkeit des verwendeten Initiatorsystems wird durch die Angabe der letzten klebefrei abgebildeten Keilstufe charakterisiert. Je höher die Zahl der Stufen ist, desto empfindlicher ist das System. Eine Erhöhung um zwei Stufen bedeutet dabei etwa eine Verdopplung der Härtungsgeschwindigkeit. Die Ergebnisse sind in Tabelle 12 angegeben.

Tabelle 12:

| Titanocen Beispiel | Zahl der abgebildeten Stufen nach 10s | 20s | 40s Belichtung |
|---|---|---|---|
| 87 | 12 | 14 | 17 |
| 90 | 8 | 10 | 13 |
| 91 | 7 | 10 | 12 |
| 92 | 12 | 14 | 16 |
| 94 | 12 | 14 | 17 |
| 95 | 9 | 12 | 15 |
| 96 | 11 | 13 | 15 |
| 97 | 11 | 13 | 16 |
| 100 | 9 | 12 | 15 |
| 101 | 9 | 12 | 14 |
| 103 | 9 | 12 | 14 |
| 106 | 11 | 13 | 15 |
| 108 | 8 | 11 | 13 |
| 109 | 10 | 13 | 15 |
| 110 | 8 | 10 | 13 |
| 111 | 8 | 10 | 13 |
| 112 | 9 | 12 | 14 |
| 113 | 8 | 12 | 14 |
| 114 | 9 | 12 | 14 |
| 115 | 8 | 12 | 14 |
| 116 | 9 | 12 | 15 |
| 117 | 9 | 12 | 14 |
| 117a | 9 | 11 | 14 |
| 118 | 7 | 11 | 14 |
| 118a | 7 | 10 | 12 |
| 119 | 9 | 12 | 14 |
| 121 | 7 | 10 | 13 |
| 124 | 8 | 11 | 13 |
| 124a | 10 | 12 | 14 |
| 126 | 6 | 9 | 12 |
| 127 | 7 | 11 | 13 |
| 128 | 7 | 10 | 13 |
| 130 | 8 | 10 | 12 |
| 131 | 8 | 11 | 14 |
| 134 | 7 | 11 | 13 |
| 135 | 9 | 12 | 13 |

| Titanocen | Zahl der abgebildeten Stufen nach | | |
|---|---|---|---|
| Beispiel | 10s | 20s | 40s Belichtung |
| 136 | 9 | 12 | 14 |
| 137 | 8 | 11 | 13 |
| 138 | 9 | 11 | 13 |
| 140 | 8 | 10 | 13 |
| 141 | 9 | 12 | 15 |
| 142 | 11 | 14 | 17 |
| 144 | 9 | 12 | 14 |
| 145 | 9 | 12 | 14 |
| 146 | 8 | 10 | 13 |
| 148 | 10 | 13 | 16 |
| 149 | 10 | 13 | 15 |
| 150 | 11 | 13 | 16 |
| 151 | 12 | 14 | 18 |
| 153 | 9 | 12 | 14 |
| 154 | 10 | 14 | 16 |
| 156 | 11 | 14 | 16 |
| 157 | 11 | 14 | 17 |
| 158 | 12 | 15 | 17 |
| 159 | 10 | 13 | 16 |
| 160 | 10 | 12 | 15 |
| 161 | 9 | 12 | 14 |
| 162 | 10 | 13 | 16 |
| 164 | 9 | 12 | 14 |
| 165 | 9 | 12 | 15 |
| 166 | 10 | 13 | 15 |
| 167 | 9 | 11 | 13 |
| 168 | 10 | 13 | 16 |
| 170 | 10 | 13 | 15 |
| 171 | 10 | 13 | 16 |
| 174 | 11 | 14 | 16 |
| 176 | 11 | 14 | 16 |
| 177 | 9 | 12 | 14 |
| 178 | 11 | 13 | 15 |
| 179 | 9 | 11 | 13 |
| 180 | 11 | 14 | 16 |
| 181 | 10 | 13 | 15 |
| 182 | 10 | 13 | 15 |
| 183 | 12 | 13 | 16 |
| 184 | 9 | 13 | 16 |
| 185 | 10 | 13 | 15 |
| 186 | 9 | 12 | 15 |
| 187 | 8 | 10 | 12 |
| 188 | 8 | 10 | 12 |
| 189 | 8 | 11 | 13 |
| 190 | 8 | 11 | 14 |
| 191 | 8 | 10 | 12 |
| 192 | 9 | 12 | 14 |
| 193 | 11 | 14 | 16 |

Beispiel 196:

Es wird die Prozedur von Beispiel 195 wiederholt, jedoch wird das jeweilige Titanocen in einem 1:1-Gemisch von Benzophenon und 1-Hydroxycyclohexyl-phenyl-keton vorgelöst. Verwendet werden jeweils (bezogen auf Feststoffgehalt) 0,3 % Titanocen, 0,85 % Benzophenon und 0,85 % 1-Hydroxycyclohexyl-phenyl-keton. Tabelle 13 gibt die dabei erreichte Anzahl der Abbildungsstufen an.

Tabelle 13

| Titanocen Beispiel | Zahl der abgebildeten Stufen nach Belichtung | | |
|---|---|---|---|
| | 10s | 20s | 40s |
| 87 | 12 | 14 | 17 |
| 90 | 11 | 13 | 15 |
| 91 | 11 | 13 | 15 |
| 92 | 11 | 13 | 15 |
| 96 | 11 | 13 | 15 |
| 97 | 11 | 13 | 16 |
| 105 | 10 | 12 | 14 |
| 106 | 11 | 13 | 16 |

**Patentansprüche**

**1.** Titanocene der Formel I

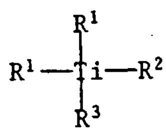

I,

worin beide $R^1$ unabhängig voneinander unsubstituiertes oder durch $C_1$-$C_{18}$-Alkyl oder -Alkoxy, $C_2$-$C_{18}$-Alkenyl, $C_5$-$C_8$-Cycloalkyl, $C_6$-$C_{16}$-Aryl, $C_7$-$C_{16}$-Aralkyl, $SiR^4{}_3$, $GeR^4{}_3$, Cyano oder Halogen substituiertes Cyclopentadienyl$^{\ominus}$, Indenyl$^{\ominus}$ oder 4,5,6,7-Tetrahydroindenyl$^{\ominus}$ bedeuten oder beide $R^1$ zusammen für einen unsubstituierten wie zuvor substituierten Rest der Formel II

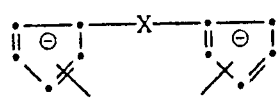

II

stehen, worin X $-(CH_2)_n-$ mit n = 1, 2 oder 3, unsubstituiertes oder durch Phenyl substituiertes Alkyliden mit 2 bis 12 C-Atomen, Cycloalkyliden mit 5 bis 7 Ringkohlenstoffatomen, $SiR^4{}_2$, $SiR^4{}_2$-O-$SiR^4{}_2$, $GeR^4{}_2$ oder $SnR^4{}_2$ ist, und $R^4$ $C_1$-$C_{12}$-Alkyl, $C_5$-$C_{12}$-Cycloalkyl, $C_6$-$C_{16}$-Aryl oder $C_7$-$C_{16}$-Aralkyl bedeutet,
$R^2$ einen 6-gliedrigen carbocyclischen oder 5- oder 6-gliedrigen heterocyclischen aromatischen Rest bedeutet, der in mindestens einer der beiden ortho-Stellungen zur Titankohlenstoffbindung mit Fluoratomen substituiert ist und wobei der aromatische Rest weitere Substituenten enthalten kann,
$R^3$ eine der für $R^2$ gegebenen Bedeutungen hat oder $R^2$ und $R^3$ zusammen einen Rest der Formel III bedeuten,

$$—Q—Y—Q— \quad III$$

in dem Q für einen carbocyclischen aromatischen Rest steht, wobei die beiden Bindungen jeweils in Orthostellung zur Y-Gruppe stehen und die zweite Orthostellung zur Titankohlenstoffbindung jeweils durch ein Fluoratom substituiert ist und wobei Q weitere Substituenten enthalten kann, und Y $CH_2$, Alkyliden mit 2 bis 12 C-Atomen, Cycloalkyliden mit 5 bis 7 Ringkohlenstoffatomen, $NR^4$, O, S, SO, $SO_2$, CO, $SiR^4{}_2$, $GeR^4{}_2$ oder $SnR^4{}_2$ bedeutet und $R^4$ die zuvor angegebene Bedeutung hat,
wobei die Titanocene dadurch gekennzeichnet sind, dass $R^2$ und $R^3$ oder der Rest der Formel III durch einen Rest der Formel IV, IVa oder IVb substituiert sind,

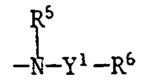
IV

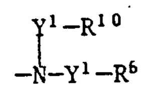
IVa

worin $R^5$ Wasserstoff, lineares oder verzweigtes $C_1$-$C_{20}$-Alkyl, $C_2$-$C_{20}$-Alkenyl, $C_3$-$C_8$-Cycloalkyl, $C_4$-$C_{20}$-Cycloalkylalkyl oder -Alkylcycloalkyl, $C_5$-$C_{20}$-Alkylcycloalkylalkyl, $C_6$-$C_{20}$-Cycloalkenylalkyl, $C_6$-$C_{14}$-Aryl, $C_7$-$C_{20}$-Aralkyl oder -Alkaryl, $C_8$-$C_{20}$-Alkaralkyl oder $C_3$-$C_{12}$-Trialkylsilyl darstellt, wobei diese Reste unsubstituiert oder durch $C_1$-$C_{18}$-Alkoxy, $C_1$-$C_{18}$-Alkylthio, $C_1$-$C_{18}$-Alkylsulfonyl, $C_6$-$C_{10}$-Arylsulfonyl, $C_7$-$C_{20}$-Alkarylsulfonyl, 2-Tetrahydrofuranyl oder Cyano substituiert sind,
$R^6$ eine der für $R^5$ gegebenen Bedeutungen hat oder $C_1$-$C_{20}$-Halogenalkyl, durch -CO- unterbrochenes $C_2$-$C_{20}$-Alkyl oder durch -COOH oder -$COOR^4$ substituiertes $C_1$-$C_{12}$-Alkyl ist und im Falle, dass $Y^1$ -CO-, -CS- oder -$SO_2$- ist, auch -$NR^7R^8$ bedeuten kann, worin $R^7$ und $R^8$ unabhängig voneinander eine der für $R^5$ gegebenen Bedeutungen haben oder $R^7$ und $R^8$ zusammen $C_3$-$C_7$-Alkylen bedeuten, das durch -O-, -S-oder -N($R^9$)- unterbrochen werden kann, worin $R^9$ Wasserstoff, $C_1$-$C_{12}$-Alkyl, $C_3$-$C_{12}$-Alkenyl, $C_7$-$C_{12}$-Aralkyl oder $C_2$-$C_{20}$-Alkanoyl bedeutet,
oder $R^5$ und $R^6$ zusammen lineares oder verzweigtes $C_2$-$C_8$-Alkylen oder durch Halogen, $C_1$-$C_4$-Alkoxy, Allyloxy oder -$NR^7R^8$ substituiertes $C_2$-$C_8$-Alkylen oder einen zweiwertigen Rest der Formel

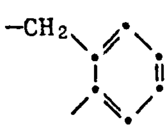 oder 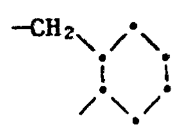 oder 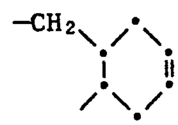

bedeuten,
$Y^1$ eine Gruppe -CO-, -CS-, -COO-, -$SO_2$- oder -$SiR^4{}_2$- bedeutet, worin $R^4$ die zuvor gegebene Bedeutung hat,
$R^{10}$ eine der für $R^6$ gegebenen Bedeutungen hat oder $R^{10}$ und $R^6$ zusammen $C_1$-$C_8$-Alkandiyl, $C_2$-$C_8$-Alkendiyl, $C_6$-$C_{14}$-Arendiyl, $C_4$-$C_{12}$-Cycloalkandiyl, $C_5$-$C_{12}$-Cycloalkendiyl, $C_6$-$C_{14}$Cycloalkadiendiyl, $C_7$-$C_{20}$-Bicycloalkandiyl, $C_7$-$C_{20}$-Bicycloalkendiyl oder durch -O-, -S-oder -N($R^9$)-unterbrochenes $C_2$-$C_4$-Alkandiyl bedeuten, wobei diese Reste unsubstituiert oder durch einen oder mehrere der Substituenten Halogen, $C_1$-$C_{10}$-Alkoxy, $C_1$-$C_{20}$-Alkyl, $C_3$-$C_{20}$-Alkenyl oder $C_6$-$C_{14}$-Aryl substituiert sind.

2. Titanocene gemäss Anspruch 1, dadurch gekennzeichnet, dass $R^1$ Cyclopentadienyl$^{\ominus}$ oder Methylcyclopentadienyl$^{\ominus}$ ist.

3. Titanocene gemäss Anspruch 1, dadurch gekennzeichnet, dass $R^1$ Cyclopentadienyl$^{\ominus}$ ist.

4. Titanocene gemäss Anspruch 1, dadurch gekennzeichnet, dass $R^2$ und $R^3$ die gleiche Bedeutung haben.

5. Titanocene gemäss Anspruch 1, dadurch gekennzeichnet, dass der Rest $R^2$ in beiden Orthostellungen mit Fluor substituiert ist.

6. Titanocene gemäss Anspruch 1, dadurch gekennzeichnet, dass $R^2$ und $R^3$ für 2,6-Difluorphen-1-yl stehen, an das ein Rest der Formel IV, IVa oder IVb gebunden ist, und das weitere 1 oder 2 gleiche oder verschiedene Substituenten enthalten kann.

7. Titanocene gemäss Anspruch 6, dadurch gekennzeichnet, dass in formel I beide $R^1$ Cyclopentadienyl$^{\ominus}$ und $R^2$ und $R^3$ Reste der Formel V

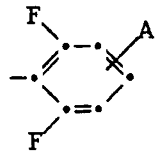

(V)

bedeuten, worin A eine Gruppe der Formel IV, IVa oder IVb bedeutet.

8. Titanocene gemäss Anspruch 7, dadurch gekennzeichnet, dass in Formel V die Gruppe A in Orthostellung zu einem F-Atom gebunden ist.

9. Titanocene gemäss Anspruch 7, dadurch gekennzeichnet, dass A eine Gruppe der Formel IV ist.

10. Titanocene gemäss Anspruch 1, dadurch gekennzeichnet, dass $R^2$ und $R^3$ durch eine Gruppe der Formel IV substituiert sind, worin $R^5$ Wasserstoff, unsubstituiertes oder durch $C_1$-$C_{12}$-Alkoxy oder Tetrahydrofuryl substituiertes $C_1$-$C_{12}$-Alkyl, $C_2$-$C_5$-Alkenyl, $C_5$-$C_7$-Cycloalkyl, $C_6$-$C_{18}$-Cycloalkylalkyl oder -Alkylcycloalkyl, $C_7$-$C_{18}$-Alkylcycloalkylalkyl, $C_7$-$C_{16}$-Aralkyl oder $C_8$-$C_{16}$-Alkaralkyl bedeutet, $R^6$ eine der für $R^5$ gegebenen Bedeutungen hat oder $C_6$-$C_{10}$-Aryl, $C_7$-$C_{18}$-Alkaryl, $C_1$-$C_{12}$-Halogenalkyl oder -$NR^7R^8$ darstellt, worin $R^7$ und $R^8$ unabhängig voneinander Wasserstoff, $C_1$-$C_{12}$-Alkyl, Phenyl, Benzyl oder Cyclohexyl bedeuten oder $R^7$ und $R^8$ zusammen $C_4$-$C_5$-Alkylen oder 3-Oxapentamethylen bedeuten, oder $R^5$ und $R^6$ zusammen $C_2$-$C_8$-Alkylen bedeuten und $Y^1$ -CO-, -CS-, -COO-oder -$SO_2$-bedeutet.

11. Titanocene gemäss Anspruch 1, dadurch gekennzeichnet, dass $R^2$ und $R^3$ durch eine Gruppe der Formel IV substituiert sind, worin $R^5$ Wasserstoff, $C_1$-$C_{12}$-Alkyl, Cyclohexyl, Cyclohexylmethyl, 2-Tetrahydrofurylmethyl, $C_2$-$C_8$-Alkoxyalkyl, Allyl oder $C_7$-$C_9$-Aralkyl ist, $R^6$ $C_1$-$C_{18}$-Alkyl, $C_1$-$C_4$-Halogenalkyl, Cyclohexyl, $C_6$-$C_{10}$-Aryl oder -Halogenaryl oder $C_7$-$C_{18}$-Alkaryl bedeutet oder $R^5$ und $R^6$ zusammen $C_2$-$C_6$-Alkylen bedeuten und $Y^1$ -CO-, -COO-oder -$SO_2$- ist oder der Rest -$Y^1$-$R^6$ eine Gruppe -CO-$NHR^7$, -CS-$NHR^7$, -CO-$NR^7R^8$ oder -$SO_2$-$N^7R^8$ bedeutet, worin $R^7$ $C_1$-$C_{12}$-Alkyl oder Phenyl ist, $R^8$ $C_1$-$C_{12}$-Alkyl ist oder $R^7$ und $R^8$ zusammen $C_4$-$C_5$-Alkylen oder 3-Oxapentamethylen bedeuten.

12. Titanocene gemäss Anspruch 11, worin $R^5$ Wasserstoff, $C_1$-$C_8$-Alkyl oder $C_7$-$C_9$-Aralkyl ist, $R^6$ $C_1$-$C_{18}$-Alkyl, Trifluormethyl, Phenyl, oder durch Halogen oder $C_1$-$C_{12}$-Alkyl substituiertes Phenyl bedeutet oder $R^5$ und $R^6$ zusammen $C_2$-$C_6$-Alkylen bedeuten und $Y^1$ -CO- oder -$SO_2$- ist.

13. Titanocene gemäss Anspruch 1, dadurch gekennzeichnet, dass $R^2$ und $R^3$ durch eine Gruppe der Formel IVa substituiert sind, worin $R^6$ und $R^{10}$ zusammen $C_2$-$C_8$-Alkandiyl, $C_2$-$C_8$-Alkendiyl, $C_6$-$C_{14}$-Arendiyl oder $C_7$-$C_{12}$-Bicycloalkendiyl bedeuten und $Y^1$ -CO- ist.

14. Verfahren zur Herstellung von Titanocenen der Formel I gemäss Anspruch 1, dadurch gekennzeichnet, dass man 1 Mol einer Verbindung der Formel VI

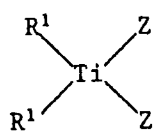

(VI),

worin $R^1$ die in Anspruch 1 angegebene Bedeutung hat und Z für Halogen, besonders Chlor, steht, entweder mit einem Mol $LiR^2$ oder $LiR^3$ und danach mit einem Mol $LiR^3$ bzw. $LiR^2$ umsetzt, oder mit 2 Mol $LiR^2$ oder mit 1 Mol $Li_2QYQ$ umsetzt, wobei $R^2$, $R^3$ und QYQ die in Anspruch 1 angegebenen Bedeutungen haben und danach die Verbindung der Formel I in an sich bekannter Weise isoliert.

15. Durch Strahlung polymerisierbare Zusammensetzung, enthaltend (a) mindestens eine nichtflüchtige, monomere, oligomere oder polymere Verbindung mit mindestens einer polymerisierbaren ethylenisch

ungesättigten Doppelbindung und (b) mindestens ein Titanocen der Formel I nach Anspruch 1 als Photoinitiator.

**16.** Zusammensetzung gemäss Anspruch 15, dadurch gekennzeichnet, dass zusätzlich mindestens ein von (b) verschiedener Photoinitiator (c) enthalten ist.

**17.** Zusammensetzung gemäss Anspruch 16, enthaltend als Photoinitiator (c) ein Benzophenon, einen Benzoinalkylether, ein Benzilketal, ein 4-Aroyl-1,3-dioxolan, ein Dialkoxyacetophenon, ein $\alpha$-Hydroxy- oder $\alpha$-Aminoacetophenon oder ein $\alpha$-Hydroxycycloalkylphenylketon oder Mischungen davon als zusätzlichen Photoinitiator.

**18.** Verwendung einer Zusammensetzung gemäss Anspruch 15 zur Herstellung von Lacken, Druckfarben, Druckplatten, Resistmaterialien sowie als Bildaufzeichnungsmaterial.

**19.** Beschichtetes Substrat, das auf mindestens einer Oberfläche mit einer Zusammensetzung gemäss Anspruch 15 beschichtet ist.

**20.** Verfahren zur photographischen Herstellung von Reliefabbildungen, dadurch gekennzeichnet, dass man ein beschichtetes Substrat gemäss Anspruch 19 bildmässig belichtet und die unbelichteten Anteile danach mit einem Lösungsmittel entfernt.

**21.** Verwendung von Titanocenen der Formel I gemäss Anspruch 1 alleine oder zusammen mit anderen Initiatoren als Photoinitiatoren für die Photopolymerisation von nichtflüchtigen monomeren, oligomeren oder polymeren Verbindungen mit mindestens einer polymerisierbaren ethylenisch ungesättigten Doppelbindung.

**22.** Photoinitiatorengemisch, enthaltend einen Photoinitiator vom Typ der Benzophenone, Benzoinalkylether, Benzilketale, 4-Aroyl-1,3-dioxolane, Dialkoxyacetophenone, $\alpha$-Hydroxyacetophenone, $\alpha$-Hydroxycycloalkylphenylketone, $\alpha$-Aminoacetophenone oder Mischungen hiervon und ein Titanocen der Formel I gemäss Anspruch 1.

**Claims**

**1.** A titanocene of the formula I

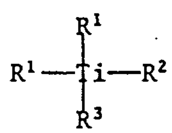

I.

in which both $R^1$ radicals, independently of one another, are cyclopentadienyl$^\ominus$, indenyl$^\ominus$ or 4,5,6,7-tetrahydroindenyl$^\ominus$, each of which is unsubstituted or substituted by $C_1$-$C_{18}$alkyl or -alkoxy, $C_2$-$C_{18}$alkenyl, $C_5$-$C_8$cycloalkyl, $C_6$-$C_{16}$aryl, $C_7$-$C_{16}$aralkyl, $SiR^4_3$, $GeR^4_3$, cyano or halogen, or both $R^1$ radicals together are a radical of the formula II

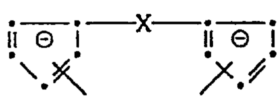

II

unsubstituted or substituted as above, in which X is $-(CH_2)-$ where n = 1, 2 or 3, unsubstituted or phenyl-substituted alkylidene having 2 to 12 carbon atoms, cycloalkylidene having 5 to 7 ring carbon atoms, $SiR^4_2$, $SiR^4_2$-O-$SiR^4_2$, $GeR^4_2$ or $SnR^4_2$, and $R^4$ is $C_1$-$C_{12}$alkyl, $C_5$-$C_{12}$cycloalkyl, $C_6$-$C_{16}$aryl or $C_7$-$C_{16}$aralkyl, $R^2$ is a 6-membered carbocyclic or 5- or 6-membered heterocyclic aromatic radical which is substituted by fluorine atoms in at least one of the two ortho-positions to the titanium-carbon bond, and in which the aromatic radical may contain further substituents, $R^3$ has one of the definitions given for $R^2$, or $R^2$ and $R^3$ together are a radical of the formula III

-Q-Y-Q- III

in which Q is a carbocyclic aromatic radical where the two bonds are each in the ortho-position to the Y group and the second ortho-position to the titanium-carbon bond is in each case substituted by a fluorine atom, and where Q may contain further substituents, and Y is $CH_2$, alkylidene having 2 to 12 carbon atoms, cycloalkylidene having 5 to 7 ring carbon atoms, $NR^4$, O, S, SO, $SO_2$, CO, $SiR^4_2$ , $GeR^4_2$ or $SnR^4_2$ , and $R^4$ is as defined above, wherein, in the titanocenes, $R^2$ and $R^3$ or the radical of the formula III are substituted by a radical of the formula IV, IVa or IVb

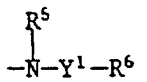
IV

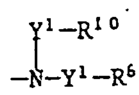
IVa

-N=C=O IVb

in which $R^5$ is hydrogen, linear or branched $C_1$-$C_{20}$alkyl, $C_2$-$C_{20}$alkenyl, $C_3$-$C_8$cycloalkyl, $C_4$-$C_{20}$cycloalkylalkyl or -alkylcycloalkyl, $C_5$-$C_{20}$alkylcycloalkylalkyl, $C_6$-$C_{20}$cycloalkenylalkyl, $C_6$-$C_{14}$aryl, $C_7$-$C_{20}$aralkyl or -alkaryl, $C_8$-$C_{20}$alkaralkyl or $C_3$-$C_{12}$trialkylsilyl, where these radicals are unsubstituted or substituted by $C_1$-$C_{18}$alkoxy, $C_1$-$C_{18}$alkylthio, $C_1$-$C_{18}$alkylsulphonyl, $C_6$-$C_{10}$arylsulphonyl, $C_7$-$C_{20}$alkarylsulphonyl, 2 -tetrahydrofuryl or cyano, $R^6$ has one of the definitions given for $R^5$ or is $C_1$-$C_{20}$halogenoalkyl, $C_2$-$C_{20}$alkyl which is interrupted by -CO-, or $C_1$-$C_{12}$alkyl which is substituted by -COOH or -$COOR^4$, and, in the case where $Y^1$ is -CO-, -CS- or -$SO_2$-, may also be -$NR^7R^8$ in which $R^7$ and $R^8$, independently of one another, have one of the definitions given for $R^5$, or $R^7$ and $R^8$ together are $C_3$-$C_7$alkylene, which may be interrupted by -O-, -S or -N($R^9$)- in which $R^9$ is hydrogen, $C_1$-$C_{12}$alkyl, $C_3$-$C_{12}$alkenyl,$C_7$-$C_{12}$aralkyl or $C_2$-$C_{20}$alkanoyl, or $R^5$ and $R^6$ together are linear or branched $C_2$-$C_8$alkylene, or $C_2$-$C_8$alkylene which is substituted by halogen, $C_1$-$C_4$alkoxy, allyloxy or -$NR^7R^8$, or are a divalent radical of the formula

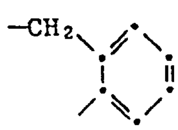 or 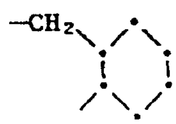 or 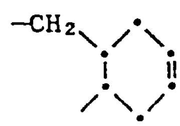 ,

$Y^1$ is a -CO-, -CS-, -COO-, -$SO_2$- or -$SiR^4_2$ - group in which $R^4$ is as defined above, $R^{10}$ has one of the definitions given for $R^6$, or $R^{10}$ and $R^6$ together are $C_1$-$C_8$alkanediyl, $C_2$-$C_8$alkenediyl $C_6$-$C_{14}$arenediyl, $C_4$-$C_{12}$cycloalkanediyl, $C_5$-$C_{12}$cycloalkenediyl, $C_6$-$C_{14}$cycloalkadienediyl, $C_7$-$C_{20}$bicycloalkanediyl, $C_7$-$C_{20}$bicycloalkenediyl, or $C_2$-$C_4$alkanediyl which is interrupted by -O-, -S- or -N($R^9$)-, where these radicals are unsubstituted or substituted by one or more of the substituents halogen, $C_1$-$C_{10}$alkoxy, $C_1$-$C_{20}$alkyl, $C_3$-$C_{20}$alkenyl or $C_6$-$C_{14}$aryl.

2. A titanocene according to Claim 1, where $R^1$ is cyclopentadienyl$^{\ominus}$ or methylcyclopentadienyl$^{\ominus}$.

3. A titanocene according to Claim 1, where $R^1$ is cyclopentadienyl$^{\ominus}$.

4. A titanocene according to Claim 1, wherein $R^2$ and $R^3$ are identical.

5. A titanocene according to Claim 1, wherein the $R^2$ radical is substituted by fluorine in both the ortho-positions.

6. A titanocene according to Claim 1, wherein $R^2$ and $R^3$ are 2,6-difluorophen-1-yl to which a radical of the formula IV, IVa or IVb is bonded, and which may contain a further 1 or 2 identical or different substituents.

**7.** A titanocene according to Claim 6, wherein, in the formula I, both $R^1$ are cyclopentadienyl$^{\ominus}$ and $R^2$ are $R^3$ are radicals of the formula V

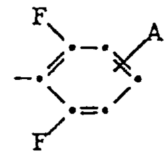

(V)

in which A is a group of the formula IV, IVa or IVb.

**8.** A titanocene according to Claim 7, wherein, in the formula V, the group A is bonded in the ortho-position to an F atom.

**9.** A titanocene according to Claim 7, wherein A is a group of the formula IV.

**10.** A titanocene according to Claim 1, wherein $R^2$ and $R^3$ are substituted by a group of the formula IV in which $R^5$ is hydrogen, unsubstituted or $C_1$-$C_{12}$alkoxy- or tetrahydrofuryl- substituted $C_1$-$C_{12}$alkyl, $C_2$-$C_5$alkenyl, $C_5$-$C_7$cycloalkyl, $C_6$-$C_{18}$cycloalkylalkyl or -alkylcycloalkyl, $C_7$-$C_{18}$alkylcycloalkylalkyl, $C_7$-$C_{16}$aralkyl or $C_8$-$C_{16}$alkaralkyl, $R^6$ has one of the definitions given for $R^5$ or is $C_6$-$C_{10}$aryl, $C_7$-$C_{18}$alkaryl, $C_1$-$C_{12}$halogenoalkyl or $-NR^7R^8$ in which $R^7$ and $R^8$, independently of one another, are hydrogen, $C_1$-$C_{12}$alkyl, phenyl, benzyl or cyclohexyl, or $R^7$ and $R^8$ together are $C_5$-$C_5$alkylene or 3-oxapentamethylene, or $R^5$ and $R^6$ together are $C_2$-$C_8$alkylene and $Y^1$ is -CO-, -CS-, -COO- or $-SO_2-$

**11.** A titanocene according to Claim 1, wherein $R^2$ and $R^3$ are substituted by a group of the formula IV in which $R^5$ is hydrogen, $C_1$-$C_{12}$alkyl, cyclohexyl, cyclohexylmethyl, 2-tetrahydrofurylmethyl, $C_2$-$C_8$alkoxyalkyl, allyl or $C_7$-$C_9$aralkyl, $R_6$ is $C_1$-$C_{18}$alkyl, $C_1$-$C_4$halogenoalkyl, cyclohexyl, $C_6$-$C_{10}$aryl or -halogenoaryl or $C_7$-$C_{18}$alkaryl, or $R^5$ and $R^6$ together are $C_2$-$C_6$alkylene, and $Y^1$ is -CO-, -COO- or $-SO_2-$ or the radical $-Y^1-R^6$ is a $-CO-NHR^7$, $-CS-NHR^7$, $-CO-NR^7R^8$ or $-SO_2-N^7R^8$ group in which $R^7$ is $C_1$-$C_{12}$alkyl or phenyl and $R^8$ is $C_1$-$C_{12}$alkyl, or $R^7$ and $R^8$ together are $C_4$-$C_5$alkylene or 3-oxapentamethylene.

**12.** A titanocene according to Claim 11, in which $R^5$ is hydrogen, $C_1$-$C_8$alkyl or $C_7$-$C_9$aralkyl, $R^6$ is $C_1$-$C_{18}$alkyl, trifluoromethyl, phenyl or halogen- or $C_1$-$C_{12}$alkyl-substituted phenyl, or $R^5$ and $R^6$ together are $C_2$-$C_6$alkylene, and $Y^1$ is -CO- or $-SO_2-$.

**13.** A titanocene according to Claim 1, wherein $R^2$ and $R^3$ are substituted by a group of the formula IVa in which $R^6$ and $R^{10}$ together are $C_2$-$C_8$alkanediyl, $C_2$-$C_8$alkenediyl, $C_6$-$C_{14}$arenediyl or $C_7$-$C_{12}$bicycloalkenediyl, and $Y^1$ is -CO-.

**14.** A process for the preparation of a titanocene of the formula I according to Claim 1, which comprises reacting 1 mole of a compound of the formula VI

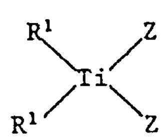

(VI),

in which $R^1$ is as defined in Claim 1 and Z is halogen, particularly chlorine, either with 1 mole of $LiR^2$ or $LiR^3$ and then with 1 mole of $LiR^3$ or $LiR^2$, or with 2 moles of $LiR^2$ or with 1 mole of $LiR_2$ QYQ, where $R^2$, $R^3$ and QYQ are as defined in Claim 1, and then isolating the compound of the formula I in a manner known per se.

**15.** A radiation-polymerizable composition containing (a) at least one non-volatile, monomeric, oligomeric or polymeric compound containing at least one polymerizable, ethylenically unsaturated double bond, and

(b) at least one titanocene of the formula I according to Claim 1 as photoinitiator.

**16.** A composition according to Claim 15, wherein, in addition, at least one photoinitiator (c) other than (b) is present.

**17.** A composition according to Claim 16, containing, as photoinitiator (c), a benzophenone, a benzoin alkyl ether, a benzil ketal, a 4-aroyl-1,3-dioxolane, a dialkoxyacetophenone, an $\alpha$-hydroxy- or $\alpha$-aminoacetophenone or an $\alpha$-hydroxycycloalkyl phenyl ketone, or mixtures thereof, as an additional photoinitiator.

**18.** The use of a composition according to Claim 15 for the production of paints, printing inks, printing plates, resist materials and as image-recording material.

**19.** A coated substrate which is coated on at least one surface with a composition according to Claim 15.

**20.** A process for photographic production of relief images, which comprises exposing a coated substrate according to Claim 19 image-wise and then removing the unexposed areas using a solvent.

**21.** The use of a titanocene of the formula I according to Claim 1, alone or together with other initiators, as photoinitiators for photopolymerization of non-volatile, monomeric, oligomeric or polymeric compounds containing at least one polymerizable, ethylenically unsaturated double bond.

**22.** A photoinitiator mixture containing a photoinitiator of the benzophenone, benzoin alkyl ether, benzil ketal, 4-aroyl-1,3-dioxolane, dialkoxyacetophenone, $\alpha$-hydroxyacetophenone, $\alpha$-hydroxycycloalkyl phenyl ketone or $\alpha$-aminoacetophenone type, or mixtures thereof, and a titanocene of the formula I according to Claim 1.

## Revendications

**1.** Titanocènes répondant à la formule I

$$\begin{array}{c} R^1 \\ | \\ R^1 - Ti - R^2 \\ | \\ R^3 \end{array} \qquad I$$

dans laquelle les deux $R^1$ signifient chacun indépendamment l'un de l'autre, un cyclopentadiényle$^{\ominus}$, un indényle$^{\ominus}$ ou un 4,5,6,7-tétrahydroindényle$^{\ominus}$ non substitué ou substitué par un alkyle ou un alcoxy en $C_1$-$C_{18}$ un alcényle en $C_2$-$C_{18}$, un cycloalkyle en $C_5$-$C_8$, un aryle en $C_6$-$C_{16}$, un aralkyle en $C_7$-$C_{16}$,$SiR_3{}^4$, $GeR_3{}^4$, un cyanogène ou un halogène, ou les deux $R^1$ ensemble représentent un radical de formule II non substitué ou substitué comme ci-dessus

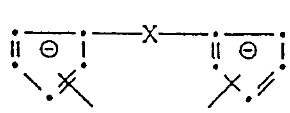

II

dans laquelle X-(-$CH_2$-)$_n$- avec n = 1, 2 ou 3, est un alkylidène comportant de 2 à 12 atomes de C éventuellement substitué par un phényle, un cycloalkylidène comportant de 5 à 7 atomes de carbone cycliques, $SiR_2{}^4$, $SiR_2{}^4$-O-$SiR_2{}^4$, $GeR_2{}^4$ ou $SnR_2{}^4$ et $R^4$ signifie un alkyle en $C_1$-$C_{12}$, un cycloalkyle en $C_5$-$C_{12}$, un aryle en $C_6$-$C_{16}$ ou un aralkyle en $C_7$-$C_{16}$,

$R^2$ signifie un radical aromatique carbocyclique à 6 chaînons ou hétérocyclique à 5 ou 6 chaînons substitué dans au moins une des deux positions ortho par rapport à la liaison titane-carbone par des atomes de fluor et le radical aromatique peut alors être porteur d'autres substituants,

$R^3$ a l'une des significations données pour $R^2$ ou $R^2$ et $R^3$ ensemble signifient un radical répondant à la formule III

-Q-Y-Q- III

où Q représente un radical aromatique carbocyclique dans lequel les deux liaisons sont chacune en position ortho par rapport au groupement Y et la deuxième position ortho par rapport à la liaison titane-carbone, est substituée chaque fois par un atome de fluor et Q peut alors être porteur d'autres substituants et Y signifie $CH_2$, un alkylidène avec de 2 à 12 atomes de C, un cycloalkylidène avec de 5 à 7 atomes de carbone cyclique, $NR^4$, O, S, SO, $SO_2$, CO, $SiR_2{}^4$, $GeR_2{}^4$ ou $SnR_2{}^4$ et $R^4$ a la signification donnée ci-dessus,

ces titanocènes étant caractérisés en ce que $R^2$ et $R^3$ ou le radical de formule III sont substitués par un radical de formule IV, IVa ou IVb,

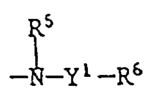

IV

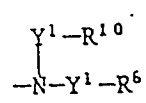

IVa

-N=C=O IVb

où $R^5$ représente un hydrogène, un alkyle en $C_1$-$C_{20}$ linéaire ou ramifié, un alcényle en $C_2$-$C_{20}$, un cycloalkyle en $C_3$-$C_8$, un cycloalkylalkyle ou un alkylcycloalkyle en $C_4$-$C_{20}$, un alkylcycloalkylalkyle en $C_5$-$C_{20}$, un cycloalcénylalkyle en $C_6$-$C_{20}$, un aryle en $C_6$-$C_{14}$, un aralkyle ou un alcaryle en $C_7$-$C_{20}$, un alcaralkyle en $C_8$-$C_{20}$ ou un trialkylsilyle en $C_3$-$C_{12}$, ces radicaux sont alors non substitués ou substitués par un alcoxy en $C_1$-$C_{18}$, un alkylthio en $C_1$-$C_{18}$, un alkylsulfonyle en $C_1$-$C_{18}$, un arylsulfonyle en $C_6$-$C_{10}$, un alcarylsulfonyle en $C_7$-$C_{20}$, un 2-tétrahydrofuryle ou un cyanogène,

$R^6$ a l'une des significations données pour $R^5$ ou est halogénoalkyle en $C_1$-$C_{20}$, un alkyle en $C_2$-$C_{20}$ interrompu par -CO- ou un alkyle en $C_1$-$C_{12}$ substitué par -COOH ou -$COOR^4$ et dans le cas où $Y^1$ est -CO-, -CS- ou -$SO_2$-, il peut aussi signifier -$NR^7R^8$ où $R^7$ et $R^8$ chacun indépendamment l'un de l'autre, ont l'une des significations données pour $R^5$ ou $R^7$ et $R^8$ ensemble signifient un alkylène en $C_3$-$C_7$ qui peut être interrompu par -O-, -S- ou -$N(R^9)$- où $R^9$ signifie l'hydrogène, un alkyle en $C_1$-$C_{12}$, un alcényle en $C_3$-$C_{12}$, un aralkyle en $C_7$-$C_{12}$, ou un alcanoyle en $C_2$-$C_{20}$,

ou $R^5$ et $R^6$ ensemble signifient un alkylène en $C_2$-$C_8$ linéaire ou ramifié ou un alkylène en $C_2$-$C_8$ substitué par un halogène, un alcoxy $C_1$-$C_4$, un allyloxy ou -$NR^7R^8$ ou un radical bivalent de formule

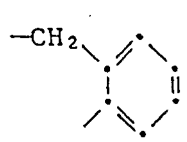 ou 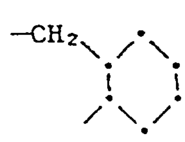 ou 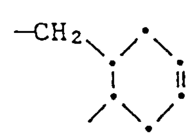

$Y^1$ signifie un groupe -CO-, -CS-, -COO-, -$SO_2$- ou -$SiR_2{}^4$-, dans lequel $R^4$ a la signification donnée auparavant,

$R^{10}$ a l'une des significations données pour $R^6$ ou $R^{10}$ et $R^6$ ensemble signifient un alcanediyle en $C_1$-$C_8$, un alcènediyle en $C_2$-$C_8$, un arènediyle en $C_6$-$C_{14}$, un cycloalcanediyle en $C_4$-$C_{12}$, un cycloalcènediyle en $C_5$-$C_{12}$, un cycloalcadiènediyle en $C_6$-$C_{14}$, un bicycloalcanediyle en $C_7$-$C_{20}$, un bicycloalcènediyle en $C_7$-$C_{20}$, ou un alcanediyle en $C_2$-$C_4$ interrompu par -O-, -S- ou -$N(R^9)$, ces radicaux pouvant être non substitués ou porteurs d'un ou de plusieurs substituants pris parmi les suivants : un halogène, un alcoxy en $C_1$-$C_{10}$, un alkyle en $C_1$-$C_{20}$, un alcényle en $C_3$-$C_{20}$ ou un aryle en $C_6$-$C_{14}$.

**2.** Titanocènes selon la revendication 1, caractérisés en ce que $R^1$ est un cyclopentadiényle$^{\ominus}$ ou méthylcyclopentadiényle$^{\ominus}$.

**3.** Titanocènes selon la revendication 1, caractérisés en ce que $R^1$ est un cyclopentadiényle$^{\ominus}$.

4. Titanocènes selon la revendication 1, caractérisés en ce que $R^2$ et $R^3$ ont la même signification.

5. Titanocènes selon la revendication 1, caractérisés en ce que le radical $R^2$ est substitué dans les deux positions ortho par le fluor.

6. Titanocènes selon la revendication 1, caractérisés en ce que $R^2$ et $R^3$ sont le 2,6-difluorophén-1-yl auquel est lié un radical de formule IV, IVa ou IVb et qui peut être porteur de 1 ou 2 autres substituants, identiques ou différents.

7. Titanocènes selon la revendication 6, caractérisés en ce que les deux $R^1$ dans la formule I signifient un cyclopentadiényle$^{\ominus}$ et $R^2$ et $R^3$ des radicaux répondant à la formule V

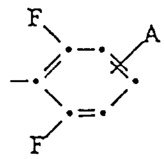

(V)

où A signifie un groupement de formule IV, IVa ou IVb.

8. Titanocènes selon la revendication 7, caractérisés en ce que le groupement A dans la formule V est lié en position ortho par rapport à un atome de fluor.

9. Titanocènes selon la revendication 7, caractérisés en ce que A est un groupement de formule IV.

10. Titanocènes selon la revendication 1, caractérisés en ce que $R^2$ et $R^3$ sont substitués par un groupement de formule IV où $R^5$ signifie un hydrogène, un alkyle en $C_1$-$C_{12}$, un alcènyle en $C_2$-$C_5$, un cycloalkyle en $C_5$-$C_7$, un cycloalkylalkyle ou alkylcycloalkyle en $C_6$-$C_{18}$, un alkylcycloalkylalkyle en $C_7$-$C_{18}$, un aralkyle en $C_7$-$C_{16}$ ou un alcaralkyle en $C_8$-$C_{16}$ non substitué ou substitué par un alcoxy en $C_1$-$C_{12}$ ou le tétrahydrofuryle, $R^6$ a l'une des significations données pour $R^5$ ou représente un aryle en $C_6$-$C_{10}$, un alcaryle en $C_7$-$C_{18}$, un halogénoalkyle en $C_1$-$C_{12}$ ou $-NR^7R^8$ où $R^7$ et $R^8$, indépendamment l'un de l'autre, signifient l'hydrogène, un alkyle en $C_1$-$C_{12}$, un phényle, un benzyle ou un cyclohexyle ou $R^7$ et $R^8$ ensemble signifient un alkylène en $C_4$-$C_5$ ou le 3-oxapentaméthylène ou $R^5$ et $R^6$ ensemble signifient un alkylène en $C_2$-$C_8$ et Y1 signifie -CO-, -CS-, -COO- ou $-SO_2-$.

11. Titanocènes selon la revendication 1, caractérisés en ce que $R^2$ et $R^3$ sont substitués par un groupement de formule IV, dans lequel $R^5$ est un hydrogène, un alkyle en $C_1$-$C_{12}$, le cyclohexyle, le cyclohexylméthyle, le 2-tétrahydrofurylméthyle, un alcoxyalkyle en $C_2$-$C_8$, un allyle ou un aralkyle en $C_7$-$C_9$, $R^6$ signifie un alkyle en $C_1$-$C_{18}$, un halogénoalkyle en $C_1$-$C_4$, le cyclohexyle, un aryle ou un halogénoaryle en $C_6$-$C_{10}$ ou un alcaryle en $C_7$-$C_{18}$ ou $R^5$ et $R^6$ ensemble signifient un alkylène en $C_2$-$C_6$ et $Y^1$ est -CO-, -COO- ou $-SO_2-$ ou le radical $-Y^1-R^6$ signifie un groupement $-CO-NHR^7$, $-CS-NHR^7$, $-CO-NR^7R^8$ ou $-SO_2-N^7R^8$ dans lequel $R^7$ est un alkyle en $C_1$-$C_{12}$, ou le phényle, $R^8$ est un alkyle en $C_1$-$C_{12}$ ou $R^7$ et $R^8$ ensemble signifient un alkylène en $C_4$-$C_5$ ou le 3-oxapentaméthylène.

12. Titanocènes selon la revendication 11, où $R^5$ est l'hydrogène, un alkyle en $C_1$-$C_8$ ou un aralkyle en $C_7$-$C_9$, $R^6$ signifie un alkyle en $C_1$-$C_{18}$, le trifluorométhyle, le phényle ou un phényle halogéné ou porteur d'un alkyle en $C_1$-$C_{12}$ ou $R^5$ et $R^6$ ensemble signifient un alkylène en $C_2$-$C_6$ et $Y^1$ est -CO-ou $-SO_2-$.

13. Titanocènes selon la revendication 1, caractérisés en ce que $R^2$ et $R^3$ sont substitués par un groupement de formule IVa, où $R^6$ et $R^{10}$ ensemble signifient un alcanediyle en $C_2$-$C_8$, un alcènediyle en $C_2$-$C_8$, un arènediyle en $C_6$-$C_{14}$, ou un bicycloalcènediyle en $C_7$-$C_{12}$ et $Y^1$ est -CO-.

14. Procédé pour la préparation de titanocènes répondant à la formule I selon la revendication 1, caractérisé en ce qu'on mélange 1 mole d'un composé de formule VI

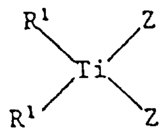

(VI),

dans laquelle $R^1$ a la signification donnée dans la revendication 1 et Z représente un halogène, surtout le chlore, soit avec une mole de $LiR^2$ ou $LiR^3$ et ensuite avec une mole de $LiR^3$ ou bien $LiR^2$ soit avec 2 moles de $LiR^2$ ou avec 1 mole de $Li^2QYQ$, $R^2$, $R^3$ et QYQ ayant alors la signification donnée dans la revendication 1, puis on isole le composé répondant à la formule I de façon connue en soi.

**15.** Composition polymérisable par irradiation contenant (a) au moins un composé non volatile monomère, oligomère ou polymère avec au moins une double liaison insaturée éthylénique polymérisable et (b) au moins un titanocène de formule I selon la revendication 1 en tant que photo-amorceur.

**16.** Composition selon la revendication 15, caractérisée en ce qu'elle contient en plus au moins un photo-amorceur (c) différent de (b).

**17.** Composition selon la revendication 16, contenant en tant que photo-amorceur (c) une benzophénone, un éther benzoinalkylique, un benzilcétal, un 4-aroyl-1,3-dioxolanne, une dialcoxyacétophénone, une $\alpha$-hydroxy- ou $\alpha$-aminoacétophénone ou une $\alpha$-hydroxycycloalkylphényl-cétone ou leurs mélanges en tant que photo-amorceurs suppplémentaires.

**18.** Utilisation d'une composition selon la revendication 15, pour la préparation de vernis, d'encres d'imprimerie, de clichés d'imprimerie, de matériaux de résists ainsi que de supports d'enregistrement d'images.

**19.** Substrat enduit, dont au moins une surface est enduite d'une composition selon la revendication 15.

**20.** Procédé pour la préparation photographique de clichés en relief caractérisé en ce qu'on irradie selon l'image un substrat enduit selon la revendication 19 et on élimine ensuite par un solvant les parties non irradiées.

**21.** Utilisation de titanocènes répondant à la formule I selon la revendication 1, seuls ou ensemble avec d'autres amorceurs en tant que photo-amorceurs pour la photopolymérisation de composés non volatiles monomères, oligomères ou polymères comportant au moins une double liaison à insaturation éthylénique.

**22.** Mélange de photo-amorceurs contenant un photoamorceur de type benzophénone, éther benzoinalkylique, benzilcétals, 4-aroyl-1,3-dioxolanne, dialcoxyacétophénone, $\alpha$-hydroxyacétophénone, $\alpha$-hydroxycycloalkylphényl-cétone, $\alpha$-aminoacétophénone ou leurs mélanges et un titanocène de formule I selon la revendication I.